(19) Europäisches Patentamt
European Patent Office
Office européen des brevets

(11) EP 3 426 801 B1

(12) # EUROPEAN PATENT SPECIFICATION

(45) Date of publication and mention of the grant of the patent:
**14.10.2020 Bulletin 2020/42**

(21) Application number: **17724104.9**

(22) Date of filing: **13.03.2017**

(51) Int Cl.:
***C12Q 1/6883*** *(2018.01)* ***G16B 15/00*** *(2019.01)*

(86) International application number:
**PCT/IB2017/000417**

(87) International publication number:
**WO 2017/153849 (14.09.2017 Gazette 2017/37)**

(54) **ALGORITHM AND AN IN VITRO METHOD BASED ON RNA EDITING TO SELECT PARTICULAR EFFECT INDUCED BY ACTIVE COMPOUNDS**

ALGORITHMUS UND IN-VITRO-VERFAHREN BASIEREND AUF RNS-MANIPULATION ZUR AUSWAHL DER SPEZIFISCHEN, DURCH AKTIVE VERBINDUNGEN INDUZIERTEN WIRKUNG

ALGORITHME ET PROCÉDÉ IN VITRO SUR LA BASE D'ÉDITION D'ARN DE MANIÈRE À SÉLECTIONNER UN EFFET PARTICULIER INDUIT PAR DES COMPOSÉS ACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2016 EP 16000600**

(43) Date of publication of application:
**16.01.2019 Bulletin 2019/03**

(73) Proprietors:
- **ALCEDIAG**
  **13790 Peynier (FR)**
- **Centre National de la Recherche Scientifique (CNRS)**
  **75016 Paris (FR)**

(72) Inventors:
- **WEISSMANN, Dinah**
  **34270 St. Mathieu de Tréviers (FR)**
- **VAN DER LAAN, Siem**
  **34190 Cazilhac (FR)**
- **SALVETAT, Nicolas**
  **34070 Montpellier (FR)**
- **MOLINA, Franck**
  **34270 Les Matelles (FR)**
- **PUJOL, Jean-François**
  **34270 St. Mathieu de Tréviers (FR)**

(74) Representative: **Touroude, Magali Linda**
**Touroude & Associates**
**2 bis, rue Alfred Nobel**
**Champs sur Marne**
**77420 Marne la vallée Cedex (FR)**

(56) References cited:
**WO-A1-2008/152146 WO-A1-2010/064231**
**WO-A1-2011/161253 WO-A2-2010/070074**

- **CAVAREC LAURENT ET AL: "In vitro screening for drug-induced depression and/or suicidal adverse effects: a new toxicogenomic assay based on CE-SSCP analysis of HTR2C mRNA editing in SH-SY5Y cells.", NEUROTOXICITY RESEARCH JAN 2013, vol. 23, no. 1, January 2013 (2013-01), pages 49-62, XP002760976, ISSN: 1476-3524 cited in the application**
- **EIFLER TRISTAN ET AL: "A screening protocol for identification of functional mutants of RNA editing adenosine deaminases.", CURRENT PROTOCOLS IN CHEMICAL BIOLOGY 1 DEC 2012, vol. 4, no. 4, 1 December 2012 (2012-12-01), pages 357-369, XP002760977, ISSN: 2160-4762**
- **KWAK SHIN ET AL: "Newly identified ADAR-mediated A-to-I editing positions as a tool for ALS research", RNA BIOLOGY, LANDES BIOSCIENCE, US, vol. 5, no. 4, 1 October 2008 (2008-10-01) , pages 193-197, XP002611508, ISSN: 1547-6286**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 426 801 B1



**(Cont. next page)**

- LEVANON E Y ET AL: "Systematic identification of abundant A-to-I editing sites in the human transcriptome", NATURE BIOTECHNOLOGY, GALE GROUP INC, US, vol. 22, no. 8, 1 August 2004 (2004-08-01) , pages 1001-1005, XP002336893, ISSN: 1087-0156, DOI: 10.1038/NBT996
- GALLO A ET AL: "A-to-I RNA editing and cancer From pathology to basic science", RNA BIOLOGY, LANDES BIOSCIENCE, US, vol. 5, no. 3, 1 July 2008 (2008-07-01), pages 135-139, XP002573375, ISSN: 1547-6286
- SEEBURG P H: "A-to-I editing: new and old sites, functions and speculations", NEURON, CELL PRESS, US, vol. 35, no. 1, 3 July 2002 (2002-07-03), pages 17-20, XP002336888, ISSN: 0896-6273, DOI: 10.1016/S0896-6273(02)00760-2
- NISWENDER C M: "Strategies and requirements for the detection of RNA editing in G protein coupled-receptor RNA", METHODS IN ENZYMOLOGY, ACADEMIC PRESS, US, vol. 343, 1 January 2002 (2002-01-01), pages 476-492, XP008029607, ISSN: 0076-6879, DOI: 10.1016/S0076-6879(02)43153-9

## Description

The present invention is drawn to an algorithm and method using the same algorithm for *in vitro* predicting the probability of a drug or a compound to induce a particular effect in a patient, said method using at least one target exhibiting an A-to-I editing of RNA. The present invention also relates to kits for the implementation of the method.

Mental disorders increasingly weight on health systems worldwide (1). They are common disorders in western societies and affect 1 out of 5 individuals at least once in their lifetime. Psychiatric disorders are caused by perturbed molecular pathways that affect brain circuitries, neurotransmission and neural plasticity. Recent work shows that alterations of epigenetic modifications on DNA and RNA such as methylation, acetylation and deamination are associated with for instance major depression, bipolar disorder and schizophrenia (2, 3). Recent studies also shed light on the importance of editing enzymes that catalyse adenosine deamination on RNA (A-to-I editing of RNA). This specific mechanism has been shown to directly regulate the function of genes encoding essentially for highly conserved neurotransmitters and synapse related factors (4-7). Importantly, the role in health and disease of this RNA editing machinery and cognate ADARs enzymes (Adenosine Deaminases Acting on RNA), has recently gained deeper ground by the accumulating evidence of its deregulation in brain of patients suffering from psychiatric disorders (8, 9). ADARs act on double stranded pre-mRNAs stem loops to specifically deaminate preferential adenosine residues. Deamination of residues residing in the coding sequence will lead to amino acid substitutions that produce receptor variants with different pharmacological properties (e.g. serotonin 2c receptor, glutamate receptor) (10).

Anomalies of serotonin biology in brain have been proposed to be a characteristic trait underlying depression and/or suicidal behaviour (11-13). By analyzing postmortem brain tissue of suicide victims, we and others have observed distinct alterations of the RNA editing activity on the serotonin receptor 2C (5HT2cR) pre-mRNA, known to greatly impair 5-HT2CR pharmacological properties (10, 14). Interestingly, these alterations in 5-HT2cR mRNA editing profile in human cortex of suicide victims partly overlaps with the interferon-induced changes observed in SH-SY5Y cells. We pinpointed specific biomarkers to characterize an 'RNA editing signature' of 5-HT2cR linked to depressed/suicide patients.

For example, the PCT patent application document published under the number WO2010/070074 (Dinah Weissmann et al.) can be cited which discloses in vitro methods for the determination of the potential toxicity of test compounds and for the selection of therapeutic compounds useful for the treatment of pathology related to an alteration of the mechanism of the mRNA editing of ADAR dependent A to I mRNA editing of the 5-HTR2cR.

The PCT patent application document published under the number WO2011/161253 (Dinah Weissmann et al.) can be also cited which discloses the use of the editing profile of PDE8A pre-mRNA as a specific bio marker of ADARs activities in Human tissues and in vitro method for predicting alteration of the mechanism of the ADARs catalysed pre-mRNA editing of target genes, by analysing the PDE8A pre-mRNA editing profile in blood sample.

Several drugs belonging to different therapeutic classes have been reported to potentially induce severe psychiatric adverse effects, notably depression and suicidality (15-18). Today, there is no approved test to identify such molecules and the Food and Drugs Administration (FDA) can only issue general alerts concerning whole therapeutic classes.

Thus there is a need to provide with *in vitro* test which can determine with high accuracy and with high discriminate power the risk of a drug or a candidate drug to induce adverse side effects

We validated a previously designed innovative in vitro assay that predicts drug-induced psychiatric side effects using a carefully selected cell line (SH-SY5Y). We screened over 260 market-approved compounds to examine drug-induced alterations of 5-HT2cR editing. Compounds were selected from a wide range of therapeutic classes (antidepressant, antipsychotic, antiobesity, antiviral, antiinflammatory, antifungic, antiepileptic, mood stabilizing agents and others), known to potentially induce suicidality (having a FDA warning label and/or numerous case reports) or not (no psychiatric side effects reported). The data was used to identify 'at risk' compounds with high specificity and sensitivity.

In a first aspect, the present invention is directed to an algorithm for *in vitro* predicting the probability of a compound, particularly a drug to induce a or particular effects in a patient, wherein said algorithm is obtained by a method comprising the steps of:

a)

- selecting at least one target exhibiting an A-to-I editing of RNA, the pre-mRNA of which being the substrate of ADARs enzymes (Adenosine Deaminases Acting on RNA), the action of said ADARs leading to the production of different isoforms/or sites,
- selecting at least one cell line which endogenously expresses said at least one target and at least the ADAR enzymes,
- selecting a positive control compound capable of dose-dependently altering the relative proportion of said target isoform(s)/or editing site(s) when cells of said cell line are treated with said positive control,
- selecting a collection of molecules composed of a ratio of drugs or compounds annotated with a risk score to induce said particular effects,

b) treating cells of said cell line with each single molecule of said collection of molecules, along with a negative control and said positive control,
c) analysing said at least one target RNA editing profile in each sample that have been treated with a molecule of the collection, in order to obtain the proportion of RNA editing level of said target for each of its editing isoforms/or sites and for each of the molecules of said collection,
d)

-i) by an univariable analysis statistical method, evaluating for each isoform/or editing site its accuracy and its power to discriminate the risk of a molecule to induce said particular effects; and/or
-ii) by a multivariable analysis statistical method, evaluating for each combination of isoforms/or editing sites, its accuracy and its power to discriminate the risk of a molecule to induce said particular effects, and
-iii) selecting the combination exhibiting the best discriminative performance,

e) building an algorithm using said selected combination of isoforms/or editing sites, and use said algorithm thus obtained for predicting the probability of a drug, compound or molecule to induce said particular effects in a patient.

By compounds, it is intended in the present description to designate mineral, chemical or biological compound, particularly which can be active on a human, animal patient, or in a plant.
In the present description, the wording "patient" also includes plant
The term "algorithm" also include statistical model (such as the Cart model).
In a preferred embodiment, in said algorithm according to the present invention said particular effects, or effect, are side effects, preferably selected from adverse or desired side effects, preferably adverse side effects.
In a preferred embodiment, said target exhibiting an A-to-I editing of RNA is selected from the group consisting of 5-HT2cR, PDE8A (Phosphodiesterase 8A), GRIA2 (Glutamate receptor 2), GRIA3, GRIA4, GRIK1, GRIK2, GRIN2C, GRM4, GRM6, FLNB (Filamin B), 5-HT2A, GABRA3 (GABA$\alpha$3), FLNA, CYFIP2.
In a preferred embodiment, said particular effects, preferably side effects, more preferably desired or adverse side effects, are selecting from the group comprising cardiovascular, allergology, CNS, particularly psychiatric, dermatology, endocrinology, gastroenterology, hematology, infectiology, metabolism, neuromuscular, oncology, inflammatory and obesity, adverse side effects.
More preferred is the psychiatric adverse side effects.
In a preferred embodiment, the cell of said cell line according to the algorithm of the invention is from cell line which endogenously expressing said target and ADAR(s).
More preferably, said cell line is selected in the group consisting of:

- human or animal cell line capable of endogenously expressing said target and displaying ADAR enzymes expression steady state similar to the one observed in human cortex,
- neuroblastoma cell lines, preferably human cells lines,
- neuroblastoma cell lines for which the positive control induced ADAR1a expression with a fold induction of at least 4, preferably at least 5 or 6 when normalised to negative or vehicule controls, and
- the human SH-SY5Y cell line.

In a preferred embodiment, in step b) of the algorithm according to the present invention, the cells of said cell line are treated during a period of time comprised between 12 h and 72 h, more preferably during 48 h +/- 4 h with the molecule or control to be tested, 48 h is the most preferred.
In a preferred embodiment, in the algorithm according to the invention, said positive control is the interferon alpha, or a compound able to reproduce the Interferon RNA editing profile curve at 100 IU/ml (as shown for example in figure 6) The SH-SY5Y human neuroblastoma cell line was used because it endogenously expresses the 5-HT2cR mRNA and displays an ADAR enzymes expression steady state similar to the one observed in human cortex interferon alpha.
In a preferred embodiment, in the algorithm or the model according to the invention, the step c) comprises a step of determining the basal level of the RNA editing for each isoform or site in said cell line compared to vehicle treated control cells, in order to obtain for each molecules and each editing isoforms or editing site the mean/median relative proportion of RNA editing level of said target.
Preferably, said vehicle treated control cells are DMSO treated control cells.
In a preferred embodiment, in the algorithm or the model according to the invention, said method is a method for in vitro predicting the probability of a compound, particularly a drug to induce said particular effects, or effect, preferably side effects, preferably selected from adverse or desired side effects, preferably adverse side effects, with no or a low risk or a high risk, preferably with no risk or a high risk.

In a particular preferred embodiment, in the algorithm or the model according to the invention, said collection of molecules is composed of an equilibrated ratio of molecules annotated with a high risk and very low risk, preferably no risk, score to induce said particular effects, or effect, are side effects, preferably selected from adverse or desired side effects, preferably adverse side effects.

By an "an equilibrated ratio of molecules" it is intended to designate a collection of well annotated molecule for said desired adverse side effects, known to be at no or low risk or high risk to induce said adverse side effects, and presenting at least 3, preferably at least 4 or 5, different therapeutic classes, particularly selected from the group of cardiovascular, allergology, CNS, particularly psychiatric, dermatology, endocrinology, gastroenterology, hematology, infectiology, metabolism, neuromuscular, oncology, inflammatory and obesity therapeutic classes.

Preferably, the number of molecules including in each of said at least 3, 4, 5, 6, 7, or 8 different therapeutic classes, represent at least 10 % of the total of the molecules of the collection.

In a more preferred embodiment, the therapeutic class representing the class of the desired particular effects, or effect, preferably side effects, preferably selected from adverse or desired side effects, preferably adverse side effects includes more than 20 %, preferably, 25 %, 30 % or 35 % of the total of the molecules of the collection.

In a preferred embodiment, in the algorithm according to the invention, in step c) said collection of molecules is analysed simultaneously, preferably at different concentrations for each molecules of the collection

In a preferred embodiment, in the algorithm according to the invention, step 1)d)i) comprises a step of calculating for each isoforms or sites, or a combination thereof:

- the optimal threshold of sensitivity (Se %), of at least 60%, preferred 70% and preferably above 80% and specificity (Sp%) of at least 60%, preferred 70% and preferably above 80% for said particular effects, or effect, preferably side effects, preferably selected from adverse or desired side effects, preferably adverse side effects adverse side effect;
- the positive (PPV, %) and negative (NPV, %) predictive values to evaluate the proportion of true presence [true positive /(true positive+ false positive] and true absence [true negative /(true negative+ false negative)], said method allowing the determination of the global performance of the choice of said isoform(s)/or site(s) or the combination thereof.

In a preferred embodiment, in the algorithm or the model according to the invention, in step c), the RNA editing profile is carried out by a method including:

- NGS method (Next-Generation-Sequencing) comprising NGS library preparation, preferably using a 2-step PCR method to selectively sequence the sequence fragment of interest (comprising the editing site(s)) of the target(s);
- the sequencing of all the NGS libraries obtained; and, optionally
- the bioinformatics analysis of said sequencing data, said bioinformatics analysis preferably comprising the steps of:
    - pre-alignment processing and quality control of the sequences
    - the alignment against reference sequence; and
    - the editing levels calling,

to obtain the editing profile of the target.

In a preferred embodiment, in the algorithm according to the invention, in step d) i) and d) ii), and in step e), said statistical method allowing the obtaining of said algorithm is carried out by a method including:

- mROC program, particularly to identify the linear combination, which maximizes the AUC (Area Under the Curve) ROC and wherein the equation for the respective combination is provided and can be used as a new virtual marker Z, as follows: $Z = a_1$ . (Isoform 1) + $a_2$ . (Isoform 2) + ...$a_i$ . (Isoform i) +....$a_n$. (Isoform n) where $a_1$ are calculated coefficients and (Isoform i) are the relative proportion of individual RNA editing level of isoform's target; and/or
- a logistic regression model applied for univariate and multivariate analysis to estimate the relative risk of molecules at different isoform(s)/or editing site(s) values; and/or
- a CART (Classification And Regression Trees) approach applied to assess isoform(s)/or editing site(s) combinations; and/or
- a Random Forest (RF) approach applied to assess the isoform/or editing site combinations, particularly to rank the importance of editing isoform/or site and to combine the best isoforms/or editing sites to classify the "relative risk" of molecule, and/or optionally
- a multivariate analysis applied to assess the isoforms/or editing sites combination for the "relative risk" of molecules selecting from the group consisting of as

- Support Vector Machine (SVM) approach;
- Artificial Neural Network (ANN) approach;
- Bayesian network approach;
- wKNN (weighted k-nearest neighbours) approach;
- Partial Least Square - Discriminant Analysis (PLS-DA); and
- Linear and Quadratic Discriminant Analysis (LDA / QDA).

In a preferred embodiment, in the algorithm according to the invention,

- said at least one target is the 5-HT2cR, and
- said adverse side effects are psychiatric adverse side effects, and-
- the cell line is the human SH-SY5Y neuroblastoma cell line,and
- the positive control is the interferon alpha, and

and wherein:

- the sites combination capable of discriminating whether the test drug is at low risk or high risk to induce said psychiatric adverse side effects comprises at least a combination of at least 2, 3, 4 or 5 of the single sites selected from the group constituted of the following 5-HT2cR, sites:

  A, B, C, D, and E,
  preferably a combination of at least 3, 4 or 5 of said sites,

- or the isoforms combination capable of discriminating whether the test drug is at low risk or high risk to induce said psychiatric adverse side effects comprises at least a combination of at least 2, 3, 4, 5, 6, 7, 8, 9, 10, 11, 12 or 13 of the single isoforms selected from the group constituted of the following 5-HT2cR, isoforms:

  A, B, AB, ABC, AC, C, D, AD, AE, ACD, AEC, ABCD and NE,
  preferably a combination of at least 5, 6 or 7 of said isoforms,

  and, optionally, wherein:
  said statistical method allowing the obtaining of said algorithm or model is carried out by a method including:
- mROC program, Random Forest approach and/or Cart algorithm.

In a second aspect, the present invention is directed to an in vitro method predicting the probability or the risk of a drug, a compound or a molecule, to induce particular effects in a patient, preferably side effects, more preferably adverse or desired side effects, said method using as a target exhibiting an A-to-I editing of RNA, the pre-mRNA of which being the substrate of ADARs enzymes, the action of said ADARs leading to the production of different isoforms or editing sites, wherein said method comprises the steps of:

A) Analysing the target RNA editing profile in sample that have been treated with said drug or compound or molecule, in order to obtain the proportion of RNA editing level of said target for each of its editing isoforms, and,
wherein said target RNA editing profile is obtained as obtained for a molecule of the collection of molecule in the algorithm or the model according to one of claims 1 to 15 obtained for said particular effects;
B) calculating the end value or applied the algorithm or model obtained for said drug or compound using the algorithm or model obtained for said target and said particular effects according to one of claims 1 to 15; and
C) determining whether said drug or compounds is at risk, particularly at low risk versus high risk, to induce said particular effects in a patient in view of the results obtained in step B).

In another embodiment, said in vitro method predicting the probability or the risk of a drug, a compound or a molecule, to induce particular effects in a patient according to the present invention, uses a combination of at least 2, 3 or 4 targets exhibiting an A-to-I editing of RNA, the pre-mRNA of which being the substrate of ADARs enzymes, the action of said ADARs leading to the production of different isoforms or sites, wherein said method comprises the steps of:

A) Analysing each of the targets RNA editing profile of said targets combination in sample that have been treated with said drug or compound or molecule, in order to obtain the proportion of RNA editing level for each of said targets for each of its editing isoforms or sites, and,
wherein said each of said targets RNA editing profile is obtained as obtained for a molecule of the collection of molecule in the algorithm or the model according to one of claims 1 to 15 obtained for said particular effects;
B) calculating the end value or applied the algorithm or model obtained for said drug or compound using the algorithm or model obtained for such of said targets and said particular effects according to one of claims 1 to 15; and
C) determining whether said drug or compounds is at risk, particularly at no risk or low risk versus high risk, to induce said particular effects in a patient in view of the results obtained in step B).

In another preferred embodiment, said combination of at least 2, 3 or 4 targets exhibiting an A-to-I editing of RNA, the pre-mRNA of which being the substrate of ADARs enzymes target exhibiting an A-to-I editing of RNA is selected from a combination of targets selected from the group consisting of 5-HT2cR, PDE8A (Phosphodiesterase 8A), GRIA2 (Glutamate receptor 2), GRIA3, GRIA4, GRIK1, GRIK2, GRIN2C, GRM4, GRM6, FLNB (Filamin B), 5-HT2A, GABRA3 (GABA$\alpha$3), FLNA, CYFIP2.

In a third aspect, the present application is directed to a kit for determining whether a compound, preferably a drug is at risk, particularly at low risk versus high risk, to induce said particular effects, or effect, preferably side effects, preferably selected from adverse or desired side effects, preferably adverse side effects adverse side effect adverse side effects in a patient comprising:

1) instructions for using an algorithm according to the invention, or to applied the method for predicting the probability or the risk of a compound or preferably a drug to induce said particular effects, or effect, preferably side effects, preferably selected from adverse or desired side effects, preferably adverse side effects in a patient according to the invention, in order to obtain the end value the analysis of which determining the risk to induce said adverse side effects in a patient for said test drug, said instructions comprising optionally a ROC curve or a Cart decision tree ; and
2) reagents for determining the editing RNA profile obtained for said test drug according to the reagents need for obtaining the editing RNA profile for each molecules of the collection of molecules used for determining said algorithm or said model of said instructions of 1).

In a preferred embodiment, said reagents include the set of primers necessary for the 2-step PCR for NGS libraries preparation when using this method in the algorithm or model of the present invention.

In a more preferred embodiment, said reagents include oligonucleotides sequences used for obtaining RNA editing profile according to claims 1 to 17 for at least one of said targets or for a combination of at least 2, 3 or 4 targets.

In a more preferred embodiment, said reagents include one or a combination of a set of primers necessary for the 2-step PCR for NGS libraries preparation and wherein said at least one target or said combination of targets is selected from targets selected from the group consisting of 5-HT2cR, PDE8A (Phosphodiesterase 8A), GRIA2 (Glutamate receptor 2), GRIA3, GRIA4, GRIK1, GRIK2, GRIN2C, GRM4, GRM6, FLNB (Filamin B), 5-HT2A, GABRA3 (GABA$\alpha$3), FLNA, CYFIP2.

In another more preferred embodiment, said reagents include one or a combination of a set of primers selected from the group consisted of:

- for PDE8A target

  PDE8A_left: 5'-CAACCCACTTATTTCTGCCTAG-3' (SEQ ID NO. 1)
  PDE8A_Right: 5'-TTCTGAAAACAATGGGCACC-3' (SEQ ID NO. 2);

- for FNLB target

  FLNB_Left: 5'- AAATGGGTCGTGCGGTGTAT-3' (SEQ ID NO. 3)
  FLNB_Right: 5'- CCTGCTCGGGTGGTGTTAAT-3' (SEQ ID NO. 4);

- for GRIA2 target

  GRIA2_Left: 5'- CTCTTTAGTGGAGCCAGAGTCT-3' (SEQ ID NO. 5)
  GRIA2_Right: 5'- TCCTCAGCACTTTCGATGGG-3' (SEQ ID NO. 6);

- for GRIK2 target

  GRIK2_Left: 5'-CCTGAATCCTCTCTCCCCTG-3' (SEQ ID NO. 7)
  GRIK2_Right: 5'-CCAAATGCCTCCCACTATCC-3' (SEQ ID NO. 8); and

- for GABRA3 target

GABRA3_Left: 5'- ccaccttgagtatcagtgcc-3' (SEQ ID NO. 9)
GABRA3_Right: 5'- cgatgttgaaggtagtgctgg-3' (SEQ ID NO. 10).

The following examples and the figures and the legends hereinafter have been chosen to provide those skilled in the art with a complete description in order to be able to implement and use the present invention.
Other characteristics and advantages of the invention will emerge in the remainder of the description with the Examples and Figures, for which the legends are given herein below.

**Figure legends:**

**Figure 1:** Interferon alpha-induced RNA editing (dose response) (IFN$\alpha$) 5-HT2cR mRNA editing 'profile' in SH-SY5Y human neuroblastoma cell line.
Dose-response analysis of the effect of interferon alpha (IFN$\alpha$) after 48 hours treatment with FN$\alpha$. The relative proportion of 5-HT2cR mRNA was analysed by NGS-based sequencing. The profile was obtained by subtraction of the relative proportion of 5-HT2cR mRNA editing in vehicle treated control cells to the relative proportion of 5-HT2cR mRNA editing measured in IFN$\alpha$ treated cells.
**Figures 2A-2B:** Chart Pie of the therapeutic classification of all 260 compounds tested in the in vitro assay. Further subclassification of the central nervous system (CNS) acting compounds is shown in part B of the figure.
**Figure 3:** Schematic representation of the experimental setup and approach applied during the testing of the selected molecules. All 260 compounds have been tested in five biological independent replicates. Each individual cell culture plate was treated with 10 molecules, a vehicle control (DMSO) as well as with 100IU/ml interferon alpha. Five independent biological replicates were tested generating exactly 1620 samples that have been processed in identical manner through the NGS-based RNA editing quantification method.
**Figures 4A-4I:** ADAR1a mRNA expression in each individual well
Quantitative PCR (qPCR) analysis of ADAR1a expression in SH-SY5Y cells treated with the molecules for 48 hours. ADAR1a mRNA expression levels have been quantified in each sample after 48 hours of treatment with the molecule, vehicle (DMSO) or IFN$\alpha$. A single biological replicate (n=1) is shown. As expected, each well treated with IFN$\alpha$ displayed increased ADAR1a expression **(A to J).** Of note, molecule 165 also displayed strong increase of ADAR1a mRNA expression levels post exposure to the molecule.
**Figures 5A-5B:**
Raw Data of all vehicle controls and IFN$\alpha$.-treated (100UI/ml) SH-SY5Y cells **(A)** Global analysis of all 150 vehicle controls (DMSO) and IFN treated wells. (A) The tables are displaying all basic statistical characteristics of all 5-HT2cR mRNA editing isoforms. Vehicle and IFN$\alpha$ treated conditions obtained during the entire experiment (n=150) were pooled in the analysis to generate the standard measurement of IFN-induced RNA editing changes on 5-HT2cR. **(B)** Histograms showing most significantly affected 5HT2cR editing isoforms by IFN treatment. Mean, median, standard deviation and coefficient of variation (CV expressed as percentage) is given for vehicle treated (DMSO) and IFN$\alpha$ -treated wells for all 5-HT2cR mRNA editing isoforms.
**Figure 6:** Profil Curve-RNA Editing Curve IFN100
5HT2cR mRNA editing profile obtained by subtraction of the relative proportion of 5-HT2cR mRNA editing in vehicle treated control cells to the relative proportion of 5-HT2cR mRNA editing measured in IFN$\alpha$ treated cells. Mean and median value are given, error bars represent standard error of the mean (sem, n= 150).
**Figures 7A-7B:** Illustrative examples of 5HT2cR mRNA editing profile obtained after 48 hours treatment with respective molecules. Example is given for a set of 4 'at risk' compounds (Aririprazole, Sertraline, Isotretinoin and Taranabant) **(A)** and 4 'low risk' molecules (Lithium, Ketamine, Ondansetron and Ribavirin) **(B).** The IFN reference (in black) is given in each graph. Mean values are given, error bars represent standard error of the mean (sem, n= 5).
**Figure 8:** Illustrative examples of diagnosis potential of most representative 5HT2cR mRNA editing isoforms for discriminating low risk molecules to high risk molecules. Boxplot representation is a convenient way of graphically depicting groups of numerical data through their five-number summaries (the smallest observation, lower quartile (Q1), median (Q2), upper quartile (Q3), and largest observation). Boxplots can be useful to display differences between populations without making any assumptions of the underlying statistical distribution. Wilcoxon sum rank test was used for p-values. The symbol * indicate a p-value $\leq$0,05, ** indicate a p-value $\leq$0,01 and *** indicate a p-value $\leq$0,001.
**Figure 9:** Illustrative example of Receiving-Operating-Characteristic (ROC) curves using a combination of 2 isoforms selected from the group of the 13 isoforms of the figure 15 on molecules dataset (n=143, low risk versus high risk molecules).
Decision rule: Z= 0,121xACD - 0,142xNE.

**Figure 10:** Illustrative example of Receiving-Operating-Characteristic (ROC) curves using a combination of 3 isoforms selected from the group of the 13 isoforms of the figure 15 on molecules dataset (n=143, low risk versus high risk molecules).

Decision rule: Z= -0,1449xC + 0,569xAE - 0,1548xNE.

**Figure 11:** Illustrative example of Receiving-Operating-Characteristic (ROC) curves using a combination of 4 isoforms selected from the group of the 13 isoforms of the figure 15 on molecules dataset (n=143, low risk versus high risk molecules).

Decision rule: Z= 0,0235xAB + 0,1567xACD + 0,3880xAEC - 0,1355xNE.

**Figure 12:** Illustrative example of Receiving-Operating-Characteristic (ROC) curves using a combination of 5 isoforms selected from the group of the 13 isoforms of the figure 15 on molecules dataset (n=143, low risk versus high risk molecules).

Decision rule: Z= 0,016xAB - 0,0563xABC + 0,183xACD + 0,386xAEC - 0,1428xNE.

**Figure 13:** Illustrative example of Receiving-Operating-Characteristic (ROC) curves using a combination of 6 isoforms selected from the group of the 13 isoforms of the figure 15 on molecules dataset (n=143, low risk versus high risk molecules).

Decision rule: Z= 0,0157xAB - 0,0557xABC + 0,0187xD + 0,1817xACD + 0,3883xAEC - 0,1426xNE.

**Figure 14:** Illustrative example of Receiving-Operating-Characteristic (ROC) curves using a combination of 7 isoforms selected from the group of the 13 isoforms of the figure 15 on molecules dataset (n=143, low risk versus high risk molecules).

Decision rule: Z=-0,0505xB + 0,0224xAB + 0,001xD + 0,163xACD + 0,389xAEC - 0,1402xABCD - 0,1385xNE.

**Figure 15:** Illustrative example of Receiving-Operating-Characteristic (ROC) curves using a combination of 13 isoforms on molecules dataset (n=143, low risk versus high risk molecules).

Decision rule: Z= 0,2035xA + 0,1283xB + 0,1979xAB + 0,1147xABC + 0,1860xAC + 0,04331xC + 0,1884xD + 0,1259xAD + 0,7739xAE + 0,4295xACD + 0,4775xAEC - 0,0415xABCD + 0,0245xNE.

**Figures 16A-16C:** illustrative examples of Receiving-Operating-Characteristic (ROC) curves of random forest (RF) algorithm using the combination of 7 isoforms of the figure 14 on molecules'dataset (n=143, low risk versus high risk molecules). ROC curve of all dataset is represented in black line and ROC curve of Test dataset is represented in dotdashed lines (A). Importance (weight) of the isoforms in RF model (B)(C).

**Figures 17A-17C:** Example of Diagnostic Performance with a RF Approach, illustrative examples of Receiving-Operating-Characteristic (ROC) curves of random forest (RF) algorithm using the combination of the 13 isoforms of the figure 15 on molecules dataset (n=143, low risk versus high risk molecules). ROC curve of all dataset is represented in black line and ROC curve of Test dataset is represented in dotdashed lines (A). Importance (weight) of the isoforms in RF model **(B)(C).**

**Figures 18A-18C:** Quantification of the RNA editing activity as measured by additional targets: GRIA2 (A), FLNB (B) and PDE8A (C). In all cases IFN treatment induced an increase in the relative proportion of the edited isoforms as illustrated by the decrease in the non-edited (NE) mRNA.

**Figures 19A-19B:** LN18 (A) and LN229 (B) neuroblastoma cell lines (HTR2C) 5HT2cR mRNA editing profile obtained by subtraction of the relative proportion of 5-HT2cR mRNA editing in vehicle treated control cells to the relative proportion of 5-HT2cR mRNA editing measured in IFN$\alpha$ treated cells in LN18 cells (A) and LN229 cells (B). Mean mRNA editing profiles of 5HT2cR mRNA is given.

**Figure 20:** Prediction y CART Algorithm

Illustrative example of representative decision tree and of diagnostic performance of CART algorithm using 6 isoforms on molecules dataset (n=143, low risk versus high risk molecules).

**Figures 21A-21D:** The RNA editing profiles obtained for two compounds with low or no risk to induce a particular effect in a patient. As example is provided the RNA editing profile obtained with Lidocaine **(A)** and Ondansetron **(B)** compared to vehicle control treated cells. The RNA editing profiles obtained for two compounds with high risk to induce a particular effect in a patient like Reserpine **(C)** and Fluoxetine **(D).**

**Figures 22A-22C:** Time course analysis of RNA editing changes observed by Aripiprazole **(A),** Interferon **(IFN)(B)** and Reserpine **(C)** on HTR2C.

**Figures 23A-23C:** Dose-dependent alterations of RNA editing profiles after treatment of SH-SY5Y cells with three different compounds: Clozapine **(A),** Sertraline **(B)** and Ketamine **(C).**

**EXAMPLE 1: Material and Methods**

Creation of a database for drug-induced psychiatric adverse side-effects

A chemical library containing a collection of 1280 small molecules dissolved in DMSO at precisely 10mM was purchased from Prestwick Chemicals. All the small molecules contained in the library are 100% approved drugs (FDA, EMA and other agencies), present the greatest possible degree of drug-likeness and have been selected for their high chemical and pharmacological diversity as well as for their known bioavailability in humans. At purchase of the chemical library (Prestwick Chemicals), a highly annotated database was provided containing detailed information on target, therapeutic class/effect, patent and ADMET of each single molecule. We searched for reports emitted for suicide and depression related adverse side effects of the drugs when prescribe to humans by inquiring databases that regularly update safety information and case reports (such as FDA Medwatch, EMEA, ...). Next, we compiled results of the queries and attributed a risk score to each drug contained in the chemical library. The scoring system was established in order to quantify the risk of the drugs to potentially induce adverse psychiatric side effects (depression and/or suicide related adverse side-effects) taking into account a variety of parameters such as number of cases reporting suicide and/or depression related adverse side effects, extent of prescription of the drug, being on the list of essential drugs according to the WHO and many more. We obtained a comprehensive database with specific information regarding risk to induce adverse psychiatric side-effects.

Cell culture

The SH-SY5Y human neuroblastoma cell line was used because it endogenously expresses the 5-HT2cR mRNA and displays an ADAR1 enzymes expression steady state similar to the one observed in human cortex (Cavarec et al. 2013, Weissmann et al. 2016 Translational Psychiatry, Patent TOXADAR). The SH-SY5Y human neuroblastoma cell line was purchased from Sigma Aldrich. Cells were routinely cultured in standard conditions at 37°C in a humified atmosphere of 5% CO2. Dialysed Foetal Bovine Serum (FBS Science Tech reference number FB-1280D/500) was preferred to non-dialyzed because of desensitisation and down-regulation of the 5-HT2cR mRNA expression by serotonin often present in serum (Saucier et al. 1998). During the course of the experiments cells were cultured between passage number P8 and P22. Prior seeding of the cells into the 12 wells cell culture plate, estimation of the number of cells was performed by two independent loading of the trypsinized cell suspension into the Kovaslide (Kova International) chamber, a disposable microscope slide made of optically clear plastic with a hemocytometer counting grid. Both chambers were counted by two laboratory technicians and the average of the four independent counting results was further used for calculation of cell number and plating of the 12-wells cell culture plates.

Pharmacological treatment and cell lysis

Upon receipt, the entire Prestwick chemical library was transferred to individual tubes, codified, aliquoted and stored at -80°C until further use. From our in-house generated drug-induced psychiatric adverse side-effects database we selected 260 molecules composed of an equilibrated ratio of drugs annotated with a high risk and very low risk score. The drugs were codified and care was taken to randomly process the molecules throughout the experimental setup. All 260 molecules were analysed simultaneously in each experiment along with a negative control (the vehicle DMSO) and a positive control (Interferon alpha). On each 12-well cell culture plate a negative control and a positive control was added leaving 10 vacant positions for testing molecules. In turn, each single replicate consisted of 27 culture plates of 12 wells (ref). The experiment was repeated five times in an exactly similar manner as such generating five independent biological replicates (n=5) for each tested molecule. Over the course of the experiment a total of 1620 samples were generated i.e. 27 (number of well plates) x12 (number of wells per plate) x5 (number of replicates). A preliminary experiment allowed identifying 7 molecules that were lethal for the SH-SY5Y cells at 10μM. For these molecules the concentration was adapted and lowered until reduced toxicity could be detected. Prior experimentation, all dilutions of molecules and controls were prepared and arranged in racks. Cell density, morphology, viability and contamination of all 324 wells (27x12 wells) were controlled by microscope prior treatment. Additionally, a picture of each well was taken using a Canon EOS700 digital camera. Exactly 48 hours after treatment of the cells with the molecules a picture of each well was taken using the defined parameters with the digital camera. After carefully removing the growing medium 350μl of RLT lysis buffer (Qiagen) containing 1% beta-mercaptoethanol was added for complete chemical lysis of the cells. The 12-well plates were stacked and stored in the freezer until RNA extraction.

Total RNA extraction, quality control and reverse transcription

Total RNA extraction was carried out following manufacturer's guidelines (Qiagen). The RNeasy Mini Kit provides fast purification of high-quality RNA from cells using silica-membrane RNeasy spin columns. All cell lysates were extracted using the fully automated sample preparation QIAcube. The extractions were processed using a standard procedure in batches of 12 samples (one complete 12-wells plate) per run, using appropriate protocol. During sample preparation and RNA extraction, standard precautions were taken to avoid RNA degradation by RNAses. All extracted RNA samples were analysed by labChipGx (Perkin Elmer) to both quantify and qualify the total RNA. Fluorescent-based quantification by Qubit was also performed to validate LabChipGx data. The RNA Quality Score (RQS score) was determined for each individual sample (Average RQS score of the 1620 samples = 9.6/10). Next, samples were normalised and reverse transcription of the purified RNA was performed using the Takara kit (PrimeScript RT Takara ref#RR037A) was performed starting from 1μg RNA material in a 20μl final reaction volume. The cDNA synthesis was performed at 42°C on a Peqstar 96x thermocycler for 15 minutes and reaction mixes were kept at 4°C until further use.

Relative mRNA expression by quantitative PCR (qPCR)

After cDNA synthesis samples were stored at 4°C prior analysis of ADAR1a mRNA expression by qPCR on a LC480 system (Roche). qPCR data were quantified using the standard curve method. mRNA expression of ADAR1a is known to be induced by Interferon alpha treatment (IFNα). As expected all samples that have been treated with IFNα for 48 hours displayed an increase of ADAR1a expression with a fold induction of gene expression between 6 and 7. In addition, Reserpine treatment did also consistently increase ADAR1a mRNA levels.

NGS Library preparation

For NGS library preparation a 2-step PCR method was employed in order to selectively sequence exon V of the 5-HT2cR previously described and confirmed by us and others to be subjected to RNA editing. Validated PCR primers were used to amplify the region of interest by PCR. For PCR amplification the Q5 Hot Start High Fidelity enzyme (New England Biolabs) was used according to manufacturer guidelines (ref#M0494S). The PCR reaction was performed on a Peqstar 96x thermocycler using optimised PCR protocol. Post PCR, all samples were analysed by LabChipGx (Perkin Elmer) and both quantity and quality of the PCR product was assessed. Purity of the amplicon was determined and quantification was performed using fluorescent based Qubit method. After quality control, the 96 PCR reactions (microplate) were purified using magnetic beads (High Prep PCR MAGbio system from Mokascience). Post purification DNA was quantified using Qubit system and purification yield was calculated. Next, samples were individually indexed by PCR amplification using Q5 Hot start High fidelity PCR enzyme (New England Biolabs) and the Illumina 96 Indexes kit (Nextera XT index kit; Illumina). Post PCR, samples were pooled into a library and purified using Magbio PCR cleanup system. The library was denatured and loaded onto a sequencing cartridge according to Illumina's guidelines for sequencing FASTQ only on a MiSeq platform. A pool of plasmid containing determined amounts of 5HT2cR isoforms was included in each library to control for sequencing quality and error in each sequencing run. In addition, a standard RNA pool was incorporated into the libraries to determine variability between different sequencing flow cells during the course of the experiment. To sequence all 1620 samples, 18 MiSeq Reagent kits V3 were required (Illumina). All NGS libraries were sequenced at 14pM and 10% Phix (PhiX Control V3) was spiked in to introduce library diversity.

**EXAMPLE 2: Bioinformatics analysis of sequencing data**

1. Pre-alignment processing and quality control of Fastq sequences

The sequencing data was downloaded from the Miseq sequencer (Illumina) as fastq file. To evaluate sequencing quality, an initial quality of each raw fastq file was performed using FastQC software version 0.11.5. A pretreatment step was performed consisting of removing adapter sequences and filtering of the sequences according to their size and quality score (all short reads (<50nts) and reads with average QC <30 were removed). Next, to facilitate and improve the quality of alignment of the sequences a flexible read trimming tool for Illumina NGS data was used (trimmomatic programs version 0.35). After pre-processing steps were performed an additional quality control of each cleaned fastq file was carried out prior further sequence processing.

2. Alignment against reference sequence

Alignment of the processed reads was performed using bowtie2 version 2.2.5 with end-to-end sensitive mode. The alignment was done to the latest annotation of the human genome sequence (UCSC hg38) and reads multiple alignment regions, reads with poor alignment quality (Q<40) or reads containing insertion/deletion (INDEL) were taken out of the further analysis. Filtering of file alignment was carried out with SAMtools software version 1.2 that provide various utilities for manipulating alignments in the SAM format, including sorting, merging, indexing and generating alignments in a per-position format.

3. Editing levels calling

Next, SAMtools mpileup was used to pileup obtained alignment results data from multiple samples simultaneously. An in-house script was run to count the number of different ATGC nucleotides in each genomic location ('base count'). So, for each genomic location, the home-made script computes the percentage of reads that have a 'G' [Number of 'G' reads/ (Number of 'G' reads + Number of 'A' reads)*100]. The genomic location 'A' reference with percentage in 'G' reads > 0.5 are automatically detected by the script and are considered as 'A-to-I edition site'. The last stage was to compute the percentage of all possible combinations of 'A-to-I edition site' previously described to obtain the editing profile of the target.

4. Comparison between baseline and molecule editing profile of target

We have analysed the 5HT2cR RNA editing profile of an extensive set of molecules (n=260). To compare molecules together, we have, in a first step, determined the basal level of the RNA editing of our target for each isoform/or sites in SH-SY5Y human neuroblastoma cell line compared to vehicle treated (DMSO) control cells. For this, we calculated, example given, the average of RNA editing level of 5HT2cR from over 150 vehicle independent experiments (replicates). Secondly, an in-house script has automatically computed the deviation of each replicates of molecule (n=5) to the control reference (CTRL).

Finally, for each molecules and each editing isoforms/or sites we obtained the mean/median relative proportion of RNA editing level of the target.

**EXAMPLE 3: Statistical analysis**

All statistics and figures were computed with the "R/Bioconductor" statistical open source software (19, 20). RNA editing values are usually presented as means ± standard error of the mean (SEM). A differential analysis was carried out with the non-parametric Wilcoxon rank sum test and the Welch's t-test. With the multiple testing methodologies, it is important to adjust the p-value of each editing isoforms (as example: 32 RNA editing isoforms including the non-edited isoform (Ne) for 5HT2cR from 5 editing sites (A,B,C,E,D)) to control the False Discovery Rate (FDR). The Benjamini and Hochberg (BH) procedure (21) was applied on all statistical tests with the "multtest package" and an adjusted p-value below 0.05 was considered as statistically significant. Relative proportion of editing levels was normally distributed and consequently no normalization was applied. All data distributions are illustrated as medians and barplots or boxplots for each significant isoforms. An editing profile curve from significant isoforms and representing the RNA editing level of 5HT2cR in SH-SY5Y human neuroblastoma cell line are also shown for each molecule. A Pearson test correlation was applied to identify isoforms correlation for all molecules groups.

The 5HT2cR editing isoform diagnostic performance could be characterised by: sensitivity, which represents its ability to detect the 'high risk molecule' group and specificity which represents its ability to detect the 'no or low risk molecule' group. The results of the evaluation of a diagnostic test can be summarised in a 2×2 contingency table comparing these two well-defined groups. By fixing a cut-off, the two groups could be classified into categories according to the results of the test, categorised as either positive or negative. Given a particular isoform, we can identify a number of molecules with a positive test result among the "high risk" group (the "True Positive": TP) and b molecules with a positive test result among the "low risk" group (the "True Negative": TN). In the same fashion, c molecules with a negative test result among the 'high risk' group (the "False Positive": FP) and d molecules with a negative test result among the 'low risk' group (the "False Negative": FN) are observed. Sensitivity is defined as TP/(TP+FN); which is herein referred to as the "true positive rate". Specificity is defined as TN/(TN+FP); which is herein referred to as the "true negative rate".

The accuracy of each 5HT2cR editing isoform and its discriminatory power was evaluated using a Receiving Operating Characteristics (ROC) analysis. ROC curves are the graphical visualization of the reciprocal relation between the sensitivity (Se) and the specificity (Sp) of a test for various values.

In addition, all 5HT2cR editing isoforms were combined with each other to evaluate the potential increase in sensibility and specificity using several approaches as mROC program [Comput. Methods Programs Biomed. 2001; 66:199-207], logistic regression (22) and with two supervised learning algorithms, CART (23) and RandomForest (24).

**mROC** is a dedicated program to identify the linear combination (25, 26), which maximizes the AUC (Area Under the Curve) ROC (27). The equation for the respective combination is provided and can be used as a new virtual marker Z, as follows:

$$Z = a \text{ x Isoform1} + b \text{ x Isoform2} + c \text{ x Isoform3},$$

where a, b, c are calculated coefficients and Isoform 1,2,3 are the relative proportion of individual RNA editing level of isoform's target.

A combination of 2, 3 or 4 targets can be combined with each other to evaluate the potential increase in sensibility and specificity using a multivariate approaches as for example mROC program or logistic regression. An equation for the respective combination can be calculated and can be used as a new virtual marker Zn, as follows:

$$Zn = n_1 \text{ x target1} + n_2 \text{ x target 2} + n_3 \text{ x target3},$$

where $n_1$, $n_2$, $n_3$ ...are calculated coefficients and target 1,2,3 are for example a value correlated with the level of targets.

A **logistic regression** model was also applied for univariate and multivariate analysis to estimate the relative risk of molecules at different isoforms or sites values. We analysed isoforms as both continuous (data not shown) and categorical (using the tertile values as cutpoints) variables. In the last cases, the odds ratio (OR) and their 95% confidence interval are computed. A penalized version of the logistic regression (LASSO, ridge or Elastic-Net approaches) was also applied on continuous variables. For these methods the packages: glmnet version 2.0-3 of R software version 3.2.3 are used.

A CART (Classification And Regression Trees) approach was also applied to assess isoforms combinations. This decision tree approach allows to produce a set of classification rules, represented by a hierarchical graph easily understandable for the user. At each node of the tree, a decision is made. By convention, the left branch corresponds to a positive response to the question of interest and the right branch corresponds to a negative response to the question of interest. The classification procedure can then be translated as a set of rules 'IF-THEN' (see Figure 20 for an example).

A **Random Forest (RF)** approach was applied as previously to assess the isoform combinations. This method combines Breiman's "bagging" idea and the random selection of features in order to construct a collection of decision trees with controlled variance. So, random forests can be used to rank the importance of editing isoform and to combine the best isoforms to classify the "relative risk" of molecule (see Figures16 and 17).

CART and RandomForest are supervised learning methods. These methods require the use of a training set used to construct the model and a test set to validate it. So, we have shared our data set: 2 / 3 of the dataset are used for the learning phase and 1/3 are used for the validation phase. This sharing has been randomized and respect the initial proportion of the various statutes in each sample. To estimate the errors prediction of these classifiers, we used the 10-fold cross-validation method, repeated 10 times in order to avoid overfitting problems. For these approaches, we used thethe "rpart package 4.1-10" and the "randomForest package 4.6-12" of the R software version 3.2.3.

Another multivariate analysis may be used to assess 5HT2cR editing isoforms combination for the "relative risk" of molecules as:

- Support Vector Machine (SVM) approach (28);
- Artificial Neural Network (ANN) approach (29);
- Bayesian network approach (30);
- wKNN (weighted k-nearest neighbours) approach (31);
- Partial Least Square - Discriminant Analysis (PLS-DA) (32);
- Linear and Quadratic Discriminant Analysis (LDA / QDA) (33);
- and more.

**EXAMPLE 4: Results**

Validation of the SH-SY5Y cell-line

Prior to the experiment, the human neuroblastoma cell line (SH-SY5Y) was treated with an increasing dose of interferon and RNA editing of 5HT2cR was measured using NGS based approach. As expected, the relative proportion of the 5HT2cR isoforms is altering and, particularly can, increase dose-dependently (Figure 1), confirming previously described IFN-induced response in this particular cultured cell-line. The IFN profile closely matched previously obtained data using a diametrically different analytical method (34, 35).

Experimental procedure

Only once the cell-line showed stable grow characteristics and responded accordingly to IFN treatment, the screen of 260 molecules was prepared. Based on in-house defined criteria a risk score was attributed to each of the 1280 molecules in the chemical library. For practical reasons 260 molecules were selected to further test on proprietary in vitro assay. During selection procedure of the molecules, care was taken to cover part of all, preferably at least 3, 4, 5, 6 or 7 of the major therapeutic classes, identified in the figure 2, contained in the chemical library (figure 2). Out of the 260 molecules, 112 are prescribed drugs for central nervous system disorders as anticonvulsant, antidepressant and others (figure 2B). All molecules were transferred and aliquoted in appropriate tubes prior treatment. The experimental setup chosen for the screening of the 260 molecules consisted of 26 wells plates (12 wells plate) treated individually with 10 molecules, a vehicle control (DMSO) and 100IU/ml interferon alpha in turn yielding a positive and negative control for each cell culture plate. An additional cell culture plate was used to add additional control wells. Each molecule was tested in 5 biological replicates within 3 weeks interval (Figure 3). Exactly 48 hours of treatment, cells were lysed in appropriate lysis buffer and stored at -20°C until further processing. All RNA extraction were performed using Qiacube automated RNA extraction and plates were processed individually (batches of 12 samples per extraction).

Relative ADAR1a mRNA expression

Following RNA extraction, cDNA was synthesised and ADAR1a expression was assessed on a LC480 lightcycler (Roche) in a 384-micro wells plate. In this way, all samples of the same batch could be analysed in a single qPCR run. An interferon dependent induction of ADAR1a was observed for all IFN treated cells on each 12-wells plate reflecting robustness of the response. Interestingly molecule 165 also induced ADAR1a mRNA expression (Figures 4A-4I, plate 17). This response could be seen in all biological replicates (n=5). As previously observed on SH-SY5Y cells, IFN induced ADAR1a expression with a fold induction of 6.6 when normalised to vehicle controls (Table 1). The coefficient of variation of 9.31% clearly illustrates the reproducibility of the biological phenomenon.

Table 1: Basic statistical characteristics of ADAR1a mRNA expression after IFN treatment in SH-SY5Y cells. Mean fold induction (compared to DMSO treated control cells) standard deviation, median and CV (expressed as percentage).

| | |
|---|---|
| Mean (fold induction) | 6,61 |
| Standard Deviation | 0,62 |
| Median | 6,62 |
| CV(%) | 9,31 |

**A) Univariable analysis of 5HT2cR editing isoforms**

Comparison of IFN RNA editing isoforms to control on SH-SY5Y cells

Post the cDNA synthesis step, a 2-step PCR approach to target exon V of the 5HT2cR was applied to build NGS libraries and accurately quantify the relative proportion of each individual 5HT2cR mRNA in all samples. The mean value of all vehicle controls and IFN treated wells (n=150) is displayed in figure 5A and depicted as a histogram. Clear differences in the relative proportion of the isoform can be observed between the vehicle controls and the IFN-treated conditions (Figure 5B). These data were expressed as an RNA editing profile generating the previously described RNA editing profile (figures 7A-7B) that very closely match previously described profile (see figure 1 and Cavarec et al).

As example, when comparing the levels of 5-HT2cR RNA editing isoforms in the presence of IFN (n=150) to vehicle control (vehicle, n=150) on SH-SY5Y cell lines, AC, ABC, AB, A, AE, ACE, D, ABCD, ABE, C, B, BC and ABCE RNA editing levels of 5-HT2cR were significantly altered (Figures 5A-5B and Figure 6). The level of the non-edited isoforms of 5-HT2cR (Ne) are the most significant for the comparison of IFN molecule to vehicle control (Basa10). Moreover, we observed an increase of levels of 5-HT2cR RNA editing of AC,ABC,AB,A,AE,AEC,ABCD,ABE,C,B,BC and ABEC and a decrease of levels of D and non-edited (Ne) isoforms of 5-HT2cR RNA editing. These results suggest that globally, the RNA editing activity on 5-HT2cR is increased in SH-SY5Y cells in presence of IFN.

Table 2: differential analysis of 5-HT2cR RNA editing levels when comparing IFN molecule (n=150) to control (n=150)

| control (n=150) | | | | | |
|---|---|---|---|---|---|
| **5-HT2cR Editing Isoforms** | **pWILCOX** | **pWILCOX_ FDR** | **pTTest** | **pTTest_FDR** | **fold Change** |
| **Ne** | <0,0001 | <0,0001 | <0,0001 | <0,0001 | 0,62 |
| **AC** | <0,0001 | <0,0001 | <0,0001 | <0,0001 | 2,27 |
| **ABC** | <0,0001 | <0,0001 | <0,0001 | <0,0001 | 3,40 |
| **AB** | <0,0001 | <0,0001 | <0,0001 | <0,0001 | 1,76 |
| **A** | <0,0001 | <0,0001 | <0,0001 | <0,0001 | 1,11 |
| **AE** | <0,0001 | <0,0001 | <0,0001 | <0,0001 | 2,77 |
| **AEC** | <0,0001 | <0,0001 | 0,0001 | 0,0004 | 2,60 |

(continued)

| control (n=150) | | | | | |
|---|---|---|---|---|---|
| **5-HT2cR Editing Isoforms** | **pWILCOX** | **pWILCOX_ FDR** | **pTTest** | **pTTest_FDR** | **fold Change** |
| **D** | <0,0001 | <0,0001 | 0,0002 | 0,0005 | 0,51 |
| **ABCD** | <0,0001 | <0,0001 | 0,0002 | 0,0005 | 3,68 |
| **ABE** | <0,0001 | 0,0001 | 0,0002 | 0,0005 | 8,60 |
| **c** | 0,0075 | 0,0144 | 0,0094 | 0,0094 | 1,20 |
| **B** | 0,0154 | 0,0256 | 0,0183 | 0,0183 | 1,22 |
| **BC** | <0,0001 | <0,0001 | 0,0550 | 0,0550 | 1,84 |
| **ABEC** | 0,0006 | 0,0012 | 0,7873 | 0,8201 | 1,26 |

Comparison of levels of RNA editing isoforms of high risk molecules to low risk molecules on SH-SY5Y cells

As example, when comparing molecules with low risk (n=82) to molecules with high risk (n=61), single editing or non-edited (Ne) levels of 5-HT2cR isoforms can be significantly altered (Figures 7A-7B). Based on Receiving-Operating-Characteristic (ROC) analysis for RNA editing levels of 5-HT2cR isoforms, the area under the curve (AUC) for individual isoforms, allowed discriminating molecules with low or high risk (Table 3).

Table 3: Discrimnative performance of single editing isoform when comparing low risk molecules(n=82) to high risk molecules (n=61)

| | **5-HT2Cr Isoforms** | **AUC ROC** | **CI 95%** | **Threshold** | **Sp (%)** | **Se (%)** | **VPP(%)** | **VPN(%)** |
|---|---|---|---|---|---|---|---|---|
| **1** | NE | 0,845 | [0,777; 0,913] | -1,02 | 90,2 | 68,9 | 84,0 | 79,6 |
| **2** | AC | 0,688 | [0,595; 0,781] | 0,32 | 86,6 | 52,5 | 74,4 | 71,0 |
| **3** | A | 0,683 | [0,592; 0,774] | 1,55 | 84,2 | 49,2 | 69,8 | 69,0 |
| **4** | ABC | 0,61 | [0,516; 0,705] | -0,31 | 46,3 | 73,8 | 50,6 | 70,4 |
| **5** | AB | 0,609 | [0,513 ; 0,706] | 0,27 | 70,7 | 55,7 | 58,6 | 68,2 |

The accuracy of each isoforms and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of relative risk of molecules by calculating optimal threshold of sensitivity (Se %) and specificity (Sp%) for single marker. Positive (PPV, %) and negative (NPV, %) predictive values for single RNA editing isoforms were calculated to evaluate the proportion of true presence [true positive /(true positive+ false positive] and true absence [true negative /(true negative+ false negative)] of high risk molecules in 'suicide side-effect group'.

**B) Multivariable analysis of 5-HT2cR editing isoforms**

Multiple marker analysis with mROC (multiple Receiving-Operating-Characteristic) approach improved significantly AUC when comparing low risk to high risk molecules. The isoforms combination associated for example 2, 3, 4, 5, 6, 7 or the 13 isoforms selected from the group of the 13 isoforms of the following combination: A + B + AB + ABC + AC + C + D + AD + AE + ACD + AEC + ABCD + NE, combination obtained by the method of the present invention, has a predictive value for higher risk of suicide side-effect in high risk molecules as reported by the higher sensitivity and specificity than those obtained in Cavarec et al. (2013). The statistical analysis combining 2, 3, 4, 5, 6, 7, 8, 9, 0, 11, 12 and the 13 isoforms as identified in the combination of the present invention, generated a series of decision rules; a new virtual marker (Z) was calculated for each combination as illustrated in Figures 9 to 15 and the following corresponding Tables 4 to 9 (low risk molecules versus high risk molecules).

The accuracy of multi-isoforms panel and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of high risk of toxicity by calculating optimal threshold of sensitivity (Se %) and specificity (Sp %) for multi-isoforms panel. Positive (PPV, %) and negative (NPV, %) predictive values for combined marker were calculated to evaluate the proportion of true presence [true positive /(true positive+ false positive] and true absence [true negative /(true negative+ false negative)] of high risk molecule of suicide/depression inducing adverse side effects.

Table 4: 5-HT2cR editing Isoforms performance using multivariable analysis with 2 isoforms (low risk versus high risk molecules)

| **C2 (combination of 2 isoforms): Top 10** | | | | | | | | | |
|---|---|---|---|---|---|---|---|---|---|
| **RD** | **Combination C2** | **AUC ROC** | **CI 95%** | **Threshold** | **Sp (%)** | **Se (%)** | **VPP (%)** | **VPN (%)** | **Accuracy** |
| **1** | ACD + NE | 0,845 | [0,776; 0,914] | 0,1252 | 87,8 | 75,4 | 82,1 | 82,8 | 82,5 |
| **2** | AEC+ NE | 0,838 | [0,768; 0,908] | 0,0921 | 82,9 | 78,7 | 77,4 | 84,0 | 81,1 |
| **3** | A+NE | 0,839 | [0,771; 0,908] | 0,0822 | 78,1 | 82,0 | 73,5 | 85,3 | 79,7 |
| **4** | ABC+ NE | 0,84 | [0,771; 0,909] | 0,1703 | 90,2 | 65,6 | 83,3 | 77,9 | 79,7 |
| **5** | B+NE | 0,842 | [0,773; 0,911] | 0,0591 | 76,8 | 82,0 | 72,5 | 85,1 | 79,0 |
| **6** | AC+NE | 0,841 | [0,771; 0,91] | 0,0542 | 76,8 | 82,0 | 72,5 | 85,1 | 79,0 |
| **7** | C+NE | 0,841 | [0,774; 0,909] | 0,0517 | 76,8 | 78,7 | 71,6 | 82,9 | 77,6 |
| **8** | AE + NE | 0,839 | [0,771; 0,907] | 0,0233 | 76,8 | 78,7 | 71,6 | 82,9 | 77,6 |
| **9** | AB+AC | 0,739 | [0,653; 0,824] | -0,0253 | 70,7 | 70,5 | 64,2 | 76,3 | 70,6 |
| **10** | A + ACD | 0,715 | [0,625; 0,804] | 0,1369 | 72,0 | 67,2 | 64,1 | 74,7 | 69,9 |
| Decision rules:<br>RD1: Z= 0,121xACD - 0,142xNE | | | | | | | | | |

Table 5: 5-HT2cR editing Isoforms performance using multivariable analysis with 3 isoforms (low risk versus high risk molecules)

| **C3: Top 25** | | | | | | | | | |
|---|---|---|---|---|---|---|---|---|---|
| **RD** | **Combination C3** | **AUC ROC** | **CI 95%** | **Threshold** | **Sp (%)** | **Se (%)** | **VPP (%)** | **VPN (%)** | **Accuracy** |
| **1** | ACD + AEC + NE | 0,839 | [0,768; 0,909] | 0,0533 | 81,7 | 85,3 | 77,6 | 88,2 | 83,2 |

(continued)

| C3: Top 25 | | | | | | | | | |
|---|---|---|---|---|---|---|---|---|---|
| **RD** | **Combination C3** | **AUC ROC** | **CI 95%** | **Threshold** | **Sp (%)** | **Se (%)** | **VPP (%)** | **VPN (%)** | **Accuracy** |
| **2** | D + ACD + NE | 0,845 | [0,776; 0,914] | 0,1248 | 87,8 | 75,4 | 82,1 | 82,8 | 82,5 |
| **3** | AB+ACD+ NE | 0,848 | [0,779; 0,916] | 0,1418 | 90,2 | 72,1 | 84,6 | 81,3 | 82,5 |
| **4** | AB+AEC+NE | 0,841 | [0,772; 0,91] | 0,0673 | 82,9 | 80,3 | 77,8 | 85,0 | 81,8 |
| **5** | ACD + ABCD + NE | 0,842 | [0,774; 0,911] | 0,1386 | 87,8 | 73,8 | 81,8 | 81,8 | 81,8 |
| **6** | B + AE + NE | 0,837 | [0,767; 0,906] | 0,2614 | 95,1 | 62,3 | 90,5 | 77,2 | 81,1 |
| **7** | AD+ACD+ NE | 0,845 | [0,776; 0,914] | 0,1814 | 91,5 | 67,2 | 85,4 | 79,0 | 81,1 |
| **8** | B + AD + NE | 0,845 | [0,776; 0,914] | 0,1551 | 89,0 | 70,5 | 82,7 | 80,2 | 81,1 |
| **9** | ABC + AC + NE | 0,839 | [0,769; 0,908] | 0,2054 | 93,9 | 63,9 | 88,6 | 77,8 | 81,1 |
| **10** | AB + AD+NE | 0,845 | [0,777; 0,914] | 0,1621 | 90,2 | 67,2 | 83,7 | 78,7 | 80,4 |
| **11** | B + AB + NE | 0,844 | [0,775; 0,913] | 0,1764 | 90,2 | 67,2 | 83,7 | 78,7 | 80,4 |
| **12** | B + ABCD + NE | 0,84 | [0,771; 0,909] | 0,171 | 90,2 | 67,2 | 83,7 | 78,7 | 80,4 |
| **13** | ABC + AEC + NE | 0,837 | [0,768; 0,907] | 0,0962 | 84,2 | 75,4 | 78,0 | 82,1 | 80,4 |
| **14** | D + ABCD + NE | 0,842 | [0,774; 0,91] | 0,1721 | 90,2 | 67,2 | 83,7 | 78,7 | 80,4 |
| **15** | AB + ABCD + NE | 0,843 | [0,775; 0,911] | 0,1773 | 90,2 | 67,2 | 83,7 | 78,7 | 80,4 |
| **16** | AB+D+NE | 0,844 | [0,776; 0,913] | 0,1635 | 90,2 | 67,2 | 83,7 | 78,7 | 80,4 |
| **17** | AC + ACD+ NE | 0,843 | [0,774; 0,913] | 0,0701 | 81,7 | 77,1 | 75,8 | 82,7 | 79,7 |
| **18** | B+ABC+NE | 0,841 | [0,771; 0,91] | 0,061 | 78,1 | 82,0 | 73,5 | 85,3 | 79,7 |
| **19** | B + AEC+NE | 0,84 | [0,771; 0,91] | 0,0158 | 75,6 | 85,3 | 72,2 | 87,3 | 79,7 |
| **20** | A+B+NE | 0,84 | [0,77; 0,909] | 0,0845 | 79,3 | 80,3 | 74,2 | 84,4 | 79,7 |
| **21** | A + ACD + NE | 0,84 | [0,771; 0,909] | 0,0913 | 78,1 | 82,0 | 73,5 | 85,3 | 79,7 |
| **22** | A+D+NE | 0,839 | [0,77; 0,907] | 0,0784 | 78,1 | 82,0 | 73,5 | 85,3 | 79,7 |

(continued)

| C3: Top 25 | | | | | | | | | |
|---|---|---|---|---|---|---|---|---|---|
| **RD** | **Combination C3** | **AUC ROC** | **CI 95%** | **Threshold** | **Sp (%)** | **Se (%)** | **VPP (%)** | **VPN (%)** | **Accuracy** |
| **23** | D + AEC+NE | 0,838 | [0,768; 0,907] | 0,0693 | 80,5 | 78,7 | 75,0 | 83,5 | 79,7 |
| **24** | C+AE+ NE | 0,84 | [0,773; 0,908] | 0,1219 | 81,7 | 77,1 | 75,8 | 82,7 | 79,7 |
| **25** | B+AC+NE | 0,842 | [0,772; 0,912] | 0,1049 | 85,4 | 72,1 | 78,6 | 80,5 | 79,7 |
| Decision rules:<br>RD1: Z= -0,1449xC + 0,569xAE - 0,1548xNE | | | | | | | | | |

Table 6: 5-HT2cR editing Isoforms performance using multivariable analysis with 4 isoforms (low risk versus high risk molecules)

| C4: Top 25 | | | | | | | | | |
|---|---|---|---|---|---|---|---|---|---|
| **RD** | **Combinaison C4** | AUC **ROC** | **CI 95%** | **Threshold** | Sp **(%)** | Se **(%)** | **VPP (%)** | **VPN (%)** | **Accuracy** |
| **1** | AB+ACD+AEC+ NE | 0,840 | [0,77; 0,91] | 0,0373 | 81,7 | 85,3 | 77,6 | 88,2 | 83,2 |
| **2** | B + AC + ACD+NE | 0,848 | [0,778; 0,917] | 0,079 | 85,4 | 78,7 | 80,0 | 84,3 | 82,5 |
| **3** | ABC + ACD + AEC + NE | 0842 | [0,772; 0,912] | 0,0677 | 81,7 | 83,6 | 77,3 | 87,0 | 82,5 |
| **4** | AB + D + ACD + NE | 0,848 | [0,779; 0,916] | 0,1417 | 90,2 | 72,1 | 84,6 | 81,3 | 82,5 |
| **5** | AB + ACD + ABCD + NE | 0,843 | [0,774; 0,912] | 0,1507 | 89,0 | 73,8 | 83,3 | 82,0 | 82,5 |
| **6** | B + ACD +AEC+ NE | 0,840 | [0,77; 0,91] | 0,0463 | 80,5 | 83,6 | 76,1 | 86,8 | 81,8 |
| **7** | D + ACD +AEC+ NE | 0,838 | [0,768; 0,909] | 0,0359 | 79,3 | 85,3 | 75,4 | 87,8 | 81,8 |
| **8** | D + ACD + ABCD + NE | 0,842 | [0,774; 0,911] | 0,1385 | 87,8 | 73,8 | 81,8 | 81,8 | 81,8 |
| **9** | B + AB + AEC+NE | 0,842 | [0,773; 0,911] | 0,0668 | 81,7 | 80,3 | 76,6 | 84,8 | 81,1 |
| **10** | ACD + AEC + ABCD + NE | 0,838 | [0,769; 0,907] | 0,0645 | 81,7 | 80,3 | 76,6 | 84,8 | 81,1 |
| **11** | B+AB+ACD+NE | 0,847 | [0,779; 0,916] | 0,1337 | 87,8 | 72,1 | 81,5 | 80,9 | 81,1 |
| **12** | AB+AD+ACD+ NE | 0,846 | [0,778; 0,915] | 0,1441 | 89,0 | 70,5 | 82,7 | 80,2 | 81,1 |
| **13** | D+AD+ACD+NE | 0,845 | [0,776; 0,914] | 0,1838 | 91,5 | 67,2 | 85,4 | 79,0 | 81,1 |
| **14** | AD + ACD + ABCD + NE | 0,841 | [0,772; 0,91] | 0,1934 | 92,7 | 65,6 | 87,0 | 78,4 | 81,1 |

(continued)

| C4: Top 25 | | | | | | | | | |
|---|---|---|---|---|---|---|---|---|---|
| **RD** | **Combinaison C4** | AUC **ROC** | **CI 95%** | **Threshold** | Sp **(%)** | Se **(%)** | **VPP (%)** | **VPN (%)** | **Accuracy** |
| **15** | B + AE + ABCD + NE | 0,836 | [0,767; 0,906] | 0,2695 | 95,1 | 62,3 | 90,5 | 77,2 | 81,1 |
| **16** | ABC+AC+ACD+ NE | 0,839 | [0,769; 0,91] | 0,1794 | 92,7 | 65,6 | 87,0 | 78,4 | 81,1 |
| **17** | B + AC + AE + NE | 0,837 | [0,768; 0,907] | 0,2757 | 95,1 | 62,3 | 90,5 | 77,2 | 81,1 |
| **18** | A+B+AD+NE | 0,845 | [0,776; 0,914] | 0,1699 | 90,2 | 68,9 | 84,0 | 79,6 | 81,1 |
| **19** | B + AE + ACD + NE | 0,837 | [0,767; 0,907] | 0,2528 | 95,1 | 62,3 | 90,5 | 77,2 | 81,1 |
| **20** | B + AB + AE + NE | 0,835 | [0,765; 0,906] | 0,2536 | 95,1 | 62,3 | 90,5 | 77,2 | 81,1 |
| **21** | B + D + AD + NE | 0,845 | [0,776; 0,914] | 0,1599 | 89,0 | 70,5 | 82,7 | 80,2 | 81,1 |
| **22** | B +AD + ABCD + NE | 0,845 | [0,776; 0,913] | 0,1629 | 89,0 | 70,5 | 82,7 | 80,2 | 81,1 |
| **23** | AB + ABC + AC + NE | 0,838 | [0,768; 0,908] | 0,2061 | 95,1 | 62,3 | 90,5 | 77,2 | 81,1 |
| **24** | B + D + AE + NE | 0,838 | [0,768; 0,907] | 0,2598 | 95,1 | 62,3 | 90,5 | 77,2 | 81,1 |
| **25** | B + ABC + ACD + NE | 0,844 | [0,774; 0,914] | 0,0686 | 78,1 | 83,6 | 73,9 | 86,5 | 80,4 |
| Decision rules:<br>RD1: Z= 0,0235xAB + 0,1567xACD + 0,3880xAEC - 0,1355xNE | | | | | | | | | |

Table 7: 5-HT2cR editing Isoforms performance using multivariable analysis with 5 isoforms (low risk versus high risk molecules)

| C5: Top 25 | | | | | | | | | |
|---|---|---|---|---|---|---|---|---|---|
| **RD** | **Combination C5** | **AUC ROC** | **CI 95%** | **Threshold** | **Sp (%)** | **Se (%)** | **VPP (%)** | **VPN (%)** | **Accuracy** |
| 1 | AB + ABC + ACD + AEC+ NE | 0,844 | [0,775; 0,914] | 0,0509 | 80,5 | 85,3 | 76,5 | 88,0 | 82,5 |
| 2 | AB+ D + ACD + AEC + NE | 0841 | [0,771; 0,911] | 0,0453 | 81,7 | 83,6 | 77,3 | 87,0 | 82,5 |
| 3 | AB + ACD + AEC + ABCD + NE | 0,84 | [0,77; 0,909] | 0,0463 | 81,7 | 83,6 | 77,3 | 87,0 | 82,5 |
| 4 | B + AC + D + ACD + NE | 0,846 | [0,777; 0,916] | 0,0731 | 84,2 | 78,7 | 78,7 | 84,2 | 81,8 |
| 5 | B + AB + ACD + AEC + NE | 0,843 | [0,773; 0,913] | 0,0195 | 79,3 | 85,3 | 75,4 | 87,8 | 81,8 |
| 6 | AB + AC + ACD + AEC + NE | 0,842 | [0,772; 0,913] | 0,0499 | 80,5 | 83,6 | 76,1 | 86,8 | 81,8 |

(continued)

| C5: Top 25 | | | | | | | | | |
|---|---|---|---|---|---|---|---|---|---|
| RD | Combination C5 | AUC ROC | CI 95% | Threshold | Sp (%) | Se (%) | VPP (%) | VPN (%) | Accuracy |
| 7 | ABC + D + ACD + AEC + NE | 0,841 | [0,772; 0,911] | 0,058 | 80,5 | 83,6 | 76,1 | 86,8 | 81,8 |
| 8 | B + D + ACD + AEC + NE | 0,84 | [0,77; 0,91] | 0,0422 | 80,5 | 83,6 | 76,1 | 86,8 | 81,8 |
| 9 | B + AB + D + AEC + NE | 0,842 | [0,773; 0,912] | 0,0613 | 80,5 | 82,0 | 75,8 | 85,7 | 81,1 |
| 10 | B + AB + AC + AEC + NE | 0,841 | [0,771; 0,911] | 0,058 | 81,7 | 80,3 | 76,6 | 84,8 | 81,1 |
| 11 | ABC + ACD + AEC + ABCD + NE | 0,839 | [0,77; 0,908] | 0,083 | 81,7 | 80,3 | 76,6 | 84,8 | 81,1 |
| 12 | B + AC + ACD +AEC+ NE | 0,84 | [0,77; 0,91] | 0,0213 | 79,3 | 83,6 | 75,0 | 86,7 | 81,1 |
| 13 | B + AB + AC+ ACD + NE | 0,846 | [0,776; 0,915] | 0,0681 | 81,7 | 78,7 | 76,2 | 83,8 | 80,4 |
| 14 | A + C + AE + AEC + NE | 0,844 | [0,777; 0,91] | 0,1296 | 81,7 | 78,7 | 76,2 | 83,8 | 80,4 |
| 15 | B + AB + ABC +ACD + NE | 0,844 | [0,774; 0,914] | 0,0681 | 78,1 | 83,6 | 73,9 | 86,5 | 80,4 |
| 16 | B + ABC + D + ACD + NE | 0,844 | [0,774; 0,915] | 0,0715 | 78,1 | 83,6 | 73,9 | 86,5 | 80,4 |
| 17 | A + B + ABC + ACD + NE | 0,841 | [0,771; 0,911] | 0,0868 | 79,3 | 82,0 | 74,6 | 85,5 | 80,4 |
| 18 | B + AB + AEC + ABCD +NE | 0,841 | [0,773; 0,91] | 0,0545 | 79,3 | 82,0 | 74,6 | 85,5 | 80,4 |
| 19 | B + AD + AEC + ABCD + NE | 0,84 | [0,771; 0,909] | 0,0922 | 82,9 | 77,1 | 77,1 | 82,9 | 80,4 |
| 20 | A + B + ABC+D+NE | 0,84 | [0,77; 0,909] | 0,083 | 80,5 | 80,3 | 75,4 | 84,6 | 80,4 |
| 21 | ABC + AC + ACD + AEC+ NE | 0,84 | [0,769; 0,91] | 0,0615 | 80,5 | 80,3 | 75,4 | 84,6 | 80,4 |
| 22 | A + B + D + ACD+NE | 0,84 | [0,77; 0,909] | 0,0854 | 79,3 | 82,0 | 74,6 | 85,5 | 80,4 |
| 23 | AC + AD + ACD + AEC + NE | 0,839 | [0,768; 0,909] | 0,1144 | 84,2 | 75,4 | 78,0 | 82,1 | 80,4 |
| 24 | A + AB + ACD + AEC + NE | 0837 | [0,767; 0,907] | 0,0971 | 80,5 | 80,3 | 75,4 | 84,6 | 80,4 |
| 25 | B + ACD + AEC + ABCD + NE | 0,837 | [0,767; 0,907] | 0,0491 | 80,5 | 80,3 | 75,4 | 84,6 | 80,4 |
| Decision rules:<br>RD1: Z= 0,016xAB - 0,0563xABC + 0,183xACD + 0,386xAEC - 0,1428xNE | | | | | | | | | |

Table 8; 5-HT2cR editing Isoforms performance using multivariable analysis with 6 isoforms (low risk versus high risk molecules)

| C6: Top 25 | | | | | | | | | |
|---|---|---|---|---|---|---|---|---|---|
| **RD** | **Combination C6** | **AUC ROC** | **CI 95%** | **Threshold** | **Sp (%)** | **Se (%)** | **VPP (%)** | **VPN (%)** | **Accuracy** |
| **1** | AB + ABC + D + ACD + AEC+ NE | 0,844 | [0,775; 0,913] | 0,0641 | 81,7 | 83,6 | 77,3 | 87,0 | 82,5 |
| **2** | A + AB + AC + AE + ACD + AEC | 0,826 | [ 0,753; 0,9] | 0,3615 | 93,9 | 67,2 | 89,1 | 79,4 | 82,5 |
| **3** | B + AB + D + AD + ACD + NE | 0,846 | [0,776; 0,916] | 0,1688 | 90,2 | 70,5 | 84,3 | 80,4 | 81,8 |
| **4** | B + AB + D + AD + ABCD + NE | 0,844 | [0,774; 0,913] | 0,1649 | 90,2 | 70,5 | 84,3 | 80,4 | 81,8 |
| **5** | AB + D + AD + ACD + ABCD + NE | 0,843 | [0,774; 0,912] | 0,1783 | 92,7 | 67,2 | 87,2 | 79,2 | 81,8 |
| **6** | B + AB + D + ACD + AEC + NE | 0842 | [0,773; 0,912] | 0,0323 | 79,3 | 85,3 | 75,4 | 87,8 | 81,8 |
| **7** | B + AB + ACD + AEC + ABCD + NE | 0841 | [0,772; 0,91] | 0,0235 | 79,3 | 85,3 | 75,4 | 87,8 | 81,8 |
| **8** | A + B + AC + AD + ACD + NE | 0841 | [0,77; 0,912] | 0,1462 | 87,8 | 73,8 | 81,8 | 81,8 | 81,8 |
| **9** | B + AB + AC + ACD + AEC + NE | 0840 | [0,77; 0,911] | 0,0473 | 80,5 | 83,6 | 76,1 | 86,8 | 81,8 |
| **10** | A + AB + ABC + AC + AE + ACD | 0,835 | [0,765; 0,906] | 0,2929 | 91,5 | 68,9 | 85,7 | 79,8 | 81,8 |
| **11** | A+B+D+AD+ABCD+ NE | 0,846 | [0,777; 0,914] | 0,153 | 87,8 | 72,1 | 81,5 | 80,9 | 81,1 |
| **12** | A + AB + AC + AE + AEC + NE | 0,845 | [0,777; 0,914] | 0,3084 | 92,7 | 65,6 | 87,0 | 78,4 | 81,1 |
| **13** | A + C + AE + ACD + AEC + NE | 0844 | [0,777; 0,912] | 0,1167 | 81,7 | 80,3 | 76,6 | 84,8 | 81,1 |
| **14** | A + B + D + AD + ACD + NE | 0,844 | [0,774; 0,914] | 0,1795 | 89,0 | 70,5 | 82,7 | 80,2 | 81,1 |
| **15** | A + B + ABC + D + ACD + NE | 0,842 | [0,772; 0,912] | 0,0913 | 80,5 | 82,0 | 75,8 | 85,7 | 81,1 |
| **16** | B + AB + AC + D + AEC + NE | 0,842 | [0,773; 0,912] | 0,0448 | 81,7 | 80,3 | 76,6 | 84,8 | 81,1 |
| **17** | B + AB + D + AEC + ABCD + NE | 0,842 | [0,773; 0,911] | 0,0497 | 80,5 | 82,0 | 75,8 | 85,7 | 81,1 |
| **18** | B+AC+AD+ACD+ABCD + NE | 0842 | [0,772; 0,912] | 0,1032 | 85,4 | 75,4 | 79,3 | 82,4 | 81,1 |
| **19** | AB+AC+AD+ACD+ AEC+ NE | 0,842 | [0,772; 0,912] | 0,12 | 86,6 | 73,8 | 80,4 | 81,6 | 81,1 |
| **20** | A + AB + AC + ACD + ABCD + NE | 0,841 | [0,771; 0,911] | 0,1197 | 84,2 | 77,1 | 78,3 | 83,1 | 81,1 |

(continued)

| C6: Top 25 | | | | | | | | | |
|---|---|---|---|---|---|---|---|---|---|
| **RD** | **Combination C6** | **AUC ROC** | **CI 95%** | **Threshold** | **Sp (%)** | **Se (%)** | **VPP (%)** | **VPN (%)** | **Accuracy** |
| **21** | A + AB + ABC + ACD + AEC+ NE | 0,840 | [0,771; 0,91] | 0,1306 | 82,9 | 78,7 | 77,4 | 84,0 | 81,1 |
| **22** | AB + ABC + ACD + AEC + ABCD + NE | 0,840 | [0,771; 0,909] | 0,0773 | 81,7 | 80,3 | 76,6 | 84,8 | 81,1 |
| **23** | AB + D + ACD + AEC + ABCD + NE | 0,840 | [0,771; 0,909] | 0,0537 | 80,5 | 82,0 | 75,8 | 85,7 | 81,1 |
| **24** | B + AB + AC + AE + ACD + NE | 0,840 | [0,77; 0,91] | 0,1887 | 91,5 | 67,2 | 85,4 | 79,0 | 81,1 |
| **25** | A + B + AB + AE + ACD + NE | 0,839 | [0,77; 0,908] | 0,278 | 92,7 | 65,6 | 87,0 | 78,4 | 81,1 |
| Decision rules:<br>RD1: Z= 0,0157xAB - 0,0557xABC + 0,0187xD + 0,1817xACD + 0,3883xAEC - 0,1426xNE | | | | | | | | | |

Table 9; 5-HT2cR editing Isoforms performance using multivariable analysis with 7 isoforms (low risk versus high risk molecules)

| C7 : Top 25 | | | | | | | | | |
|---|---|---|---|---|---|---|---|---|---|
| **RD** | **Combination C7** | **AUC ROC** | **CI 95%** | **Threshold** | **Sp (%)** | **Se (%)** | **VPP (%)** | **VPN (%)** | **Accuracy** |
| **1** | B + AB + D +ACD + AEC + ABCD + NE | 0,841 | [0,771; 0,91] | 0,0208 | 79,3 | 85,3 | 75,4 | 87,8 | 81,8 |
| **2** | A + B + C + AE + ACD + ABCD + NE | 0,846 | [0,78; 0,913] | 0,1983 | 86,6 | 73,8 | 80,4 | 81,6 | 81,1 |
| **3** | B + C + AD + AE + ACD + AEC + NE | 0,845 | [0,777; 0,913] | 0,0886 | 82,9 | 78,7 | 77,4 | 84,0 | 81,1 |
| **4** | A + C + AD + AE + ACD + AEC + NE | 0,844 | [0,776; 0,911] | 0,134 | 81,7 | 80,3 | 76,6 | 84,8 | 81,1 |
| **5** | A + AC + C + AE + ACD+ AEC + NE | 0,843 | [0,775; 0,912] | 0,1102 | 81,7 | 80,3 | 76,6 | 84,8 | 81,1 |
| **6** | A + AB + AC+ D + ACD+ ABCD + NE | 0,842 | [0,773; 0,912] | 0,1063 | 84,2 | 77,1 | 78,3 | 83,1 | 81,1 |
| **7** | A + B + AB + AC + ACD+ ABCD + NE | 0,842 | [0,772; 0,911] | 0,1201 | 84,2 | 77,1 | 78,3 | 83,1 | 81,1 |
| **8** | A + AB + ABC + AC + ACD + ABCD + NE | 0,840 | [0,77; 0,91] | 0,1199 | 84,2 | 77,1 | 78,3 | 83,1 | 81,1 |
| **9** | B + AB + AC + ACD + AEC + ABCD + NE | 0,840 | [0,771; 0,91] | 0,0484 | 80,5 | 82,0 | 75,8 | 85,7 | 81,1 |
| **10** | A + B + ABC + AC + ACD + ABCD + NE | 0,836 | [0,765; 0,907] | 0,1261 | 85,4 | 75,4 | 79,3 | 82,4 | 81,1 |
| **11** | B + AB + AC + D + ACD + AEC + NE | 0,842 | [0,772; 0,912] | 0,0407 | 79,3 | 83,6 | 75,0 | 86,7 | 81,1 |
| **12** | A + B + AC+D + AD + ACD + NE | 0,841 | [0,77; 0,912] | 0,1144 | 85,4 | 75,4 | 79,3 | 82,4 | 81,1 |

(continued)

| C7 : Top 25 | | | | | | | | | |
|---|---|---|---|---|---|---|---|---|---|
| **RD** | **Combination C7** | **AUC ROC** | **CI 95%** | **Threshold** | **Sp (%)** | **Se (%)** | **VPP (%)** | **VPN (%)** | **Accuracy** |
| **13** | A + ABC + C + D + AE + AEC + NE | 0,845 | [0,779; 0,912] | 0,1059 | 81,7 | 78,7 | 76,2 | 83,8 | 80,4 |
| **14** | A+C+D+AD+AE+AEC + NE | 0,845 | [0,778; 0,911] | 0,1008 | 81,7 | 78,7 | 76,2 | 83,8 | 80,4 |
| **15** | A + C+ D + AE + ACD + AEC + NE | 0,845 | [0,778; 0,912] | 0,1026 | 80,5 | 80,3 | 75,4 | 84,6 | 80,4 |
| **16** | A + AB + ABC + C + AE + AEC + NE | 0,844 | [0,777; 0,911] | 0,094 | 80,5 | 80,3 | 75,4 | 84,6 | 80,4 |
| **17** | A + ABC + AC + C + AE + AEC + NE | 0,844 | [0,777; 0,911] | 0,0948 | 81,7 | 78,7 | 76,2 | 83,8 | 80,4 |
| **18** | A + ABC + C + AD + AE+ AEC + NE | 0,844 | [0,777; 0,911] | 0,0899 | 81,7 | 78,7 | 76,2 | 83,8 | 80,4 |
| **19** | A + B + C + AE + ACD + AEC | 0,844 | [0,776; 0,912] | 0,133 | 81,7 | 78,7 | 76,2 | 83,8 | 80,4 |
| **20** | A + C + AE + ACD + AEC + ABCD + NE | 0,844 | [0,778; 0,91] | 0,1322 | 81,7 | 78,7 | 76,2 | 83,8 | 80,4 |
| **21** | A + AB + C + AD + AE + AEC + NE | 0,843 | [0,776; 0,91] | 0,0999 | 81,7 | 78,7 | 76,2 | 83,8 | 80,4 |
| **22** | A + AB + C + D + AE + AEC + NE | 0,843 | [0,776; 0,911] | 0,1286 | 81,7 | 78,7 | 76,2 | 83,8 | 80,4 |
| **23** | B + AB + D + AD + ACD + AEC+ NE | 0,843 | [0,773; 0,913] | 0,0736 | 80,5 | 80,3 | 75,4 | 84,6 | 80,4 |
| **24** | A + AB + C + AE + ACD + AEC + NE | 0,842 | [0,774; 0,911] | 0,1102 | 80,5 | 80,3 | 75,4 | 84,6 | 80,4 |
| **25** | B + AB + ABC + AC + ACD + AEC+ NE | 0,842 | [0,772; 0,912] | 0,0472 | 81,7 | 78,7 | 76,2 | 83,8 | 80,4 |
| Decision rules: RD1 : Z=-0,0505xB + 0,0224xAB + 0,001xD + 0,163xACD + 0,389xAEC - 0,1402xABCD - 0,1385xNE | | | | | | | | | |

Table 10: 5-HT2cR editing Isoforms performance using multivariable analysis with 13 isoforms (low risk versus high risk molecules)

| C13 | | | | | | | | |
|---|---|---|---|---|---|---|---|---|
| **Combination C13** | **AUC ROC** | **CI 95%** | **Threshold** | **Sp (%)** | **Se (%)** | **VPP (%)** | **VPN (%)** | **Accuracy** |
| A + B + AB + ABC + AC + C + D + AD + AE + ACD + AEC + ABCD + NE | 0,848 | [0,781; 0,915] | 0,066 | 79,3 | 80,3 | 74,2 | 84,4 | 79,7 |
| Z= 0,2035xA + 0,1283xB + 0,1979xAB + 0,1147xABC + 0,1860xAC + 0,04331xC + 0,1884xD + 0,1259xAD + 0,7739xAE + 0,4295xACD + 0,4775xAEC - 0,0415xABCD + 0,0245xNE | | | | | | | | |

### C) Decision tree approach: multivariate analysis

CART algorithm which stands for "Classification And Regression Trees" is a decision tree approach. These trees will help to build a set of classification rules, represented as a hierarchical graph easily understandable for the user. The tree consists of internal node (decision node), edge and terminal leaf. These nodes are labeled by tests and possible responses to the test match with the labels of edges from this node. If the decision tree is binary, by convention, the left edge corresponds to a positive response to test and right edge correspond to the negative response. The procedure for classification obtained will have an immediate translation in terms of decision rule.

Decision trees are popular and efficient methods of supervised classification. This method requires the use of a training set to construct the model and a test set to validate it. So, for building the dataset, we have shared our list of 'no ambiguous' molecules (n=143): 90% of the dataset are used for the learning phase (n=93 drugs) and 10% are used for the test phase (50 drugs). This sharing has been randomized and respects the initial proportion of the various statutes in each molecule. Moreover,

As example we have combined the 6 RNA editing isoforms in the 'IFN profile', with CART method for building a model of decision making (figure 20).

Table 11: 5-HT2cR editing Isoforms diagnostic performances using CART algorithm with a combination of 5 isoforms X1, X2, X3, X4 and X5 selected from the isoforms or the C13 combination, on molecules' dataset (low risk versus high risk molecules)

| | **Learning** (k-fold=10,N=10) | **Test** (k-fold=10,N=10) |
|---|---|---|
| **Sensitivity** | 87,9 % | 81,6 % |
| **Specificity** | 78,9 % | 68,8 % |
| **PPV** | 82,6 % | 73,8 % |
| **NPV** | 84,8 % | 77,7 % |
| **Error rate** | 16,1 % | 23,9 % |

The diagnostic performances of CART model using 5 RNA editing isoforms of 5-HT2cR on the data test can be also very interesting for discriminating the low risk molecules versus high risk molecules.

### D) Random forest approach: multivariate analysis

RandomForest is a popular and efficient method of supervised classification. This method requires the use of a training set to construct the model and a test set to validate it. So, for building the dataset, we have shared our list of 'no ambiguous' molecules (n=143): 65% of the dataset are used for the learning phase (n=93 drugs) and 35% are used for the test phase (50 drugs). This sharing has been randomized and respects the initial proportion of the various statutes in each molecule. Moreover, we have weigthing the learning dataset by IFN to improve the separation power of drugs with 'IFN profile' and drugs with 'basal0 profile'. So, we have added 12 IFN molecules and 8 control (basal0) taken randomly in the learning set (n=113).

As example, we have combined 7 and the 13 representatives RNA editing isoforms (See RD1 of C7, Table 9, and C13, Table 10) in the 'IFN profile', with RandomForest (RF) algorithm for building a model of decision making (Parameters of RF model: mtryStart = 1, stepFactor = 2, ntree = 500, improve = 0.01; Out Of Bag (OOB) estimate of RF model=0.21) (figures 16A-C, and 17A-C)).

Table 12: Contingency tables using random forest algorithm with 7 isoforms on 'molecules dataset (low risk versus high risk molecules)

| | **LEARNING** | **TEST** | **ALL DATA** |
|---|---|---|---|
| **Specificity** | 100 | 76 | 92 |
| **Sensitivity** | 100 | 90 | 96 |
| **Accuracy** | 100 | 84 | 94 |

Table 13: 5-HT2cR editing Isoforms diagnostic performances using random forest algorithm with 13 isoforms on molecules dataset (low risk versus high risk molecules)

| | LEARNING | TEST | ALL DATA |
|---|---|---|---|
| **Specificity** | 100 | 90 | 96 |
| **Sensitivity** | 100 | 86 | 95 |
| **Accuracy** | 100 | 88 | 96 |

The diagnostic performances of RF model using 7 or 13 RNA editing isoforms of 5-HT2cR are very interesting for discriminating the low risk molecules versus high risk molecules with a sensitivity, specificity and accuracy superior to 90% (for C7) and superior to 95 % (for C13), high significantly superior to those disclosed in Cavarec et al (2013).

**EXAMPLE 5: Target diversification**

To further supplement the 5HT2cR mRNA editing in SH-SY5Y cells we analysed additional ADAR substrates (GRIA2, FLNB, PDE8A, GRIK2 and GABRA3). Interestingly, IFN treatment altered the relative proportion of the RNA editing isoforms for all three targets studied (figure 11). It is therefore foreseeable to add additional biomarkers to further increase the diagnostic performances of the test.

**EXAMPLE 6: Compound specific RNA editing profiles obtained by NGS-based analysis of various targets**

Compound specific RNA editing profiles have been obtained by NGS-based analysis of GABRA3, GRIA2, GRIK2 and HTR2C targets (see Figures 21A-21B).
In figures 21A and 21B, the histograms display the relative proportion of the RNA editing level quantified at each specific site in the human SH-SY5Y neuroblastoma cell-line treated with the indicated compounds compared to the vehicle control treated cells.
A positive value (%) indicates an increase in RNA editing at the specific site that is induced by the compound compared to the vehicle treated cells. Oppositely, a negative value (%) indicates a decrease in RNA editing at the specific site as a result of treatment with the compound compared to the vehicle treated cells.
The RNA editing profiles has been obtained for two compounds with low or no risk to induce a particular effect in a patient (see FIG.21A, 21B). As example is provided the RNA editing profile obtained with Lidocaine (A) and Ondansetron (B) compared to vehicle control treated cells.
The RNA editing profiles has been obtained for two compounds with high risk to induce a particular effect in a patient like Reserpine (see FIG. 21C) and Fluoxetine (see Fig. 21D).

**EXAMPLE 7: Time course analysis of RNA editing**

Time course analysis of RNA editing changes has been observed by Aripiprazole, Interferon (IFN) and Reserpine on HTR2C (see figures 22A-22C). Treatment of SH-SY5Y cells with all three compounds led to time-dependent alterations of the RNA editing profile. This is clearly illustrated by the respective relative proportion of the non-edited HTR2C displaying a decrease over time. Interestingly, the specificity of the changes induced by the treatment is illustrated by the different profiles obtained between Aripiprazole (see FIG. 22A) and Interferon (see FIG. 22B) or Reserpine (see FIG. 22C). The most preferred algorithm was applied to determine the risk score of each compound at each studied time point (prob(Algorithm)). While for Interferon and Reserpine risk scores were high at all time points, Aripiprazole treatment was identified positively at risk starting from 24 hours and beyond (see Table 14 below).

Table 14: Level of risk scores at time points, after Aripiprazole, Interferon and Reserpine treatment

| MOLECULE | prob(Algorithm) | prediction |
|---|---|---|
| **Aripiprazole 12h** | 0.528 | ND |
| **Aripiprazole 24h** | 0.632 | Pos |
| **Aripiprazole 48h** | 0.760 | Pos |
| **IFN 100 UI 12h** | 0.720 | Pos |
| **IFN 100 UI 24h** | 0.968 | Pos |

(continued)

| MOLECULE | prob(Algorithm) | prediction |
|---|---|---|
| **IFN 100 UI 48h** | 0.970 | Pos |
| **Reserpine 12h** | 0.878 | Pos |
| **Reserpine 24h** | 0.986 | Pos |
| **Reserpine 48h** | 0.976 | Pos |

**EXAMPLE 8: Dose-dependent alterations of RNA editing profiles after treatment of SH-SY5Y cells with different compounds**

Dose-dependent alterations of RNA editing profiles have been obtained after treatment of SH-SY5Y cells with three different compounds, Clozapine, Sertraline and Ketamine (see figures 23A-23C).

The RNA editing profiles represent the respective relative proportion of HTR2C RNA editing as compared to vehicle-treated SH-SY5Y cells.

**References**

1. (WHO) WHO. Preventing suicide: A global imperative. 2014.
2. Labonte B, Turecki G. The epigenetics of suicide: explaining the biological effects of early life environmental adversity. Archives of suicide research : official journal of the International Academy for Suicide Research. 2010;14(4):291-310. PubMed PMID: 21082447.
3. Gurevich I, Englander MT, Adlersberg M, Siegal NB, Schmauss C. Modulation of serotonin 2C receptor editing by sustained changes in serotonergic neurotransmission. The Journal of neuroscience: the official journal of the Society for Neuroscience. 2002 Dec 15;22(24):10529-32. PubMed PMID: 12486144.
4. Sodhi MS, Burnet PW, Makoff AJ, Kerwin RW, Harrison PJ. RNA editing of the 5-HT(2C) receptor is reduced in schizophrenia. Molecular psychiatry. 2001 Jul;6(4):373-9. PubMed PMID: 11443520.
5. Alon S, Garrett SC, Levanon EY, Olson S, Graveley BR, Rosenthal JJ, et al. The majority of transcripts in the squid nervous system are extensively recoded by A-to-I RNA editing. eLife. 2015;4. PubMed PMID: 25569156. Pubmed Central PMCID: 4384741.
6. Khermesh K, D'Erchia AM, Barak M, Annese A, Wachtel C, Levanon EY, et al. Reduced levels of protein recoding by A-to-I RNA editing in Alzheimer's disease. Rna. 2016 Feb;22(2):290-302. PubMed PMID: 26655226. Pubmed Central PMCID: 4712678.
7. Porath HT, Carmi S, Levanon EY. A genome-wide map of hyper-edited RNA reveals numerous new sites. Nature communications. 2014;5:4726. PubMed PMID: 25158696. Pubmed Central PMCID: 4365171.
8. Seeburg PH, Higuchi M, Sprengel R. RNA editing of brain glutamate receptor channels: mechanism and physiology. Brain research Brain research reviews. 1998 May;26(2-3):217-29. PubMed PMID: 9651532.
9. Yang W, Wang Q, Kanes SJ, Murray JM, Nishikura K. Altered RNA editing of serotonin 5-HT2C receptor induced by interferon: implications for depression associated with cytokine therapy. Brain research Molecular brain research. 2004 Apr 29;124(1):70-8. PubMed PMID: 15093687.
10. Dracheva S, Patel N, Woo DA, Marcus SM, Siever LJ, Haroutunian V. Increased serotonin 2C receptor mRNA editing: a possible risk factor for suicide. Molecular psychiatry. 2008 Nov;13(11):1001-10. PubMed PMID: 17848916.
11. Mann JJ, Brent DA, Arango V. The neurobiology and genetics of suicide and attempted suicide: a focus on the serotonergic system. Neuropsychopharmacology: official publication of the American College of Neuropsychopharmacology. 2001 May;24(5):467-77. PubMed PMID: 11282247.
12. Mann JJ, Currier DM. Stress, genetics and epigenetic effects on the neurobiology of suicidal behavior and depression. European psychiatry: the journal of the Association of European Psychiatrists. 2010 Jun;25(5):268-71. PubMed PMID: 20451357. Pubmed Central PMCID: 2896004.
13. Mann JJ, Huang YY, Underwood MD, Kassir SA, Oppenheim S, Kelly TM, et al. A serotonin transporter gene promoter polymorphism (5-HTTLPR) and prefrontal cortical binding in major depression and suicide. Archives of general psychiatry. 2000 Aug;57(8):729-38. PubMed PMID: 10920459.
14. Dinah Weissmann, Laurent Vincent, Mark D. Underwood, Laurent Cavarec, Nicolas Salvetat, Siem van der Laan, et al. REGION SPECIFIC ALTERATIONS OF RNA EDITING OF SEROTONIN 2C RECEPTOR IN CORTEX OF SUICIDES WITH MAJOR DEPRESSION. Translational psychiatry. 2016; Minor revision.

15. Christensen R, Kristensen PK, Bartels EM, Bliddal H, Astrup A. Efficacy and safety of the weight-loss drug rimonabant: a meta-analysis of randomised trials. Lancet. 2007 Nov 17;370(9600):1706-13. PubMed PMID: 18022033.
16. Mihanovic M, Restek-Petrovic B, Bodor D, Molnar S, Oreskovic A, Presecki P. Suicidality and side effects of antidepressants and antipsychotics. Psychiatria Danubina. 2010 Mar;22(1):79-84. PubMed PMID: 20305596.
17. Moreira FA, Crippa JA. The psychiatric side-effects of rimonabant. Revista brasileira de psiquiatria. 2009 Jun;31(2):145-53. PubMed PMID: 19578688.
18. Sundstrom A, Alfredsson L, Sjolin-Forsberg G, Gerden B, Bergman U, Jokinen J. Association of suicide attempts with acne and treatment with isotretinoin: retrospective Swedish cohort study. Bmj. 2010;341:c5812. PubMed PMID: 21071484. Pubmed Central PMCID: 2978759.
19. Ge Y, S D, Speed TP. Resampling-based multiple testing for microarray data hypothesis. Sociedad de estadistica e investigacion operativa test. 2003;12:1-77.
20. Gentleman RC, Carey VJ, Bates DM, Bolstad B, Dettling M, Dudoit S, et al. Bioconductor: open software development for computational biology and bioinformatics. Genome biology. 2004;5(10):R80. PubMed PMID: 15461798. Pubmed Central PMCID: 545600.
21. Y B, Y H. Controlling the false discovery rate: a practical and powerful approach to multiple testing. J Roy Statist Soc Ser. 1995; B 57, 1: 289-300.
22. D.G. K, L.L. K, E.M. M. Applied regression analysis and other multivariate methods. PWS-KENT Publishing Company, Boston. 1988.
23. L. Breiman, J. Friedman, Olshen R, Stone. C. CART: Classification and Regression Trees. Wadsworth International. 1984.
24. Breiman L. Random Forests. Machine Learning. 2001;45 ((1)):5-32.
25. Su JQ, Liu JS. Linear combinations of multiple diagnostic markers. Journal of the American Statistical Association; 1993 (88):1350-5.
26. Wang H. A note on iterative marginal optimization: a simple algorithm for maximum rank correlation estimation. Computational Statistics and Data Analysis 2007 (51):2803-12.
27. Staack A, Badendieck S, Schnorr D, Loening SA, Jung K. Combined determination of plasma MMP2, MMP9, and TIMP1 improves the non-invasive detection of transitional cell carcinoma of the bladder. BMC urology. 2006;6:19. PubMed PMID: 16901349. Pubmed Central PMCID: 1560390.
28. Cortes C, Vapnik. V. Support-Vector Networks. Machine Learning. 1995;20.
29. Baxt WG. Application of artificial neural networks to clinical medicine. Lancet. 1995 Oct 28;346(8983):1135-8. PubMed PMID: 7475607.
30. N. F, al. e. Using Bayesian networks to analyze expression data. J Comput Biol. 2000;7((3-4)):601-20.
31. K. H, K.P. S. Weighted k-Nearest-Neighbor Techniques and Ordinal Classification. Discussion Paper 399, SFB 386, Ludwig-Maximilians University Munich 2004.
32. Wold S. PLS-regression: a basic tool of chemometrics. Chemometrics and Intelligent Laboratory Systems. 2001;58(108-130).
33. Fisher RA. The Use of Multiple Measurements in Taxonomic Problems. Annals of Eugenics. 1936;(2)(179-188).
34. D W. EVALUATION OF THE POTENTIAL RISK OF DRUG INDUCED MOOD DISTURBANCE AND SUICIDE: USE OF A DEDICATED PLATFORM. Patent WO2008/152146. 2008.
35. Cavarec L, Vincent L, Le Borgne C, Plusquellec C, Ollivier N, Normandie-Levi P, et al. In vitro screening for drug-induced depression and/or suicidal adverse effects: a new toxicogenomic assay based on CE-SSCP analysis of HTR2C mRNA editing in SH-SY5Y cells. Neurotoxicity research. 2013 Jan;23(1):49-62. PubMed PMID: 22528247.

## Claims

1. A computer implemented method of predicting the probability or the risk of a drug, a compound or a molecule, to induce particular effects in a patient, preferably side effects, more preferably adverse or desired side effects, said method using as a target exhibiting an A-to-I editing of RNA, the pre-mRNA of which being the substrate of ADARs enzymes, the action of said ADARs leading to the production of different isoforms or sites, wherein said method comprises the steps of:

A) Analysing the target RNA editing profile in sample that have been treated with said drug or compound or molecule, in order to obtain the proportion of RNA editing level of said target for each of its editing isoforms, and, wherein said target RNA editing profile is obtained as obtained for a molecule of the collection of molecule in the algorithm obtained for said particular effects, and wherein said algorithm is an algorithm for *in vitro* predicting the probability of a compound to induce a particular effect in a patient, wherein said algorithm or model is obtained by a method comprising the steps of:

a)

- selecting at least one target exhibiting an A-to-I editing of RNA, the pre-mRNA of which being the substrate of ADARs enzymes (Adenosine Deaminases Acting on RNA), the action of said ADARs on at least one editing site leading to the production of different isoforms or sites,
- selecting at least one cell line which endogenously expresses said at least one target and at least the ADAR enzymes,
- selecting a positive control compound capable of dose-dependently altering the relative proportion of said target isoforms or editing sites when cells of said cell line are treated with said positive control,
- selecting a collection of molecules composed of a ratio of compounds annotated with a risk score to induce said particular effects,

b) treating cells of said cell line with each single molecule of said collection of molecules, along with a negative control and said positive control,
c) analysing said at least one target RNA editing profile in each sample that have been treated with a molecule of the collection, in order to obtain the proportion of RNA editing level of said target for each of its editing isoforms and/or sites for each of the molecules of said collection,
d)

-i) by an univariable analysis statistical method, evaluating for each isoform/or editing site its accuracy and its power to discriminate the risk of a molecule to induce said particular effects; and/or
-ii) by a multivariable analysis statistical method, evaluating for each combination of isoforms/or editing sites, its accuracy and its power to discriminate the risk of a molecule to induce said particular effects, and
-iii) selecting the combination exhibiting the best discriminative performance,

e) building an algorithm using said selected combination of isoforms/or editing sites, and use said algorithm thus obtained for predicting the probability said compound to induce said particular effects in a patient,

and :
wherein step d)-i) comprises a step of calculating for each isoform or a combination thereof:

- the optimal threshold of sensitivity (Se %) of at least 60 and specificity (Sp%) of at least 60%for said particular effects;
- the positive (PPV, %) and negative (NPV, %) predictive values to evaluate the proportion of true presence [true positive /(true positive+ false positive] and true absence [true negative /(true negative+ false negative)]; and

wherein in step c), the RNA editing profile is carried out by a method including:

- NGS method (Next-Generation-Sequencing) comprising NGS library preparation; and
- the sequencing of all the NGS libraries obtained,

to obtain the editing profile of the target; and

wherein in step d)-i) and d)-ii), said statistical method allowing the obtaining of said algorithm or model is carried out by a method including one method or a combination of methods selected from the group consisting of:

- mROC program, particularly to identify the linear combination, which maximizes the AUC (Area Under the Curve) ROC and wherein the equation for the respective combination is provided and can be used as a new virtual marker Z, as follows:

  $Z = a_1 . (\text{Isoform } 1) + a_2 . (\text{Isoform } 2) + ...a_i . (\text{Isoform } i) +....a_n . (\text{Isoform } n)$
  where $a_1$ are calculated coefficients and (Isoform i) are the relative proportion of individual RNA editing level of isoform's target; and/or

- a logistic regression model applied for univariate and multivariate analysis to estimate the relative risk of molecules at different isoforms values; and/or
- a CART (Classification and Regression Trees) approach applied to assess isoforms combinations; and/or
- a Random Forest (RF) approach applied to assess the isoform combinations, particularly to rank the importance of editing isoform and to combine the best isoforms to classify the "relative risk" of molecule, and/or
- a multivariate analysis applied to assess the isoforms combination for the "relative risk" of molecules selecting from the group consisting of as
- Support Vector Machine (SVM) approach;
- Artificial Neural Network (ANN) approach;
- Bayesian network approach;
- wKNN (weighted k-nearest neighbours) approach;
- Partial Least Square - Discriminant Analysis (PLS-DA);
- Linear and Quadratic Discriminant Analysis (LDA / QDA);

B) calculating the end value or applied the algorithm or model obtained for said drug or compound using said algorithm or model obtained for said target and said particular effects; and
C) determining whether said drug or compounds is at risk, particularly at low risk versus high risk, to induce said particular effects in a patient in view of the results obtained in step B).

**2.** The computer implemented method according to claim 1 wherein in said algorithm, said effects are side effects selected from adverse or desired side effects.

**3.** The computer implemented method to claim 1 or 2, wherein in said algorithm said target exhibiting an A-to-I editing of RNA is selected from the group consisting of 5-HT2cR, PDE8A (Phosphodiesterase 8A), GRIA2 (Glutamate receptor 2), GRIA3, GRIA4, GRIK1, GRIK2, GRIN2C, GRM4, GRM6 FLNB (Filamin B), 5-HT2A, GABRA3, FLNA, CYFIP2.

**4.** The computer implemented method according to one of claims 1 to 3, wherein in said algorithm said particular effects are adverse psychiatric side effects.

**5.** The computer implemented method according to one of claims 1 to 4, wherein in said algorithm said cell line endogenously expressing said target and ADAR(s), and is selected in the group consisting of:

- neuroblastoma cell lines,
- neuroblastoma cell lines for which the positive control induced ADAR1a gene expression with a fold induction of at least 4, 5 or 6 when normalised to negative or vehicle controls, and
- the human SH-SY5Y cell line.

**6.** The computer implemented method according to one of claims 1 to 5, wherein in step b) of said algorithm the cells of said cell line are treated during a period of time comprised between 12 h and 72 h with the molecules or controls to be tested.

**7.** The computer implemented method according to one of claims 1 to 6, wherein in said algorithm said positive control is interferon alpha.

**8.** The computer implemented method according to one of claims 1 to 7, wherein the step c) of said algorithm comprises a step of determining the basal level of the RNA editing for each isoform/or site in said cell line compared to vehicle treated control cells, in order to obtain for each molecule and each editing isoforms/or editing sites the mean/median relative proportion of RNA editing level of said target.

**9.** The computer implemented method according to one of claims 1 to 8, wherein said method is a computer implemented method for *in vitro* predicting the probability of a drug or a compound to induce particular effects with no risk or a low risk or a high risk.

**10.** The computer implemented method according to one of claims 1 to 8, wherein in said algorithm said collection of molecules is composed of an equilibrated ratio of therapeutic classes of molecules, each molecule being annotated with a high risk and low risk score to induce said particular effects

**11.** The computer implemented method according to one of claims 1 to 10, wherein said algorithm further comprises in step c) the bioinformatics analysis of said sequencing data, said bioinformatics analysis comprising the steps of:

- pre-alignment processing and quality control of the sequences
- the alignment against reference sequence; and
- the editing levels calling.

**12.** The computer implemented method of one of claims 1 to 11, wherein: in said algorithm:

- said target is the 5-HT2cR,
- said particular effects are adverse psychiatric adverse side effects, -
- the cell line is the human SH-SY5Y neuroblastoma cell line,
- the positive control is the interferon alpha,

and wherein:
the sites combination capable of discriminating whether the test drug is at low risk or high risk to induce said psychiatric adverse side effects comprises at least a combination of at least 2, 3, 4 or 5 of the single sites selected from the group constituted of the following 5-HT2cR, sites:
A, B, C, D, and E,

- or the isoforms combination capable of discriminating whether the test drug is at low risk or high risk to induce said psychiatric adverse side effects comprises at least a combination of at least 2, 3, 4, 5, 6, 7, 8, 9, 10, 11, 12 or 13 of the single isoforms selected from the group constituted of the following 5-HT2cR, isoforms: A, B, AB, ABC, AC, C, D, AD, AE, ACD, AEC, ABCD and NE,

and wherein:
said statistical method allowing the obtaining of said algorithm or model is carried out by a method including:

- mROC program, Random Forest approach and/or Cart algorithm.

**13.** The computer implemented method of claim 12, wherein in said algorithm the sites combination capable of discriminating whether the test drug is at low risk or high risk to induce said psychiatric adverse side effects comprises at least a combination of at least 3, 4 or 5 of the single sites selected from the group constituted of the 5-HT2cR A, B, C, D, and E sites.

**14.** The computer implemented method of claim 12 or 13, wherein in said algorithm the isoforms combination capable of discriminating whether the test drug is at low risk or high risk to induce said psychiatric adverse side effects comprises at least a combination of at least 5, 6 or 7 of the single isoforms selected from the group constituted of the 5-HT2cR A, B, AB, ABC, AC, C, D, AD, AE, ACD, AEC, ABCD and NE, isoforms.

## Patentansprüche

**1.** Computerimplementiertes Verfahren zum Vorhersagen der Wahrscheinlichkeit oder des Risikos, dass ein Arzneimittel, eine Verbindung oder ein Molekül bei einem Patienten bestimmte Wirkungen, bevorzugt Nebenwirkungen, stärker bevorzugt unerwünschte oder gewünschte Nebenwirkungen, induzieren, wobei das Verfahren als ein Ziel ein A-bis-I-Editieren von RNA vorweist, deren prä-mRNA das Substrat von ADARs-Enzymen ist, wobei die Einwirkung der ADARs zu der Erzeugung verschiedener Isoformen oder Stellen führt, wobei das Verfahren die folgenden Schritte umfasst:

A) Analysieren des Ziel-RNA-Editierprofils in einer Probe, die mit dem Arzneimittel oder der Verbindung oder dem Molekül behandelt wurde, um den Anteil eines RNA-Editierniveaus des Ziels für jede seiner Editierisoformen zu erhalten, und wobei das Ziel-RNA-Editierprofil erhalten wird wie für ein Molekül der Molekülsammlung in dem Algorithmus erhalten, der für die bestimmten Wirkungen erhalten wurde, und wobei der Algorithmus ein Algorithmus zum *In-vitro*-Vorhersagen der Wahrscheinlichkeit ist, dass eine Verbindung eine bestimmte Wirkung bei einem Patienten induziert, wobei der Algorithmus oder das Modell durch ein Verfahren erhalten wird, das die folgenden Schritte umfasst:

a)

- Auswählen wenigstens eines Ziels, das ein A-zu-I-Editieren von RNA vorweist, deren prä-mRNA das Substrat von *ADARs*(*Adenosine Deaminases Acting on RNA*)-Enzymen ist, wobei die Einwirkung der ADARs auf wenigstens eine Editierstelle zu der Erzeugung verschiedener Isoformen oder Stellen führt
- Auswählen wenigstens einer Zelllinie, die das wenigstens eine Ziel und wenigstens die ADAR-Enzyme endogen exprimiert,
- Auswählen einer Positivkontrollverbindung, die in der Lage ist, den relativen Anteil der Zielisoformen oder Editierstellen dosisabhängig zu verändern, wenn Zellen der Zelllinie mit der Positivkontrolle behandelt werden,
- Auswählen einer Molekülsammlung, die aus einem Verhältnis von Verbindungen zusammengesetzt ist, die mit einem Risiko-Score kommentiert sind, die bestimmten Wirkungen zu induzieren,

b) Behandeln von Zellen der Zelllinie mit jedem einzelnen Molekül der Molekülsammlung zusammen mit einer Negativkontrolle und der Positivkontrolle,
c) Analysieren des wenigstens einen Ziel-RNA-Editierprofils in jeder Probe, die mit einem Molekül der Sammlung behandelt wurde, um den Anteil des RNA-Editierungsniveaus des Ziels für jede seiner Editierisoformen und/oder Stellen für jedes der Moleküle der Sammlung zu erhalten,
d)

-i) durch ein statistisches Verfahren mit univariabler Analyse, Auswerten, für jede Isoform/oder Editierstelle, ihrer Genauigkeit und ihrer Fähigkeit, das Risiko, dass ein Molekül die bestimmten Wirkungen induziert, zu unterscheiden; und/oder
-ii) durch ein statistisches Verfahren mit multivariabler Analyse, Auswerten, für jede Kombination von Isoformen/oder Editierstellen, ihrer Genauigkeit und ihrer Fähigkeit, das Risiko, dass ein Molekül die bestimmten Wirkungen induziert, zu unterscheiden; und
-iii) Auswählen der Kombination, die die beste Unterscheidungsleistung vorweist,

e) Erstellen eines Algorithmus unter Verwendung der ausgewählten Kombination von Isoformen/oder Editierstellen und Verwenden des so erhaltenen Algorithmus zum Vorhersagen der Wahrscheinlichkeit, dass die Verbindung die bestimmten Wirkungen bei einem Patienten induziert,

und:
wobei Schritt d-i) einen Schritt eines Berechnens von Folgendem für jede Isoform oder eine Kombination davon umfasst:

- die optimale Empfindlichkeitsschwelle (Se-%) von wenigstens 60 und die Spezifität (Sp-%) von wenigstens 60 % für die bestimmten Wirkungen;
- die positiven (PPV, %) und negativen (NPV, %) Vorhersagewerte, um das Verhältnis von echtem Vorliegen [echt positiv/(echt positiv + falsch positiv] und echter Abwesenheit [echt negativ/(echt negativ + falsch negativ)] auszuwerten; und

wobei in Schritt c) das RNA-Editierprofil durch ein Verfahren ausgeführt wird, das Folgendes beinhaltet:

- ein *NGS*(*Next-Generation-Sequencing*)-Verfahren, das eine Vorbereitung der NGS-Bibliothek umfasst; und

- die Sequenzierung aller der erhaltenen NGS-Bibliotheken,

um das Editierprofil des Ziels zu erhalten; und
wobei in Schritt d)-i) und d)-ii) das statistische Verfahren, das das Erhalten des Algorithmus oder des Modells ermöglicht, durch ein Verfahren ausgeführt wird, das ein Verfahren oder
eine Kombination von Verfahren beinhaltet, die aus der Gruppe ausgewählt sind, die aus Folgendem besteht:

- mROC-Programm, insbesondere, um die lineare Kombination zu identifizieren, die die AUC-(*Area Under the Curve*)ROC maximiert und wobei die Gleichung für die jeweilige Kombination bereitgestellt wird und als ein neuer virtueller Marker Z wie folgt verwendet werden kann:

$$Z = a_1 . (\text{Isoform } 1) + a_2 . (\text{Isoform } 2) + ... \; a_1 . (\text{Isoform } i) + .... \; a_n . (\text{Isoform } n),$$

wobei $a_1$ berechnete Koeffizienten sind und (Isoform i) der relative Anteil des individuellen RNA-Editierniveaus des Ziels der Isoform ist; und/oder
- einem logistischen Regressionsmodell, das für die univariate und multivariate Analyse angewendet wird, um das relative Risiko von Molekülen bei verschiedenen Isoformenwerten abzuschätzen; und/oder
- einem CART(*Classification and Regression Trees*)-Ansatz, der angewendet wird, um Isoformenkombinationen zu bewerten; und/oder
- einem Random Forest(RF)-Ansatz, der angewendet wird, um die Isoformenkombinationen zu bewerten, insbesondere, um die Bedeutung der Editierisoform einzustufen und um die besten Isoformen zu kombinieren, um das "relative Risiko" eines Moleküls zu klassifizieren, und/oder
- einer multivariaten Analyse, die angewendet wird, um die Isoformenkombination für das "relative Risiko" von Molekülen zu bewerten, die aus der Gruppe ausgewählt ist, die aus Folgendem besteht:
- Stützvektormaschine(SVM)-Ansatz;
- Künstliches Neuronales Netzwerk(*Artificial Neural Network* - ANN)-Ansatz;
- Bayes'sches Netzwerk-Ansatz;
- *wKNN(weighted k-nearest neighbours*)-Ansatz;
- Diskriminanzanalyse der partiellen kleinsten Abweichungsquadrate (*Partial Least Square - Discriminant Analysis* - PLS-DA);
- Lineare und quadratische Diskriminanzanalyse (LDA/QDA);

B) Berechnen des Endwerts oder Anwenden des für das Arzneimittel oder die Verbindung erhaltenen Algorithmus oder Modells unter Verwendung des für das Ziel und die bestimmten Wirkungen erhaltenen Algorithmus oder Modells; und
C) Festlegen, ob das Risiko, insbesondere ein niedriges Risiko verglichen mit einem hohen Risiko, besteht, dass das Arzneimittel oder die Verbindungen die bestimmten Wirkungen bei einem Patienten induziert in Anbetracht der in Schritt B) erhaltenen Ergebnisse.

**2.** Computerimplementiertes Verfahren nach Anspruch 1, wobei in dem Algorithmus die Wirkungen Nebenwirkungen sind, die aus unerwünschten oder gewünschten Nebenwirkungen ausgewählt sind.

**3.** Computerimplementiertes Verfahren nach Anspruch 1 oder 2, wobei in dem Algorithmus das Ziel, das ein A-zu-I-Editieren von RNA vorweist, aus der Gruppe ausgewählt ist, die aus 5-HT2cR, PDE8A (Phosphodiesterase 8A), GRIA2 (Glutamatrezeptor 2), GRIA3, GRIA4, GRIK1, GRIK2, GRIN2C, GRM4, GRM6 FLNB (Filamin B), 5-HT2A, GABRA3, FLNA, CYFIP2 besteht.

**4.** Computerimplementiertes Verfahren nach einem der Ansprüche 1 bis 3, wobei in dem Algorithmus die bestimmten Wirkungen unerwünschte psychiatrische Nebenwirkungen sind.

**5.** Computerimplementiertes Verfahren nach einem der Ansprüche 1 bis 4, wobei in dem Algorithmus die Zelllinie das Ziel und ADAR(s) endogen exprimiert und aus der Gruppe ausgewählt ist, die aus Folgendem besteht:

- Neuroblastomzelllinien,
- Neuroblastomzelllinien, für die die Positivkontrolle die ADAR1a-Genexpression mit einer wenigstens 4-, 5- oder 6-fachen Induktion induziert hat, wenn sie auf Negativ- oder Vehikelkontrollen normalisiert werden, und
- die Human-SH-SY5Y-Zelllinie.

**6.** Computerimplementiertes Verfahren nach einem der Ansprüche 1 bis 5, wobei in Schritt b) des Algorithmus die Zellen der Zelllinie während eines Zeitraums zwischen 12 h und 72 h mit den zu testenden Molekülen oder Kontrollen behandelt werden.

**7.** Computerimplementiertes Verfahren nach einem der Ansprüche 1 bis 6, wobei in dem Algorithmus die Positivkontrolle Interferon alpha ist.

**8.** Computerimplementiertes Verfahren nach einem der Ansprüche 1 bis 7, wobei der Schritt c) des Algorithmus einen Schritt des Festlegens des Grundniveaus des RNA-Editierens für jede Isoform/oder Stelle in der Zelllinie im Vergleich zu mit Vehikel behandelten Kontrollzellen umfasst, um für jedes Molekül und jede Editierisoform/oder Editierstelle den durchschnittlichen/mittleren relativen Anteil des RNA-Editierniveaus des Ziels zu erhalten.

**9.** Computerimplementiertes Verfahren nach einem der Ansprüche 1 bis 8, wobei das Verfahren ein computerimplementiertes Verfahren zum *In-vitro*-Vorhersagen der Wahrscheinlichkeit ist, dass ein Arzneimittel oder eine Verbindung bestimmte Wirkungen ohne Risiko oder mit geringem Risiko oder hohem Risiko induziert.

**10.** Computerimplementiertes Verfahren nach einem der Ansprüche 1 bis 8, wobei in dem Algorithmus die Molekülsammlung aus einem äquilibrierten Verhältnis von therapeutischen Molekülklassen zusammengesetzt ist, wobei jedes Molekül mit einem hohen Risiko und einem niedrigen Risiko-Score kommentiert ist, die bestimmten Wirkungen zu induzieren.

**11.** Computerimplementiertes Verfahren nach einem der Ansprüche 1 bis 10, wobei der Algorithmus ferner in Schritt c) die Bioinformatik-Analyse der Sequenzierungsdaten umfasst, wobei die Bioinformatik-Analyse die folgenden Schritte umfasst:

- Prä-Ausrichtungsverarbeiten und Qualitätskontrolle der Sequenzen
- die Ausrichtung gegen eine Referenzsequenz; und
- Aufrufen der Editierniveaus.

**12.** Computerimplementiertes Verfahren nach einem der Ansprüche 1 bis 11, wobei:

in dem Algorithmus:

- das Ziel das 5-HT2cR ist,
- die bestimmten Wirkungen unerwünschte psychiatrische Nebenwirkungen sind, -
- die Zelllinie die Human-SH-SY5Y-Neuroblastom-Zelllinie ist,
- die Positivkontrolle das Interferon alpha ist,

und wobei:
die Stellenkombination, die in der Lage ist, zu unterscheiden, ob das Testarzneimittel ein niedriges Risiko oder ein hohes Risiko aufweist, die unerwünschten psychiatrischen Nebenwirkungen zu induzieren, wenigstens eine Kombination von wenigstens 2, 3, 4 oder 5 der einzelnen Stellen umfasst, die aus der Gruppe ausgewählt sind, die aus den folgenden 5-HT2cR-Stellen gebildet wird:
A, B, C, D, und E,

- oder die Isoformenkombination, die in der Lage ist, zu unterscheiden, ob das Testarzneimittel ein niedriges Risiko oder ein hohes Risiko aufweist, die unerwünschten psychiatrischen Nebenwirkungen zu induzieren, wenigstens eine Kombination von wenigstens 2, 3, 4, 5, 6, 7, 8, 9, 10, 11, 12 oder 13 der einzelnen Isoformen umfasst, die aus der Gruppe ausgewählt sind, die aus den folgenden 5-HT2cR-Isoformen gebildet wird: A, B, AB, ABC, AC, C, D, AD, AE, ACD, AEC, ABCD und NE,

und wobei:
das statistische Verfahren, das das Erhalten des Algorithmus oder des Modells ermöglicht, durch ein Verfahren ausgeführt wird, das Folgendes beinhaltet:

- mROC-Programm, Random Forest-Ansatz und/oder Cart-Algorithmus.

**13.** Computerimplementiertes Verfahren nach Anspruch 12, wobei in dem Algorithmus die Stellenkombination, die in der Lage ist, zu unterscheiden, ob das Testarzneimittel ein niedriges Risiko oder ein hohes Risiko aufweist, die unerwünschten psychiatrischen Nebenwirkungen zu induzieren, wenigstens eine Kombination von wenigstens 3, 4 oder 5 der einzelnen Stellen umfasst, die aus der Gruppe ausgewählt sind, die aus den 5-HT2cR A, B, C, D, und E Stellen gebildet wird.

**14.** Computerimplementiertes Verfahren nach Anspruch 12 oder 13, wobei in dem Algorithmus die Isoformenkombination, die in der Lage ist, zu unterscheiden, ob das Testarzneimittel ein niedriges Risiko oder ein hohes Risiko aufweist, die unerwünschten psychiatrischen Nebenwirkungen zu induzieren, wenigstens eine Kombination von wenigstens 5, 6, oder 7 der einzelnen Isoformen umfasst, die aus der Gruppe ausgewählt sind, die aus den 5-HT2cR A, B, AB, ABC, AC, C, D, AD, AE, ACD, AEC, ABCD und NE Isoformen gebildet wird.

## Revendications

**1.** Procédé mis en œuvre par ordinateur permettant de prédire la probabilité ou le risque qu'un médicament, un composé ou une molécule induise des effets particuliers chez un patient, de préférence des effets secondaires, plus préférablement des effets secondaires indésirables ou souhaités, ledit procédé utilisant comme cible présentant une édition A-à-I d'ARN, dont le pré-ARNm est le substrat des enzymes ADAR, l'action desdits ADAR conduisant à la production de différents isoformes ou sites, ledit procédé comprenant les étapes consistant à :

A) Analyser le profil d'édition de l'ARN cible dans un échantillon qui a été traité avec ledit médicament ou composé ou molécule, afin d'obtenir la proportion du niveau d'édition de l'ARN de ladite cible pour chacun de ses isoformes d'édition, et,
ledit profil d'édition de l'ARN cible étant obtenu tel qu'obtenu pour une molécule de la collection de molécules dans l'algorithme obtenu pour lesdits effets particuliers, et ledit algorithme étant un algorithme pour prédire de manière *in vitro* la probabilité qu'un composé induise un effet particulier chez un patient, ledit algorithme ou modèle étant obtenu par un procédé comprenant les étapes consistant à :

a)

- sélectionner au moins une cible présentant une édition A-à-I de l'ARN, le pré-ARNm dont le substrat d'enzymes ADAR (Adénosine désaminases agissant sur l'ARN), l'action desdits ADAR sur au moins un site d'édition conduisant à la production de différents isoformes ou sites,
- sélectionner au moins une lignée cellulaire qui exprime de manière endogène ladite une cible et au moins les enzymes ADAR,
- sélectionner un composé de contrôle positif capable de modifier de manière dépendante de la dose la proportion relative desdits isoformes ou sites d'édition cibles lorsque des cellules de ladite lignée cellulaire sont traitées avec ledit contrôle positif,
- sélectionner une collection de molécules composée d'un rapport de composés annotés avec un score de risque afin d'induire lesdits effets particuliers,

b) traiter les cellules de ladite lignée cellulaire avec chaque molécule unique de ladite collection de molécules, conjointement avec un contrôle négatif et ledit contrôle positif,
c) analyser ledit un profil d'édition de l'ARN cible dans chaque échantillon qui a été traité avec une molécule de la collection, afin d'obtenir la proportion du niveau d'édition de l'ARN de ladite cible pour chacun de ses isoformes et/ou sites d'édition pour chacune des molécules de ladite collection,
d)

-i) par une méthode statistique d'analyse univariée, évaluer pour chaque isoforme/ou site d'édition son exactitude et son pouvoir de discriminer le risque qu'une molécule induise lesdits effets particuliers ; et/ou
-ii) par une méthode statistique d'analyse multivariable, évaluer pour chaque combinaison d'isoformes/ou de sites d'édition, son exactitude et son pouvoir de discriminer le risque qu'une molécule induise lesdits effets particuliers, et
-iii) sélectionner la combinaison présentant la meilleure performance de discrimination,

e) construire un algorithme à l'aide de ladite combinaison sélectionnée d'isoformes/ou de sites de modification, et utiliser ledit algorithme ainsi obtenu pour prédire la probabilité que ledit composé induise lesdits effets particuliers chez un patient,

et :
l'étape d)-i) comprenant une étape de calcul pour chaque isoforme ou une combinaison de celles- ci :

- le seuil optimal de sensibilité (Se %) d'au moins 60 et de spécificité (Sp %) d'au moins 60 % pour lesdits effets particuliers ;
- les valeurs prédictives positives (VPP, %) et négatives (VPN, %) pour évaluer la proportion de présence réelle [vrai positif / (vrai positif + faux positif] et d'absence réelle [vrai négatif / (vrai négatif + faux négatif)] ; et

à l'étape c), le profil d'édition de l'ARN étant mis en œuvre par un procédé comportant

- la méthode NGS (séquençage de nouvelle génération) comprenant la préparation de banque NGS ; et
- le séquençage de toutes les banques NGS obtenues,

pour obtenir le profil d'édition de la cible ; et
à l'étape d)-i) et d)-ii), ledit procédé statistique permettant l'obtention dudit algorithme ou modèle étant mis en œuvre par un procédé comportant un procédé ou une combinaison de procédés choisis dans le groupe constitué par :

- le programme mROC, en particulier pour identifier la combinaison linéaire, qui maximise la courbe ROC AUC (surface sous la courbe) et l'équation de la combinaison respective étant fournie et pouvant être utilisée comme nouveau marqueur virtuel Z, comme suit :

  $Z = a_1$. (Isoforme 1) + $a_2$. (Isoforme 2) + ... $a_1$. (Isoforme i) + ... $a_n$. (Isoform n)
  où $a_1$ sont les coefficients calculés et (Isoform i) sont la proportion relative du niveau d'édition de l'ARN individuel de la cible d'isoforme ; et/ou

- un modèle de régression logistique appliqué pour une analyse univariée et multivariée afin d'estimer le risque relatif des molécules au niveau de différentes valeurs d'isoformes ; et/ou
- une approche CART (arbres de classification et de régression) appliquée pour mesurer les combinaisons d'isoformes ; et/ou
- une approche de forêt aléatoire (RF) appliquée pour mesurer les combinaisons d'isoformes, en particulier pour classer l'importance de l'édition d'isoformes et pour combiner les meilleurs isoformes afin de classer le « risque relatif » de molécule, et/ou
- une analyse multivariée appliquée pour mesurer la combinaison d'isoformes pour le « risque relatif » de molécules choisies dans le groupe constitué par
- l'approche de la machine à vecteurs de support (SVM) ;
- l'approche du réseau de neurones artificiels (RNA) ;
- l'approche par réseau bayésien ;
- l'approche wKNN (k plus proches voisins pondérés) ;

  - la régression des moindres carrés partiels - analyse discriminante (PLS-AD) ;
  - l'analyse discriminante linéaire et quadratique (ADL/QDA) ;

B) le calcul de la valeur finale ou l'application de l'algorithme ou du modèle obtenu pour ledit médicament ou composé à l'aide dudit algorithme ou modèle obtenu pour ladite cible et lesdits effets particuliers ; et
C) le fait de déterminer si ledit médicament ou lesdits composés sont à risque, notamment à faible risque par rapport au risque élevé, pour induire lesdits effets particuliers chez un patient au vu des résultats obtenus à l'étape B).

**2.** Procédé mis en œuvre par ordinateur selon la revendication 1, dans lequel, dans ledit algorithme, lesdits effets sont des effets secondaires choisis parmi des effets secondaires indésirables ou souhaités.

**3.** Procédé mis en œuvre par ordinateur selon la revendication 1 ou 2, dans lequel, dans ledit algorithme, ladite cible présentant une édition A-à-I de l'ARN est choisie dans le groupe constitué par 5-HT2cR, PDE8A (Phosphodiesterase 8A), GRIA2 (récepteur de glutamate 2), GRIA3, GRIA4, GRIK1, GRIK2, GRIN2C, GRM4, GRM6 FLNB (filamine B), 5-HT2A, GABRA3, FLNA, CYFIP2.

**4.** Procédé mis en œuvre par ordinateur selon l'une des revendications 1 à 3, dans lequel, dans ledit algorithme, lesdits effets particuliers sont des effets secondaires psychiatriques indésirables.

**5.** Procédé mis en œuvre par ordinateur selon l'une des revendications 1 à 4, dans lequel, dans ledit algorithme, ladite lignée cellulaire exprime de manière endogène ladite cible et ledit/lesdits ADAR, et est sélectionnée dans le groupe constitué par :

- des lignées cellulaires de neuroblastome,
- des lignées cellulaires de neuroblastome pour lesquelles le contrôle positif a induit l'expression du gène ADAR1a avec une induction de pli d'au moins 4, 5 ou 6 lorsqu'il est normalisé en contrôle négatif ou de véhicule, et
- la lignée cellulaire humaine SH-SY5Y.

**6.** Procédé mis en œuvre par ordinateur selon l'une des revendications 1 à 5, dans lequel, à l'étape b) dudit algorithme, les cellules de ladite lignée cellulaire sont traitées pendant une durée comprise entre 12 h et 72 h avec les molécules ou les contrôles à tester.

**7.** Procédé mis en œuvre par ordinateur selon l'une des revendications 1 à 6, dans lequel, dans ledit algorithme, ledit contrôle positif est l'interféron alpha.

**8.** Procédé mis en œuvre par ordinateur selon l'une des revendications 1 à 7, dans lequel l'étape c) dudit algorithme comprend une étape de détermination du niveau basal de l'édition de l'ARN pour chaque isoforme/ou site dans ladite lignée cellulaire par rapport à des cellules témoins traitées par un véhicule, afin d'obtenir, pour chaque molécule et chaque isoforme d'édition /ou site d'édition, la proportion relative moyenne/médiane du niveau d'édition de l'ARN de ladite cible.

**9.** Procédé mis en œuvre par ordinateur selon l'une des revendications 1 à 8, dans lequel ledit procédé est un procédé mis en œuvre par ordinateur permettant de prédire de manière *in vitro* la probabilité qu'un médicament ou un composé induise des effets particuliers sans risque ou avec un risque faible ou un risque élevé.

**10.** Procédé mis en œuvre par ordinateur selon l'une des revendications 1 à 8, dans lequel, dans ledit algorithme, ladite collection de molécules est composée d'un rapport équilibré de classes thérapeutiques de molécules, chaque molécule étant annotée avec un score de risque élevé et faible pour induire ladite effets particuliers.

**11.** Procédé mis en œuvre par ordinateur selon l'une des revendications 1 à 10, dans lequel ledit algorithme comprend en outre à l'étape c) l'analyse bio-informatique desdites données de séquençage, ladite analyse bio-informatique comprenant les étapes consistant à :

- traiter le pré-alignement et contrôler la qualité des séquences
- aligner par rapport à la séquence de référence ; et
- appeler des niveaux d'édition.

**12.** Procédé mis en œuvre par ordinateur selon l'une des revendications 1 à 11, dans lequel : dans ledit algorithme :

- ladite cible est le 5-HT2cR,
- lesdits effets particuliers sont des effets secondaires indésirables psychiatriques, -
- la lignée cellulaire est la lignée cellulaire de neuroblastome humain SH-SY5Y,
- le contrôle positif est l'interféron alpha, et :

la combinaison de sites capable de discriminer si le médicament à tester présente un risque faible ou élevé d'induire lesdits effets secondaires indésirables psychiatriques comprenant au moins une combinaison d'au moins 2, 3, 4 ou 5 des sites uniques choisis dans le groupe constitué des sites 5-HT2cR suivants :

A, B, C, D et E,

- ou la combinaison d'isoformes capable de discriminer si le médicament à tester présente un risque faible ou élevé d'induire lesdits effets secondaires indésirables psychiatriques comprend au moins une combinaison d'au moins 2, 3, 4, 5, 6, 7, 8, 9, 10 , 11, 12 ou 13 des isoformes uniques sélectionnés dans le groupe constitué par des isoformes de 5-HT2cR suivants : A, B, AB, ABC, AC, C, D, AD, AE, ACD, AEC, ABCD et NE,

et :

ledit procédé statistique permettant d'obtenir ledit algorithme ou modèle étant mis en œuvre par un procédé comportant :

- le programme mROC, l'approche de forêt aléatoire et/ou l'algorithme CART.

**13.** Procédé mis en œuvre par ordinateur selon la revendication 12, dans lequel, dans ledit algorithme, la combinaison de sites capable de discriminer si le médicament à tester présente un faible risque ou un risque élevé d'induire lesdits effets secondaires indésirables psychiatriques comprend au moins une combinaison d'au moins 3, 4 ou 5 des sites uniques choisis dans le groupe constitué des sites 5-HT2cR A, B, C, D et E.

**14.** Procédé mis en œuvre par ordinateur selon la revendication 12 ou 13, dans lequel, dans ledit algorithme, la combinaison d'isoformes capable de discriminer si le médicament à tester est à faible risque ou à risque élevé d'induire lesdits effets secondaires indésirables psychiatriques comprend au moins une combinaison d'au moins 5, 6 ou 7 des isoformes uniques choisis dans le groupe constitué des isoformes de 5-HT2cR A, B, AB, ABC, AC, C, D, AD, AE, ACD, AEC, ABCD et NE.

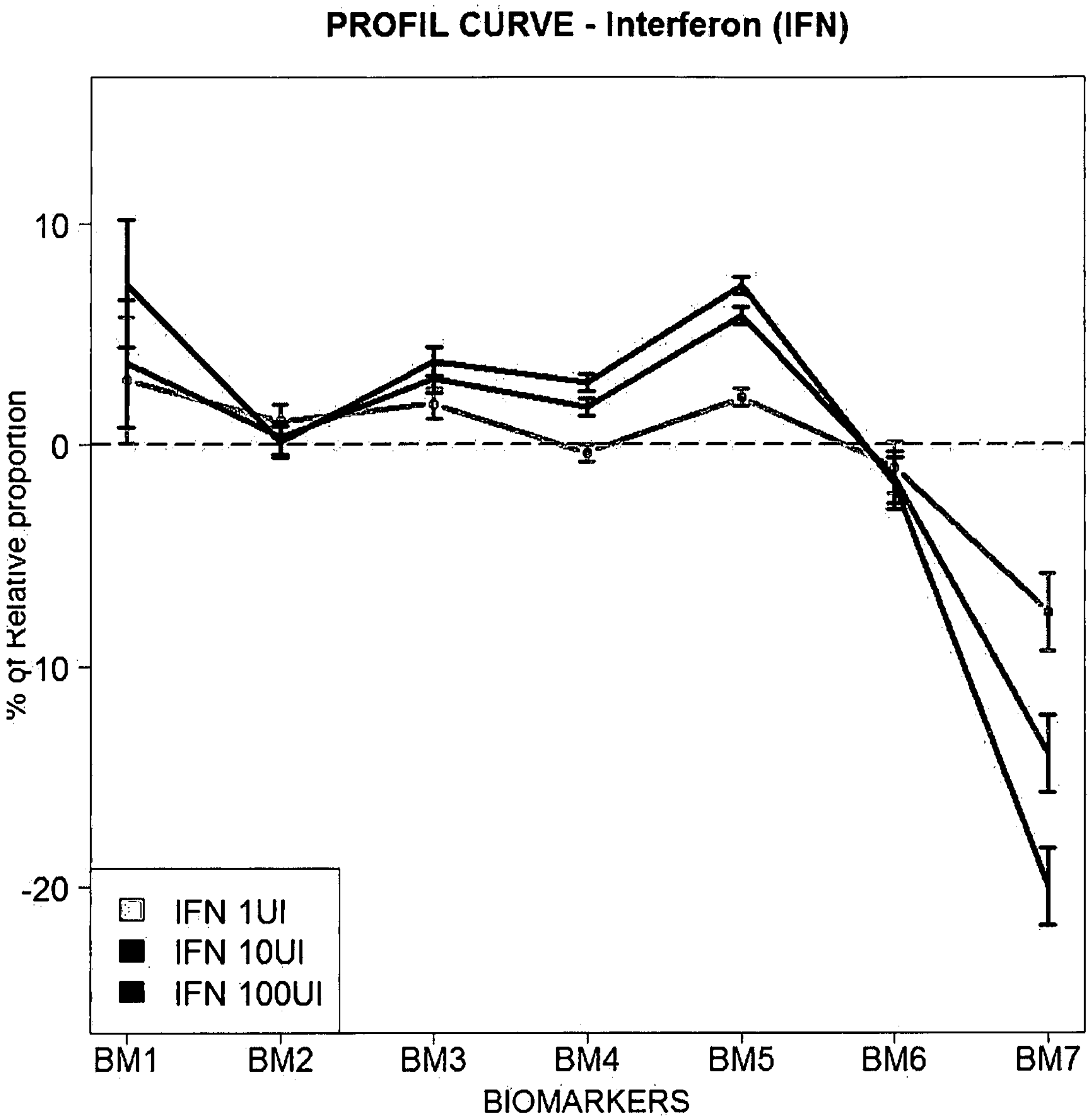
PROFIL CURVE - Interferon (IFN)
10
0
-10
-20
% of Relative proportion
IFN 1UI
IFN 10UI
IFN 100UI
BM1
BM2
BM3
BM4
BM5
BM6
BM7
BIOMARKERS

FIG. 1

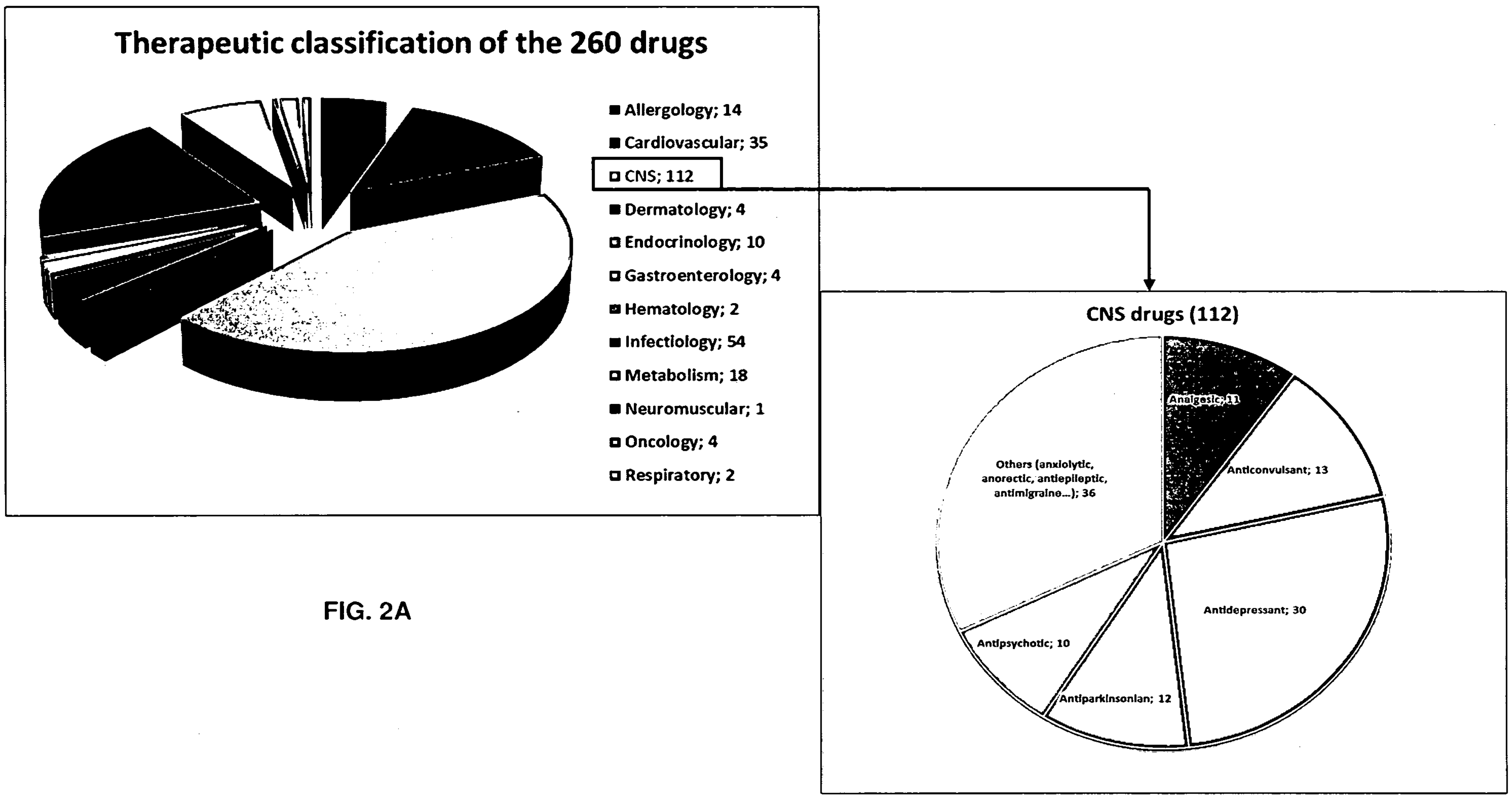
Therapeutic classification of the 260 drugs
Allergology; 14
Cardiovascular; 35
CNS; 112
Dermatology; 4
Endocrinology; 10
Gastroenterology; 4
Hematology; 2
Infectiology; 54
Metabolism; 18
Neuromuscular; 1
Oncology; 4
Respiratory; 2
CNS drugs (112)
Analgesic; 11
Anticonvulsant; 13
Antidepressant; 30
Antiparkinsonian; 12
Antipsychotic; 10
Others (anxiolytic, anorectic, antiepileptic, antimigraine...); 36

FIG. 2A

FIG. 2B

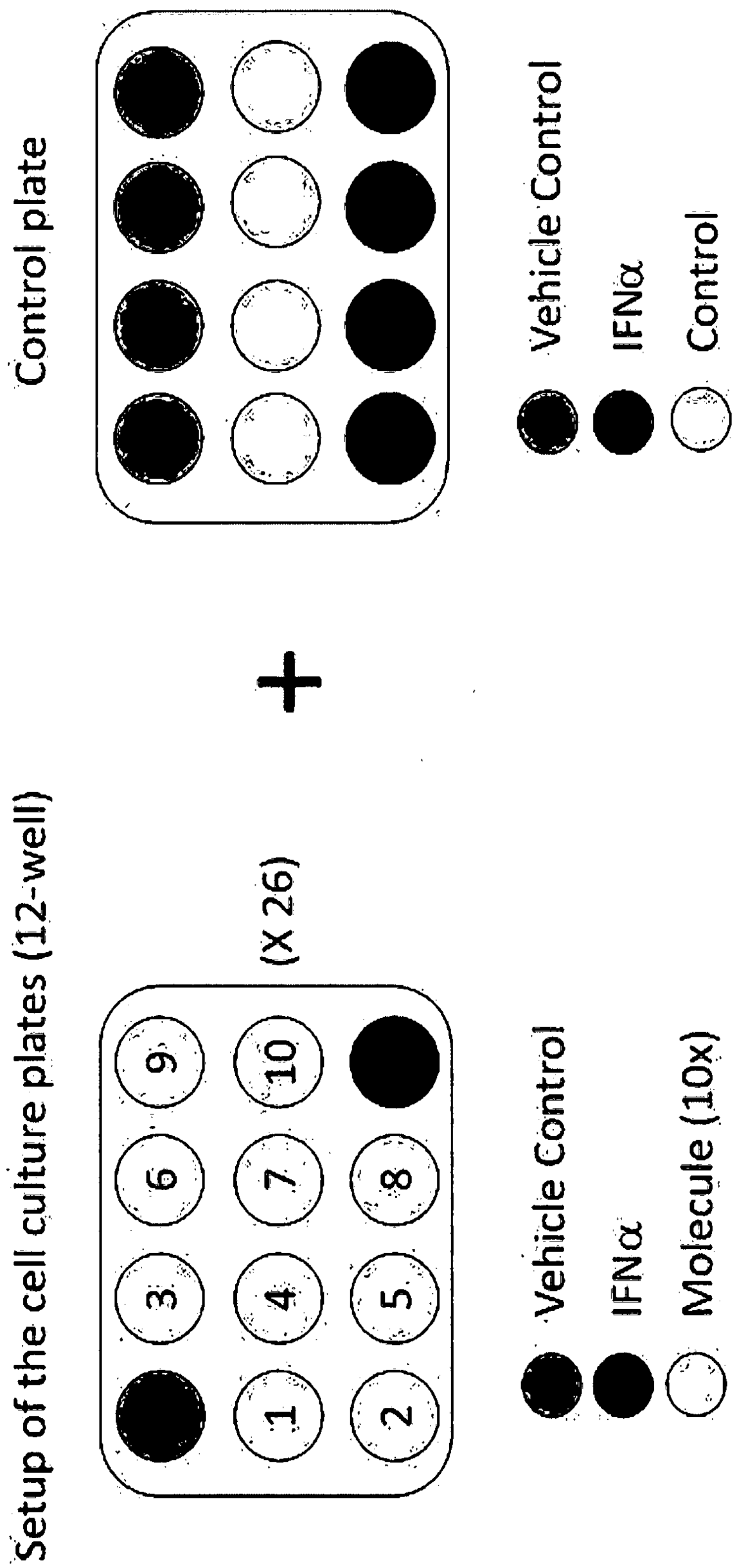
Setup of the cell culture plates (12-well)
1
2
3
4
5
6
7
8
9
10
(X 26)
Vehicle Control
IFNα
Molecule (10x)
Control plate
Vehicle Control
IFNα
Control

FIG. 3

FIG. 4A

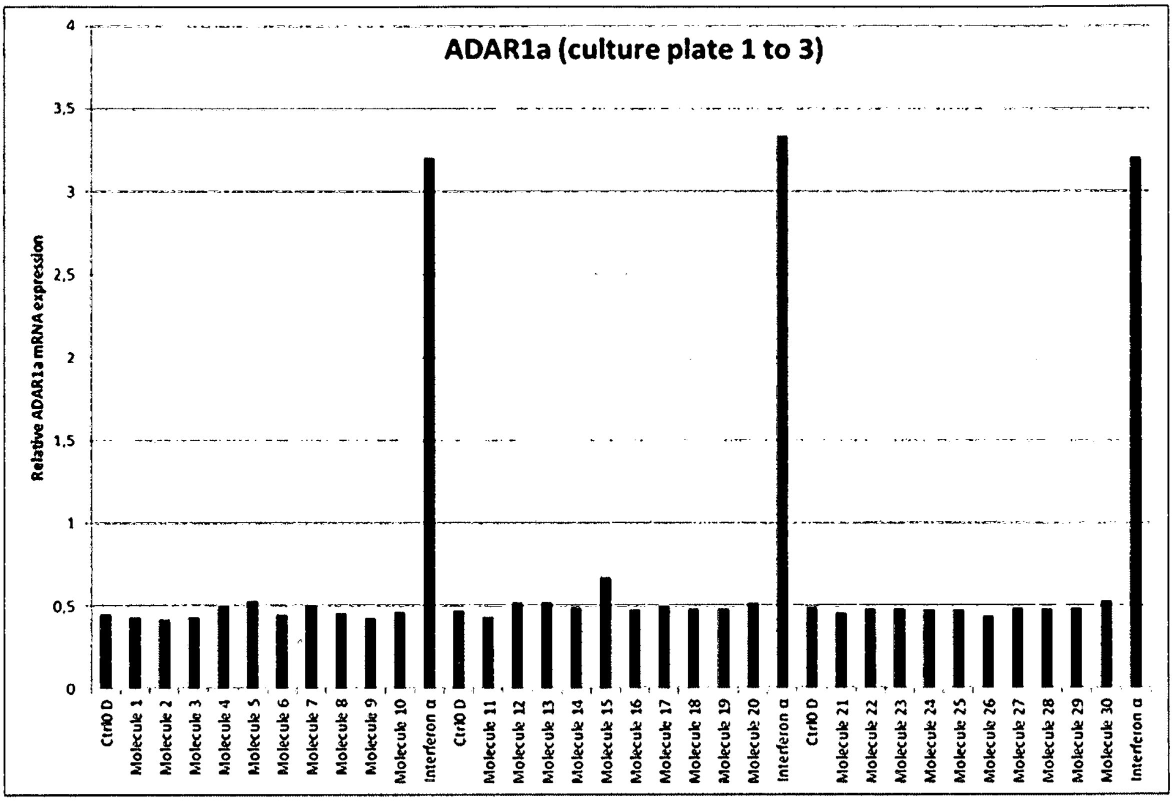


FIG. 4B

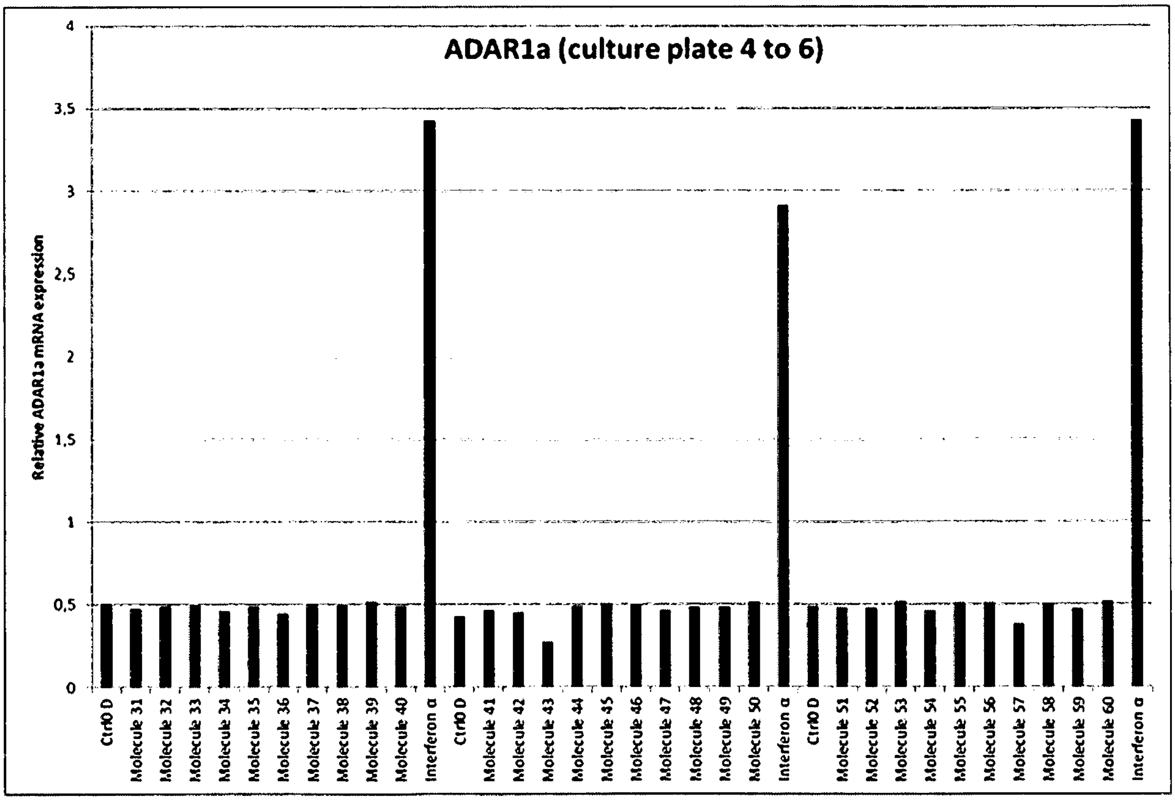


FIG. 4A-4B

FIG. 4C

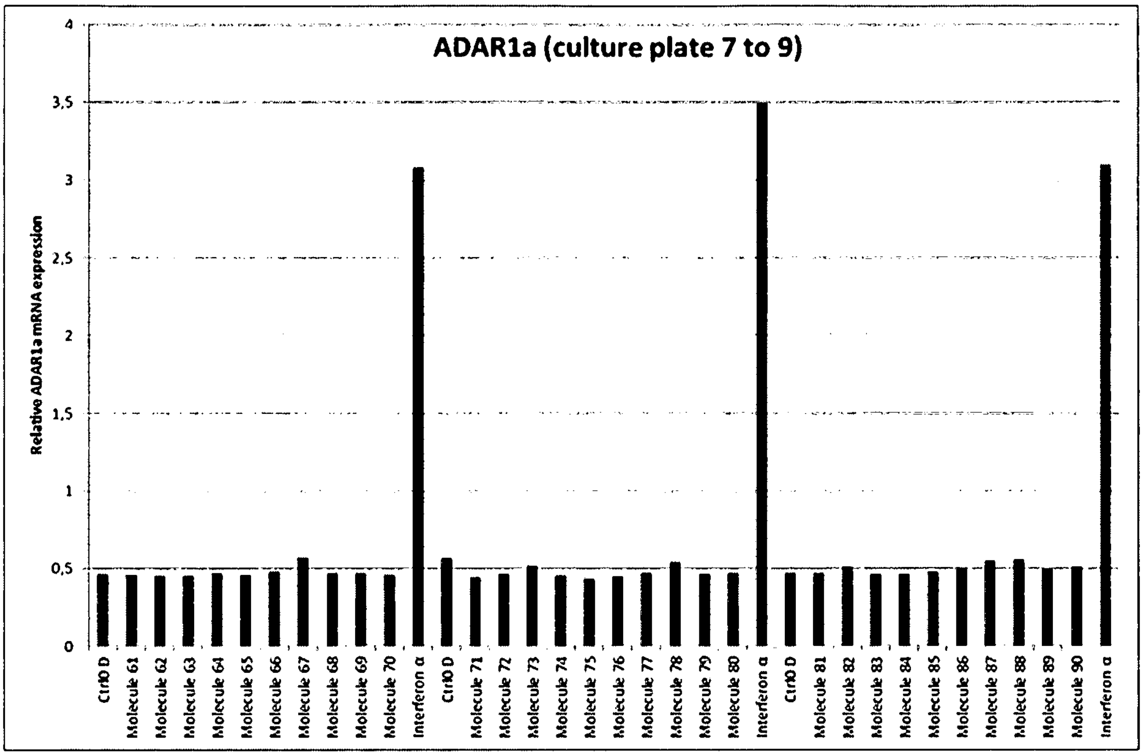


FIG. 4 D

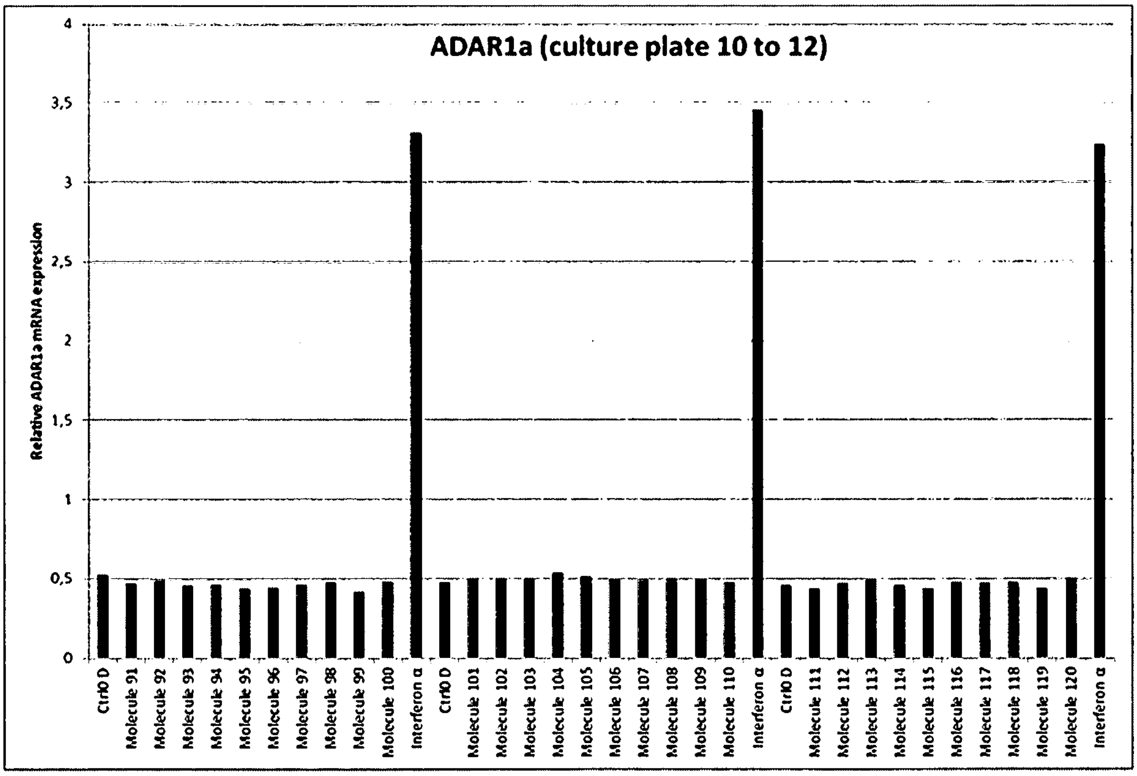


FIG. 4C-4D

FIG. 4E

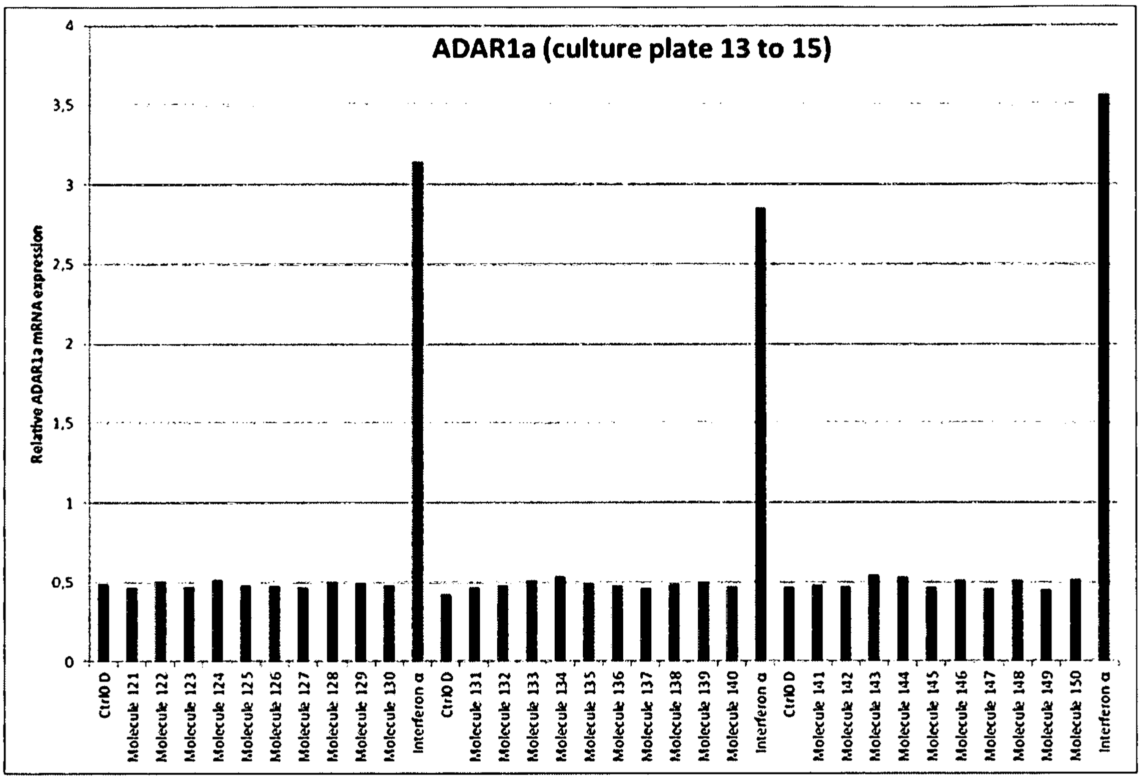


FIG. 4F

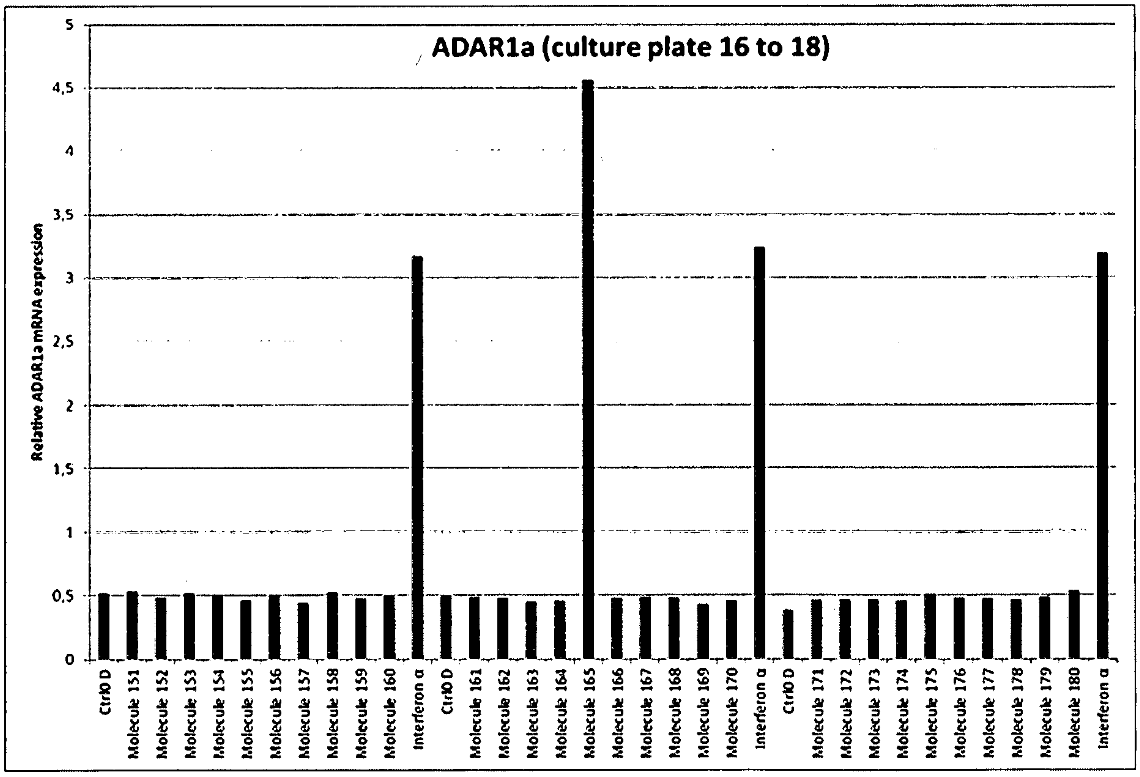


**FIG. 4E-4F**

FIG. 4G

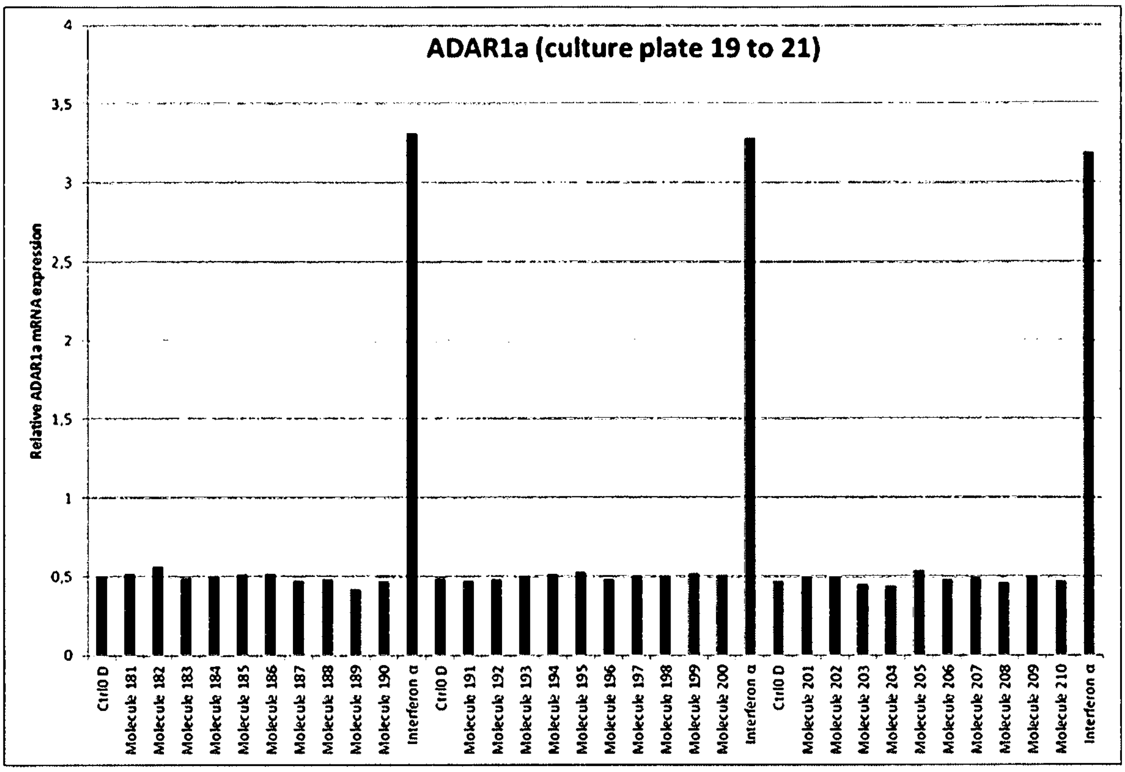


FIG. 4H

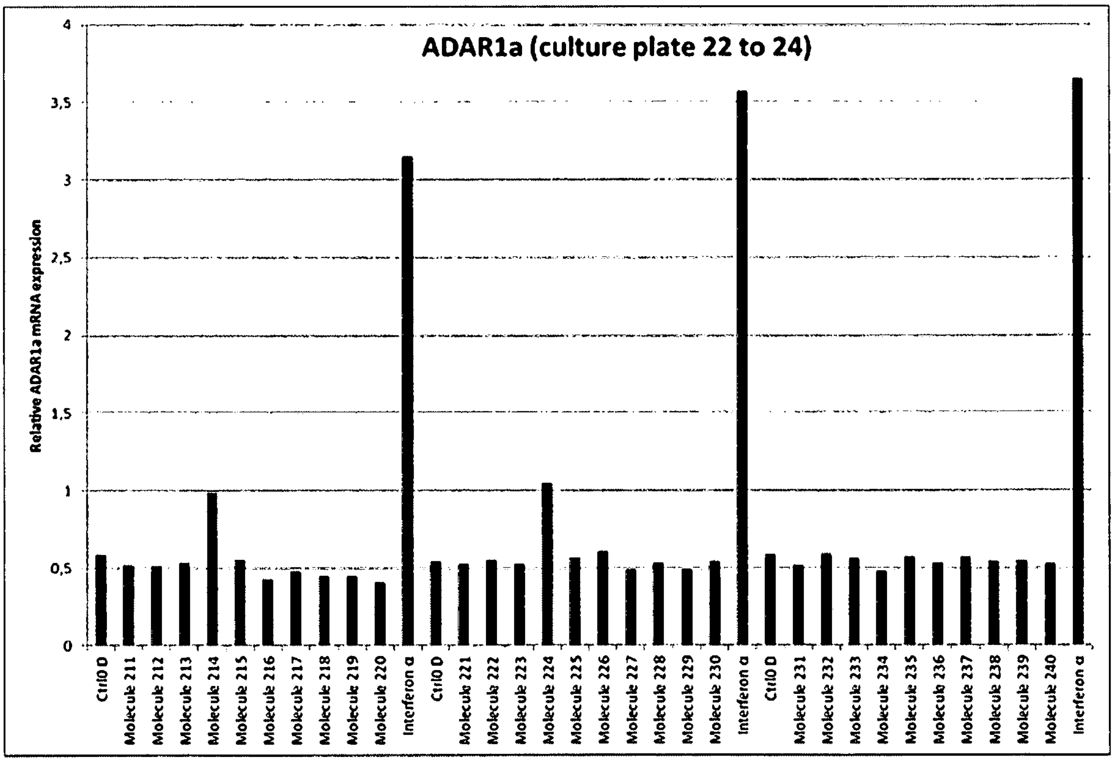


FIG. 4G-4H

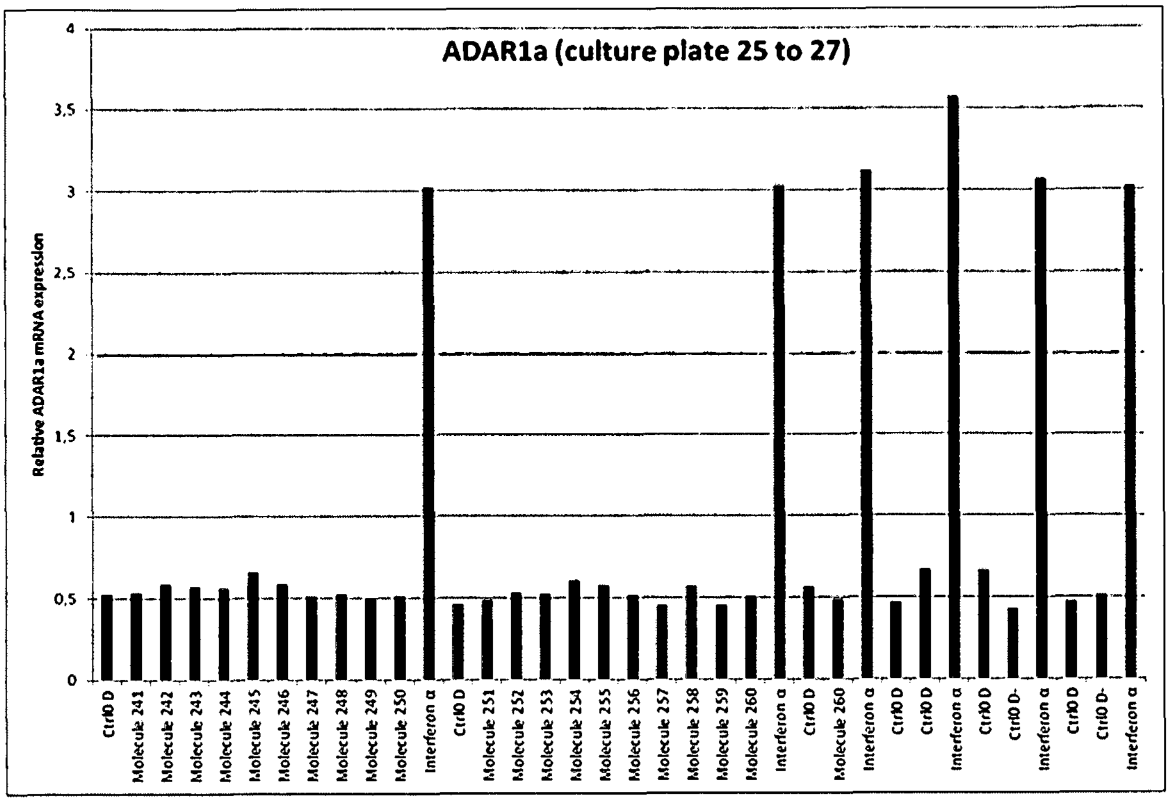
ADAR1a (culture plate 25 to 27)
Relative ADAR1a mRNA expression
4
3,5
3
2,5
2
1,5
1
0,5
0
CtrlO D
Molecule 241
Molecule 242
Molecule 243
Molecule 244
Molecule 245
Molecule 246
Molecule 247
Molecule 248
Molecule 249
Molecule 250
Interferon α
CtrlO D
Molecule 251
Molecule 252
Molecule 253
Molecule 254
Molecule 255
Molecule 256
Molecule 257
Molecule 258
Molecule 259
Molecule 260
Interferon α
CtrlO D
Molecule 260
Interferon α
CtrlO D
CtrlO D
Interferon α
CtrlO D
CtrlO D-
Interferon α
CtrlO D
CtrlO D-
Interferon α

FIG. 4I

| Vehicle Control | | | | | | | | | | | | | | | | | | | | | | | | | |
|---|---|---|---|---|---|---|---|---|---|---|---|---|---|---|---|---|---|---|---|---|---|---|---|---|---|
| | A | B | AB | ABC | AC | C | D | E | AD | AE | BC | BD | BE | CD | EC | ED | ABD | ACD | ABE | AEC | AED | ABCD | ABEC | AECD | NE |
| Mean | 33,1 | 1,9 | 3,5 | 1,4 | 5,6 | 2,7 | 0,6 | 0,2 | 0,7 | 0,2 | 0,1 | 0,1 | 0,0 | 0,1 | 0,0 | 0,0 | 0,3 | 0,3 | 0,0 | 0,2 | 0,0 | 0,1 | 0,0 | 0,0 | 48,8 |
| Median | 32,79 | 1,52 | 3,055 | 1,165 | 5,315 | 2,515 | 0,12 | 0,02 | 0,15 | 0,02 | 0,03 | 0,01 | 0 | 0,01 | 0 | 0 | 0,02 | 0,02 | 0 | 0,01 | 0 | 0,01 | 0 | 0 | 48,42 |
| E-TYPE | 5,52 | 1,61 | 2,37 | 1,66 | 2,51 | 1,88 | 0,87 | 0,54 | 0,93 | 0,44 | 0,27 | 0,28 | 0,03 | 0,30 | 0,25 | 0,19 | 0,62 | 0,65 | 0,10 | 0,49 | 0,09 | 0,27 | 0,23 | 0,04 | 6,31 |
| CV | 16,7 | 83,7 | 67,3 | 114,6 | 45,0 | 68,8 | ND | ND | ND | ND | ND | ND | ND | ND | ND | ND | ND | ND | ND | ND | ND | ND | ND | ND | 12,9 |

| IFN 100UI/ml | | | | | | | | | | | | | | | | | | | | | | | | | |
|---|---|---|---|---|---|---|---|---|---|---|---|---|---|---|---|---|---|---|---|---|---|---|---|---|---|
| | A | B | AB | ABC | AC | C | D | E | AD | AE | BC | BD | BE | CD | EC | ED | ABD | ACD | ABE | AEC | AED | ABCD | ABEC | AECD | NE |
| Mean | 36,76 | 2,347 | 6,171 | 4,938 | 12,66 | 3,294 | 0,32 | 0,15 | 0,65 | 0,46 | 0,12 | 0,04 | 0,01 | 0,11 | 0,03 | 0,01 | 0,21 | 0,31 | 0,13 | 0,46 | 0,01 | 0,274 | 0,03 | 0,014 | 30,51 |
| Median | 37,06 | 2,15 | 6,065 | 4,42 | 12,85 | 3,125 | 0,06 | 0,02 | 0,12 | 0,04 | 0,06 | 0,01 | 0 | 0,01 | 0,01 | 0 | 0,02 | 0,04 | 0 | 0,02 | 0 | 0,02 | 0 | 0 | 29,57 |
| E-TYPE | 5,89 | 1,709 | 2,873 | 2,645 | 3,917 | 2,063 | 0,59 | 0,38 | 0,95 | 0,77 | 0,27 | 0,15 | 0,11 | 0,34 | 0,21 | 0,05 | 0,47 | 0,6 | 0,37 | 0,78 | 0,05 | 0,616 | 0,18 | 0,128 | 6,933 |
| CV | 16,02 | 72,82 | 46,56 | 53,56 | 30,93 | 62,62 | ND | ND | ND | ND | ND | ND | ND | ND | ND | ND | ND | ND | ND | ND | ND | ND | ND | ND | 22,72 |

FIG 5A

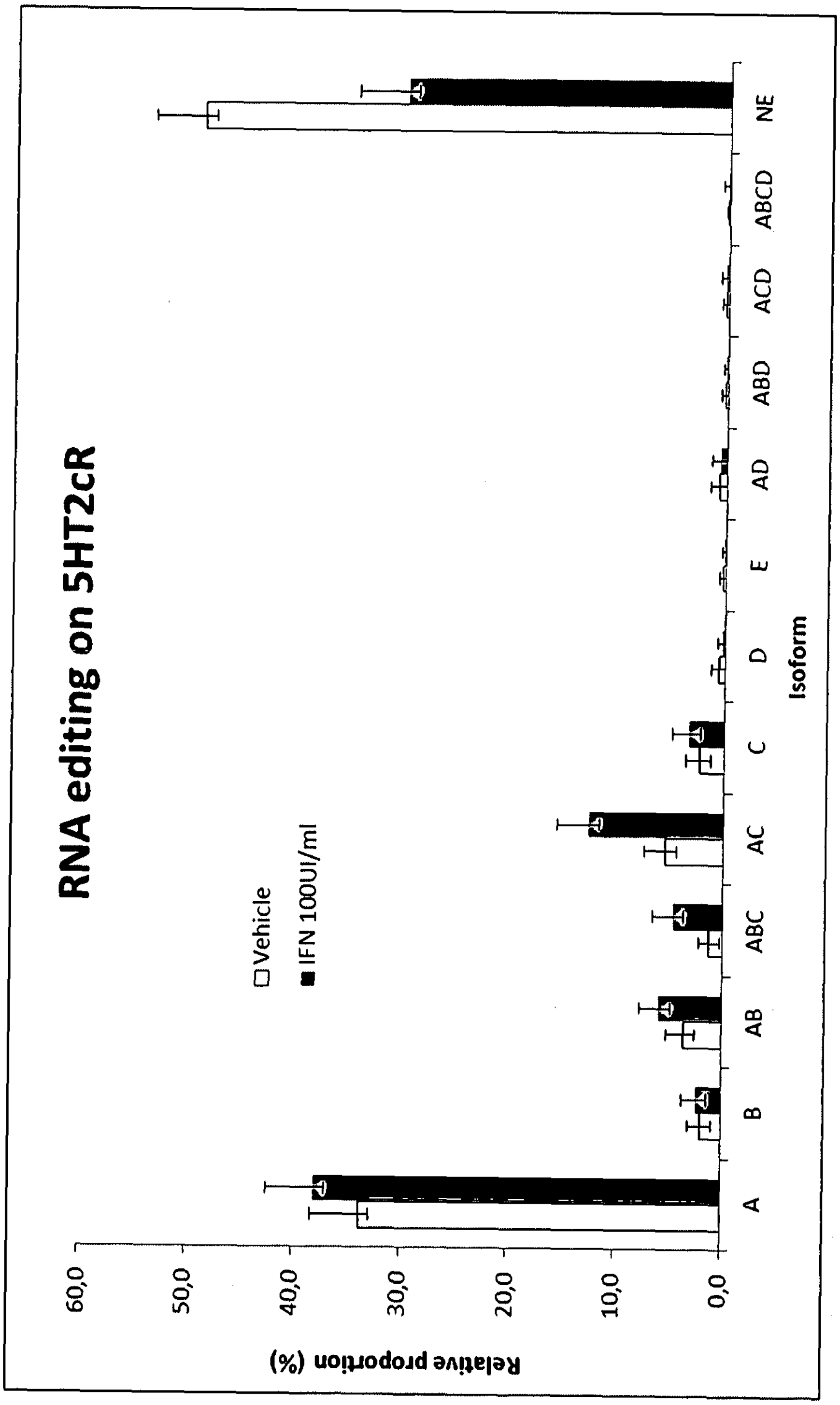
RNA editing on 5HT2cR
Vehicle
IFN 100UI/ml
Relative proportion (%)
60,0
50,0
40,0
30,0
20,0
10,0
0,0
A
B
AB
ABC
AC
C
D
E
AD
ABD
ACD
ABCD
NE
Isoform

FIG. 5B

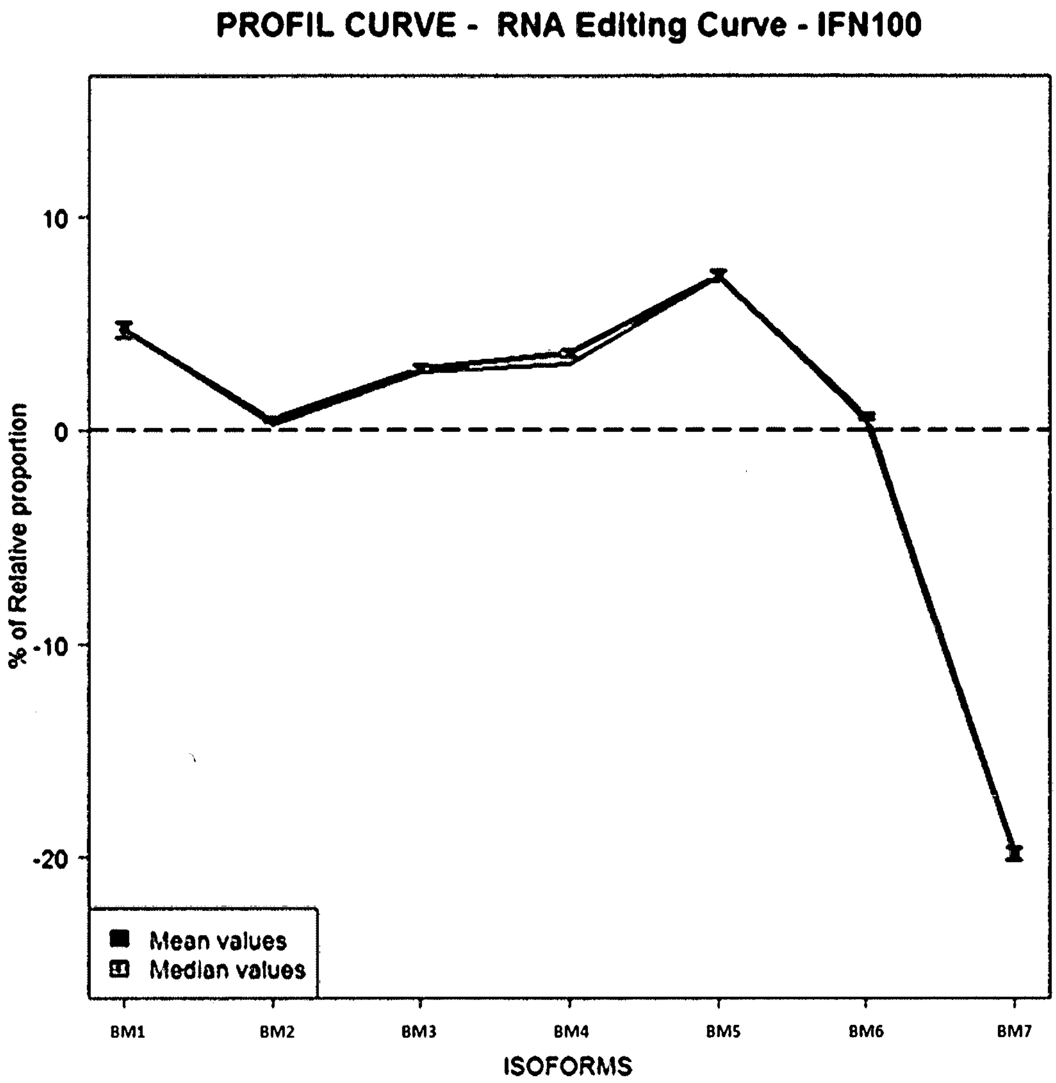
PROFIL CURVE - RNA Editing Curve - IFN100
10
0
-10
-20
% of Relative proportion
Mean values
Median values
BM1
BM2
BM3
BM4
BM5
BM6
BM7
ISOFORMS

FIG. 6

Compound tested With FDA alerts

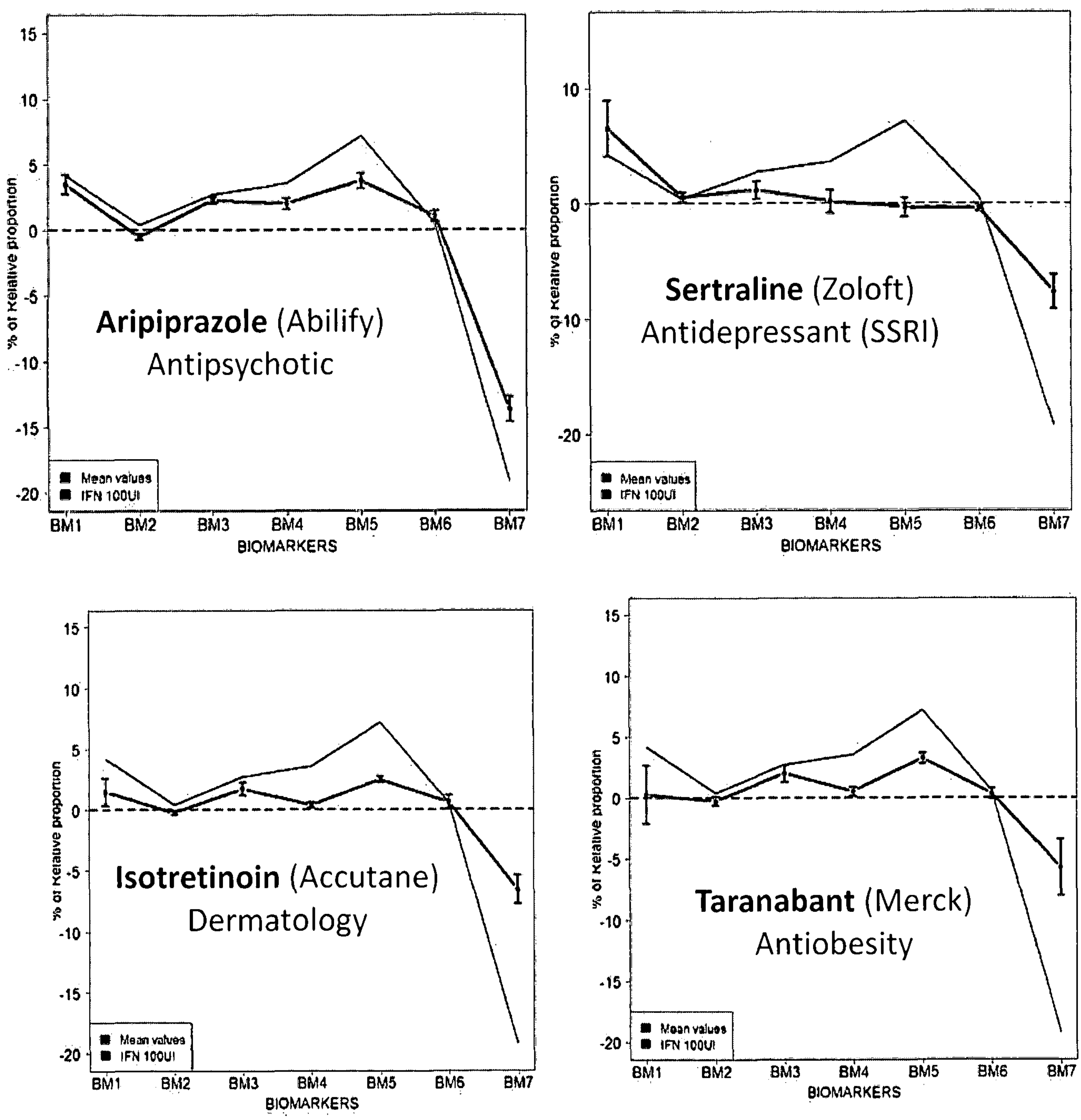


FIG. 7A

Compound tested Without FDA alerts

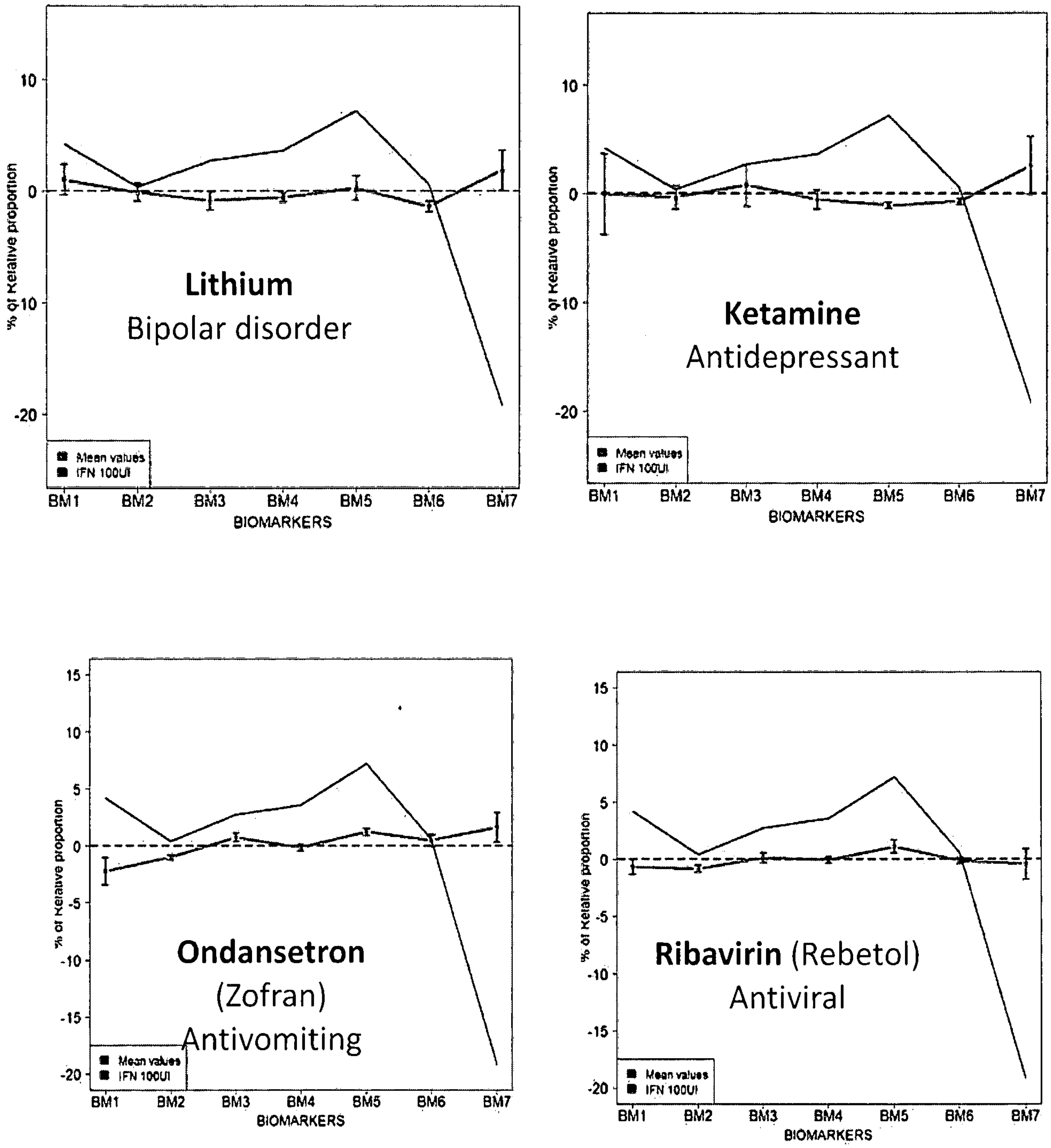


FIG. 7B

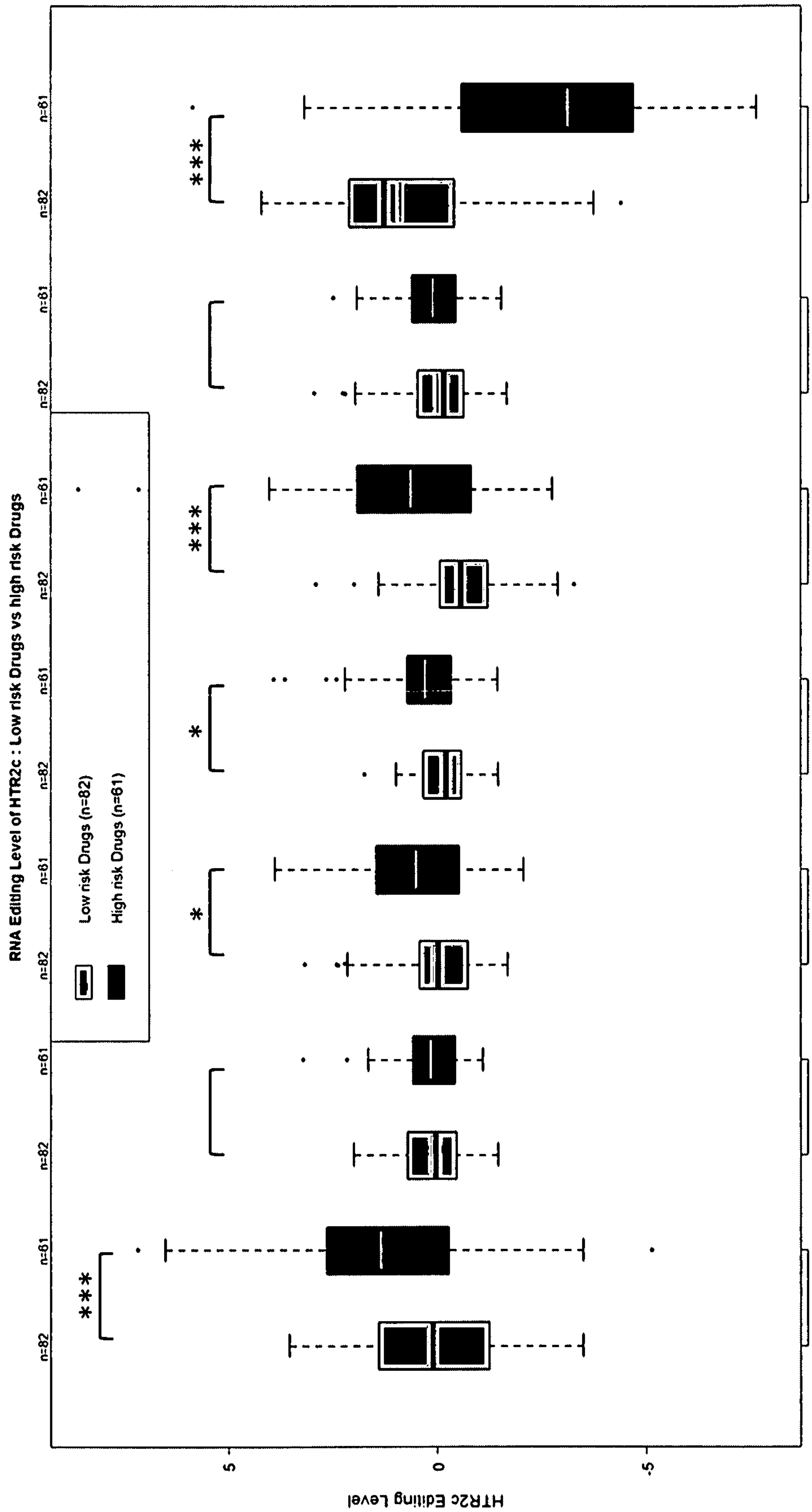
RNA Editing Level of HTR2c : Low risk Drugs vs high risk Drugs
Low risk Drugs (n=82)
High risk Drugs (n=61)
HTR2c Editing Level
5
0
-5
n=82
n=61
***
*
BM1
BM2
BM3
BM4
BM5
BM6
BM7

FIG. 8

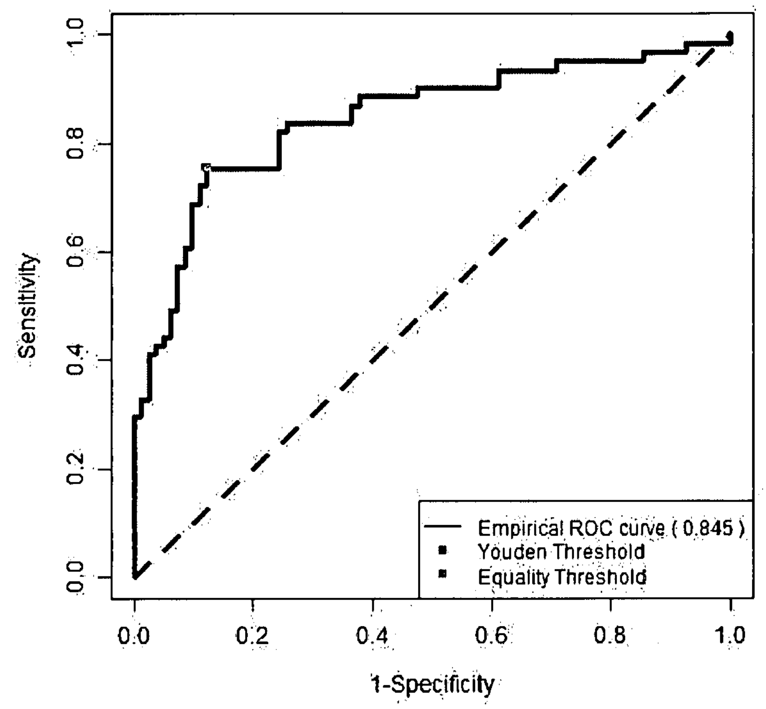
Sensitivity
1-Specificity
0.0
0.2
0.4
0.6
0.8
1.0
Empirical ROC curve ( 0.845 )
Youden Threshold
Equality Threshold

FIG. 9

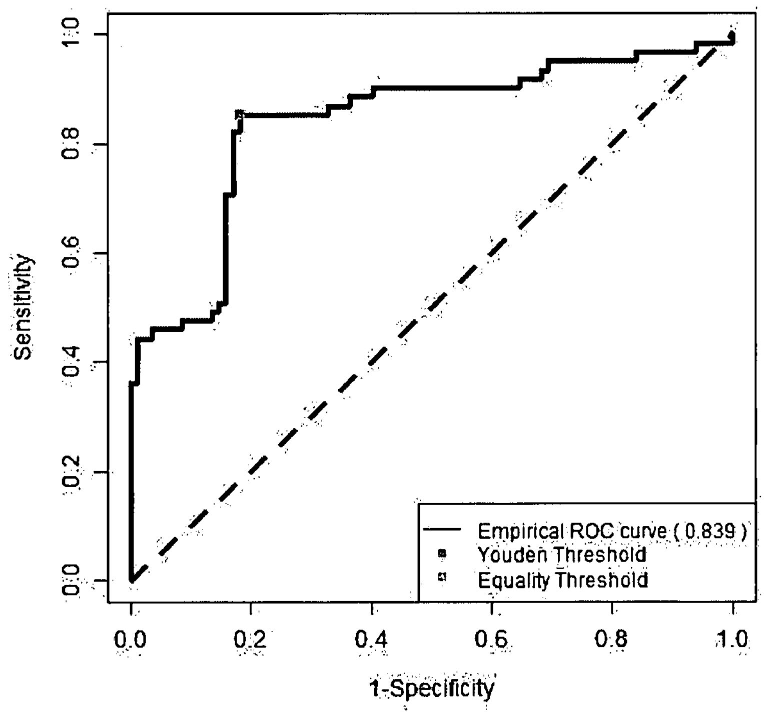
Sensitivity
1-Specificity
0.0
0.2
0.4
0.6
0.8
1.0
Empirical ROC curve ( 0.839 )
Youden Threshold
Equality Threshold

FIG. 10

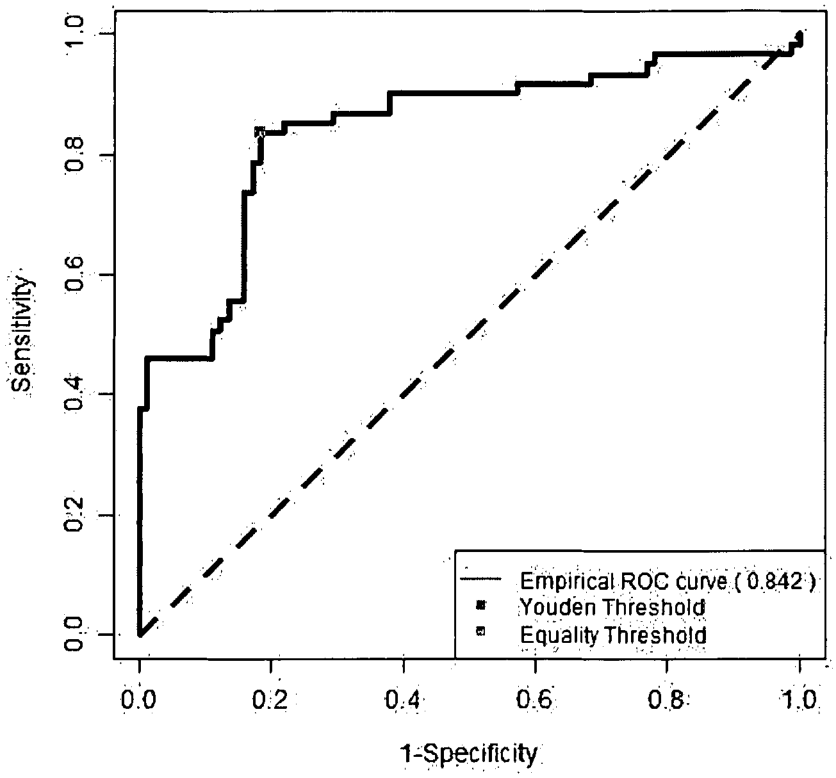
1.0
0.8
0.6
0.4
0.2
0.0
Sensitivity
0.0
0.2
0.4
0.6
0.8
1.0
1-Specificity
Empirical ROC curve ( 0.842 )
Youden Threshold
Equality Threshold

FIG. 11

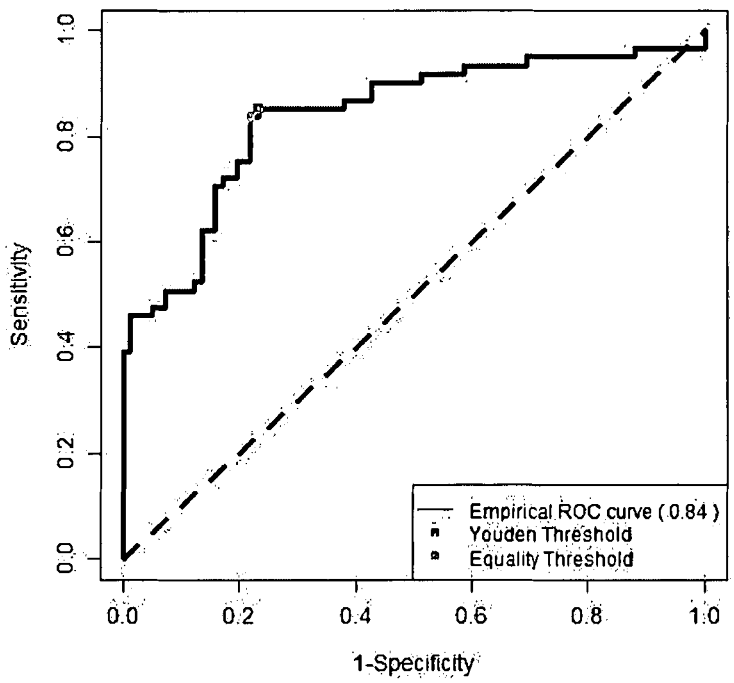
1.0
0.8
0.6
0.4
0.2
0.0
Sensitivity
0.0
0.2
0.4
0.6
0.8
1.0
1-Specificity
Empirical ROC curve ( 0.84 )
Youden Threshold
Equality Threshold

FIG. 12

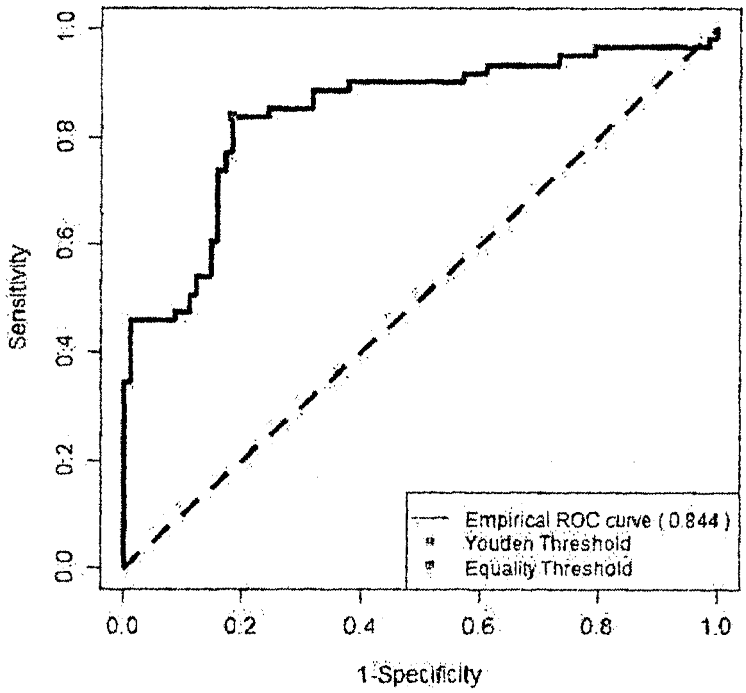
Sensitivity
1-Specificity
0.0
0.2
0.4
0.6
0.8
1.0
Empirical ROC curve ( 0.844 )
Youden Threshold
Equality Threshold

FIG. 13

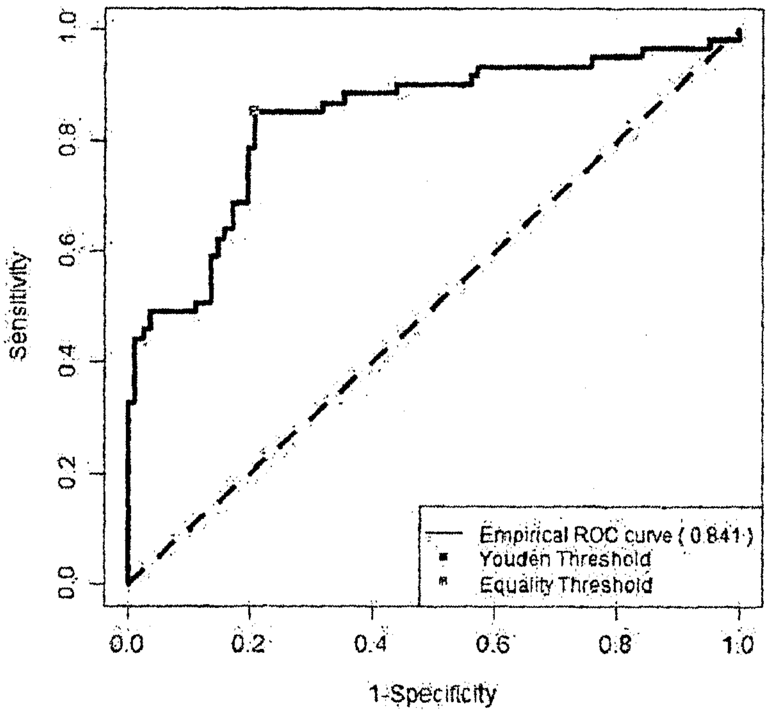
Sensitivity
1-Specificity
0.0
0.2
0.4
0.6
0.8
1.0
Empirical ROC curve ( 0.841 )
Youden Threshold
Equality Threshold

FIG. 14

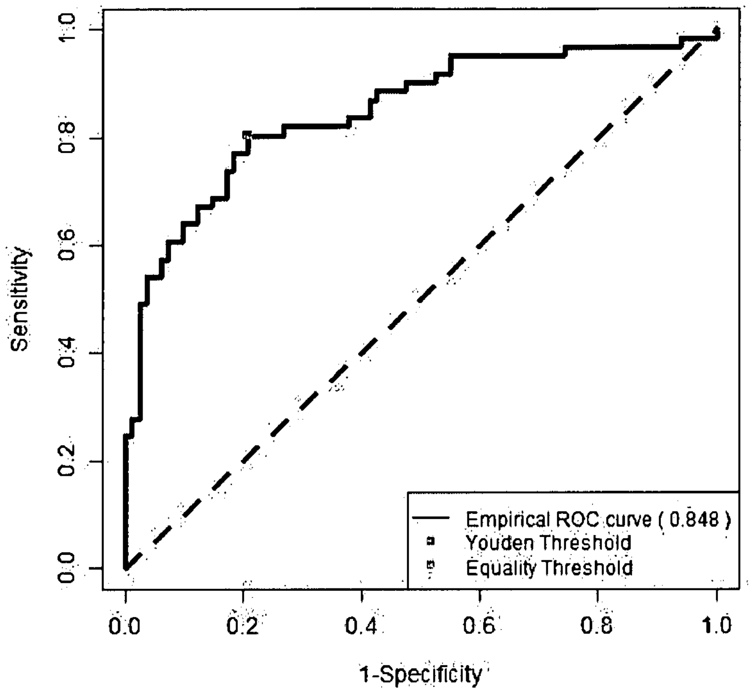
Sensitivity
0.0
0.2
0.4
0.6
0.8
1.0
1-Specificity
Empirical ROC curve ( 0.848 )
Youden Threshold
Equality Threshold

FIG. 15

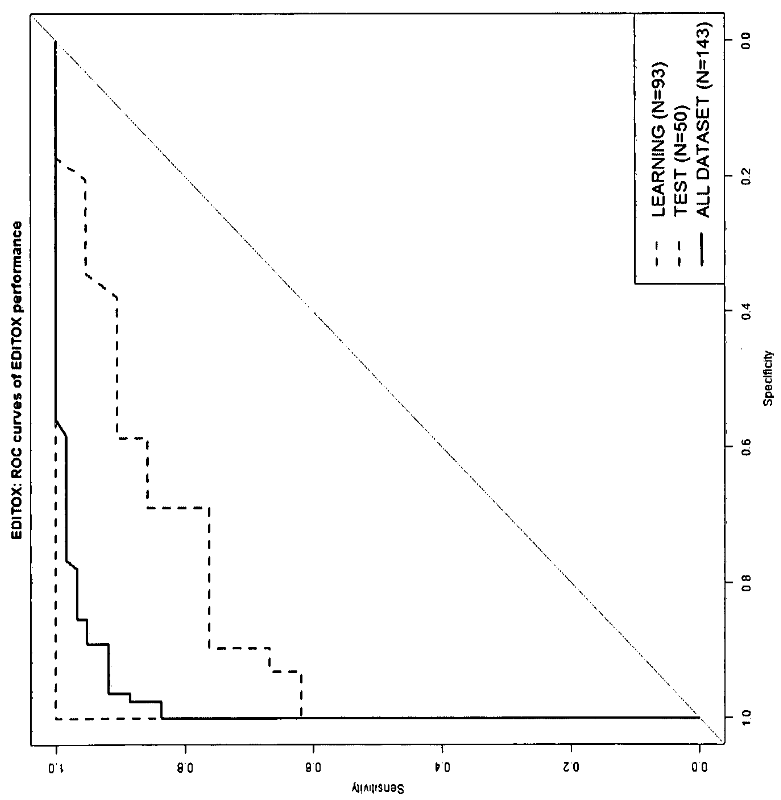


FIG. 16A

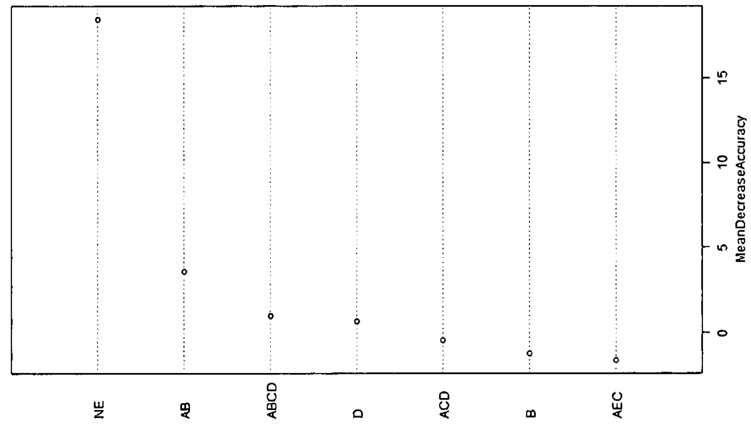


FIG. 16B

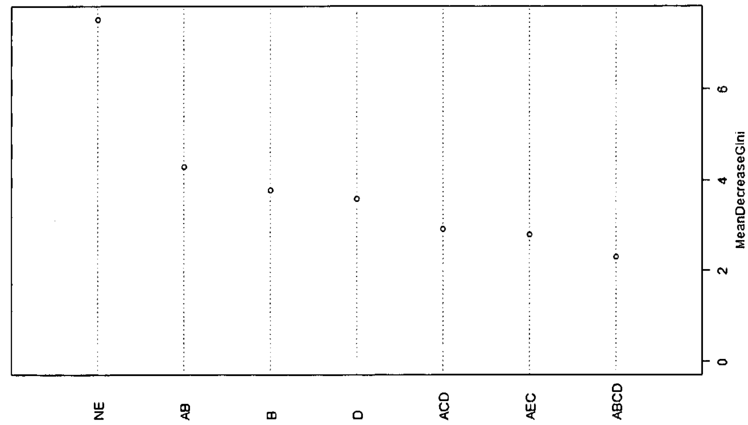


FIG. 16C

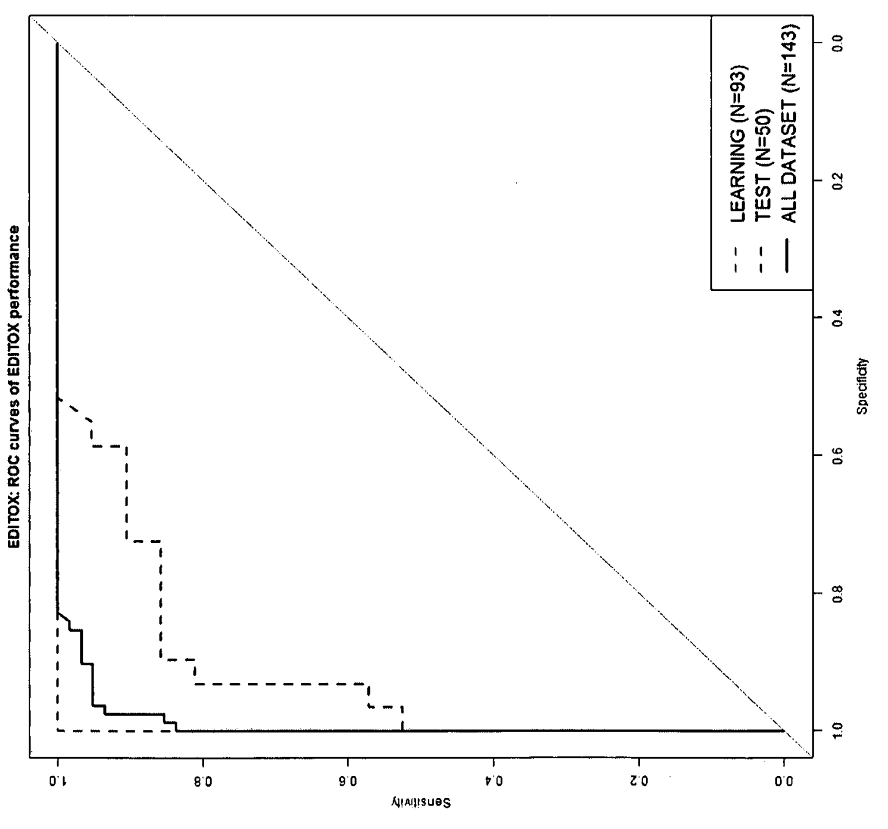


FIG. 17A

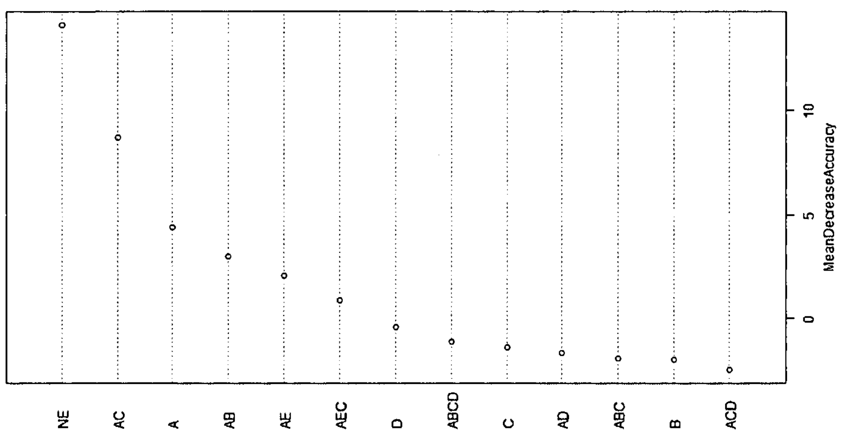


FIG. 17B

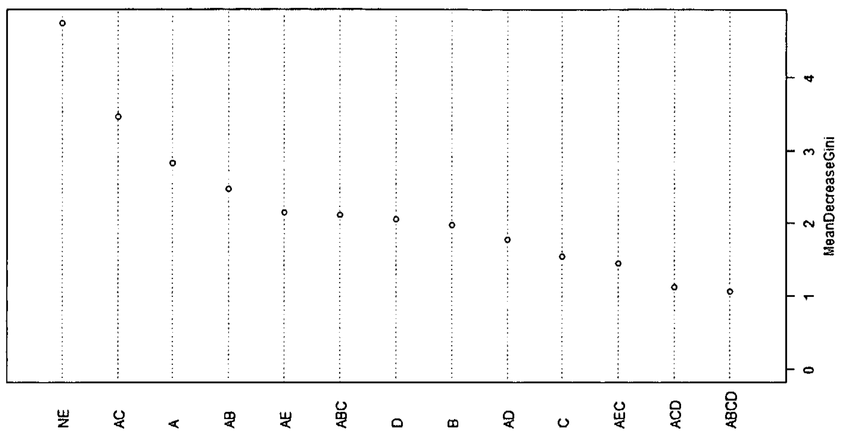


FIG. 17C

FIG. 18A

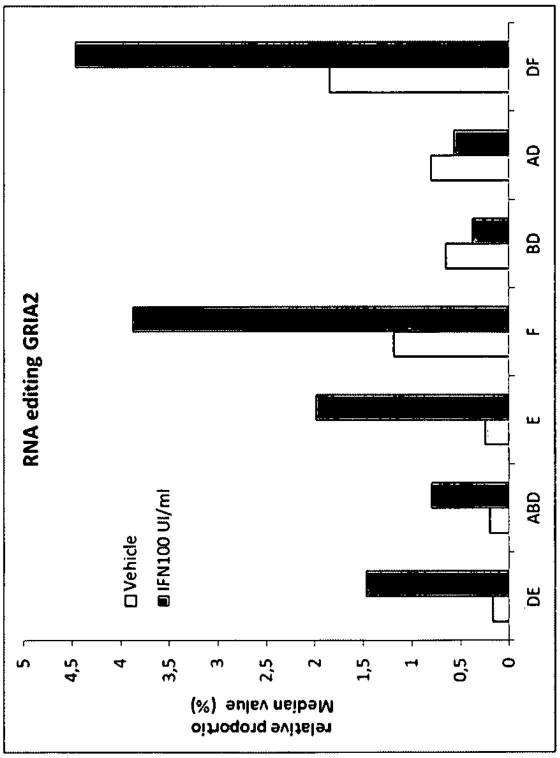


FIG. 18B

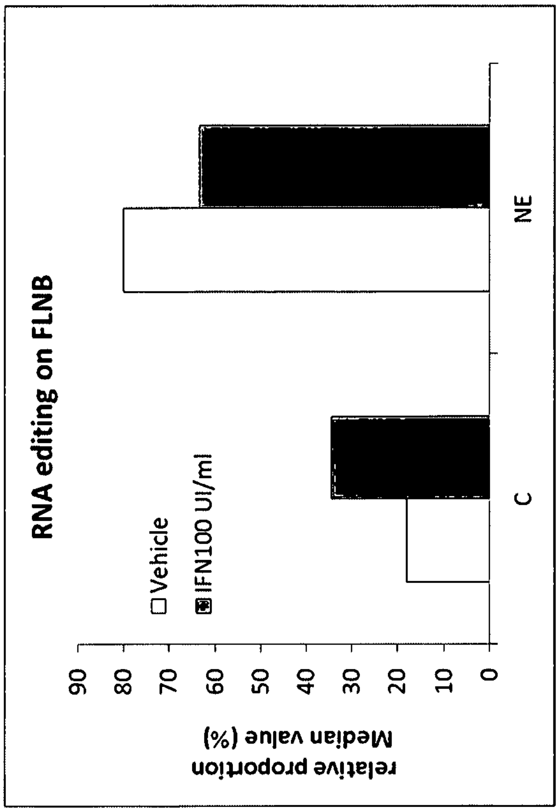


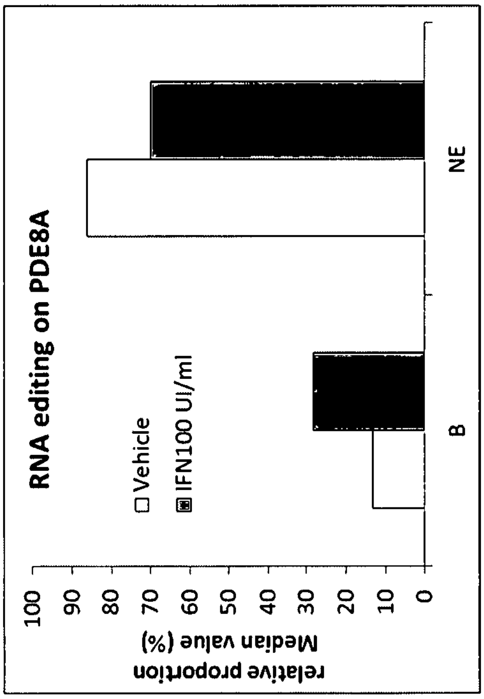


FIG. 18C

LN18

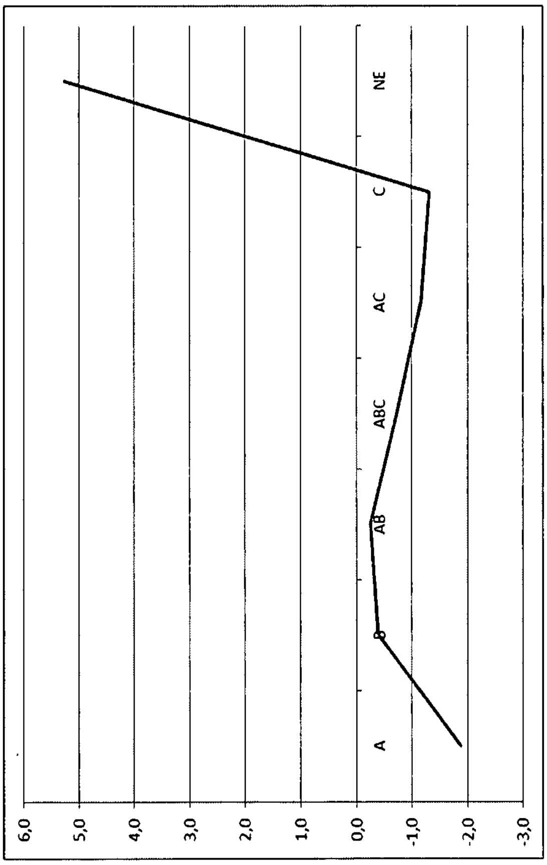


FIG. 19A

LN229

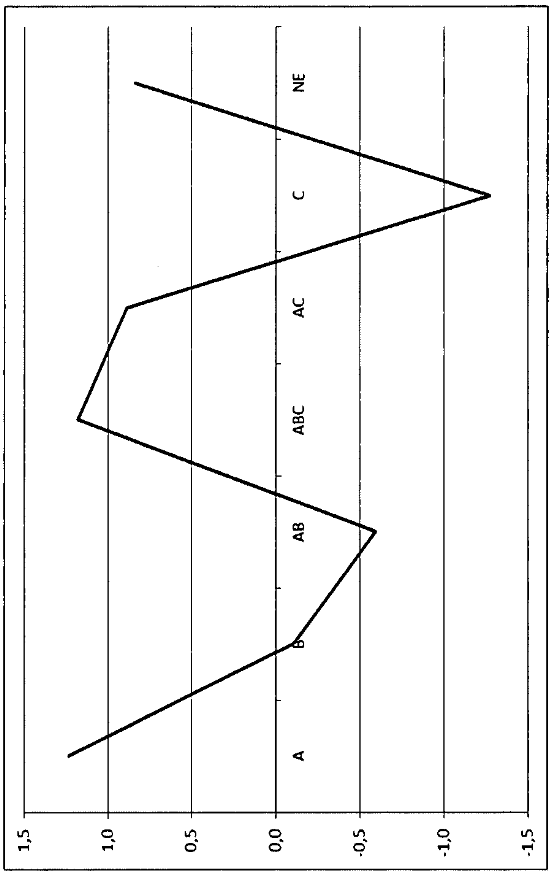


FIG. 19B

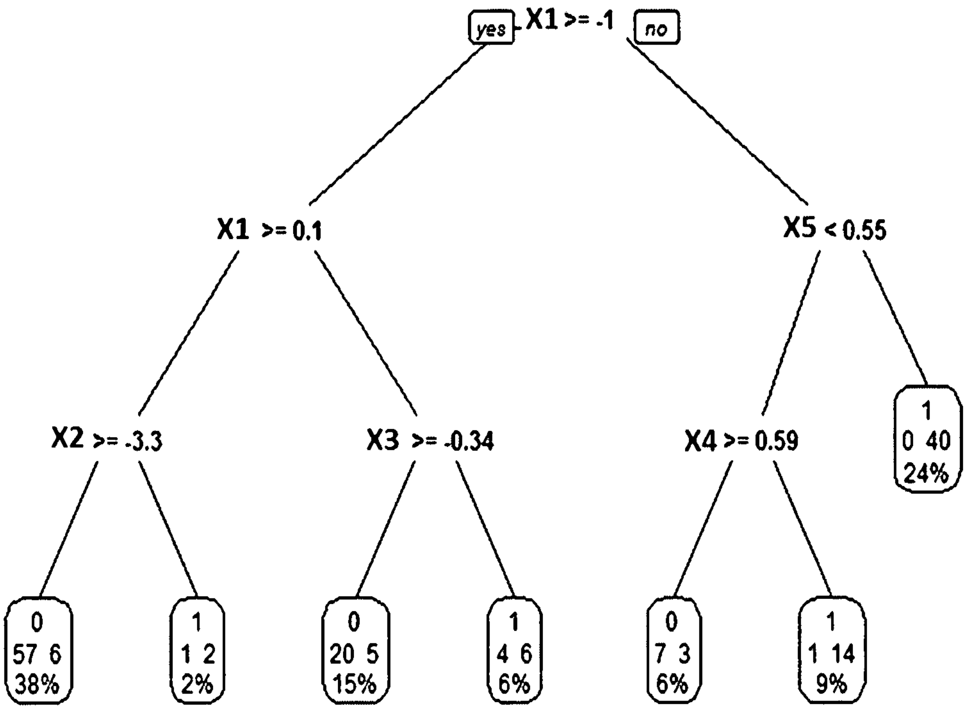
yes
X1 >= -1
no
X1 >= 0.1
X5 < 0.55
X2 >= -3.3
X3 >= -0.34
X4 >= 0.59
1
0 40
24%
0
57 6
38%
1
1 2
2%
0
20 5
15%
1
4 6
6%
0
7 3
6%
1
1 14
9%

FIG. 20

FIG.21A

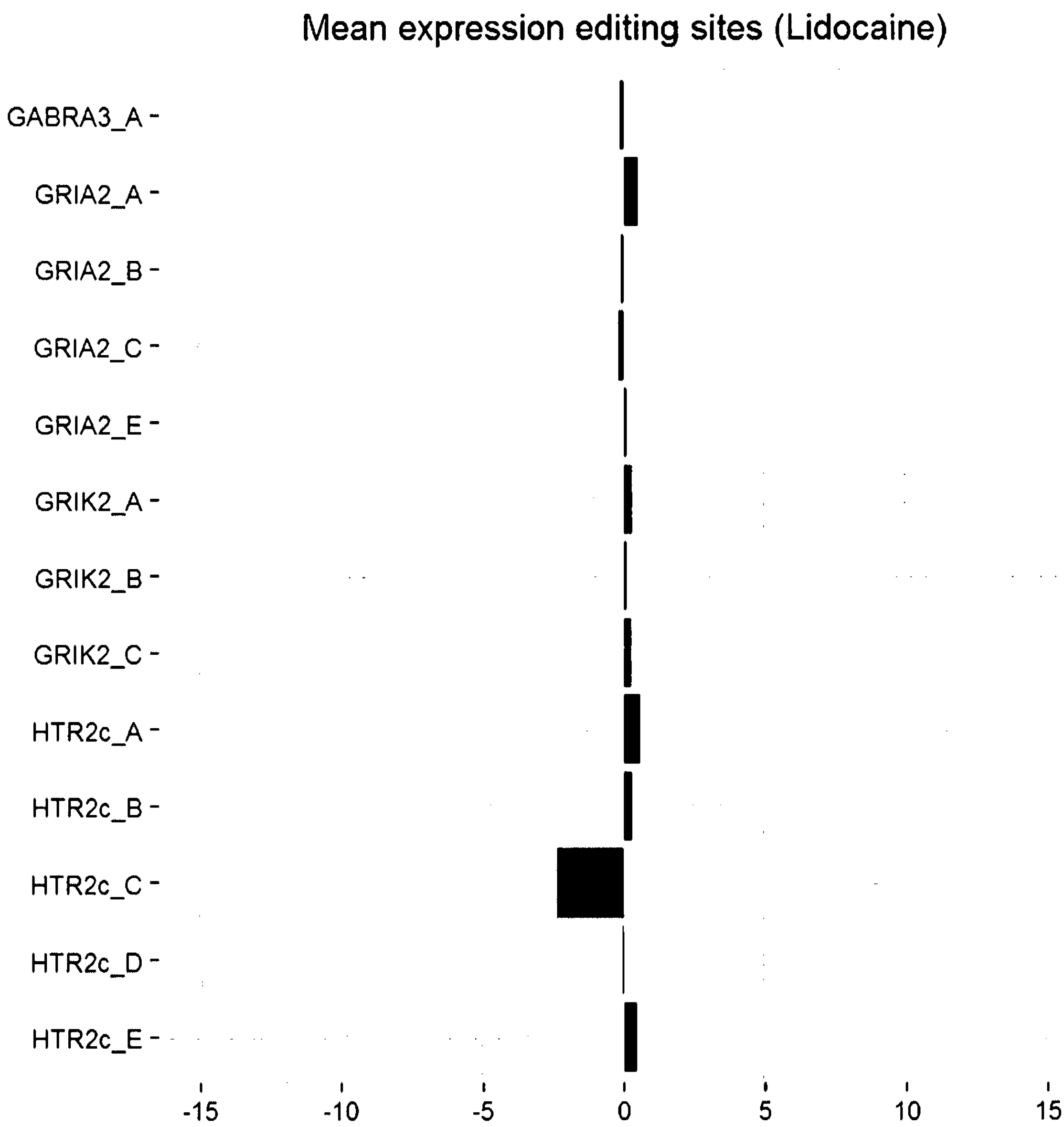
Mean expression editing sites (Lidocaine)
GABRA3_A
GRIA2_A
GRIA2_B
GRIA2_C
GRIA2_E
GRIK2_A
GRIK2_B
GRIK2_C
HTR2c_A
HTR2c_B
HTR2c_C
HTR2c_D
HTR2c_E
-15
-10
-5
0
5
10
15

FIG.21B

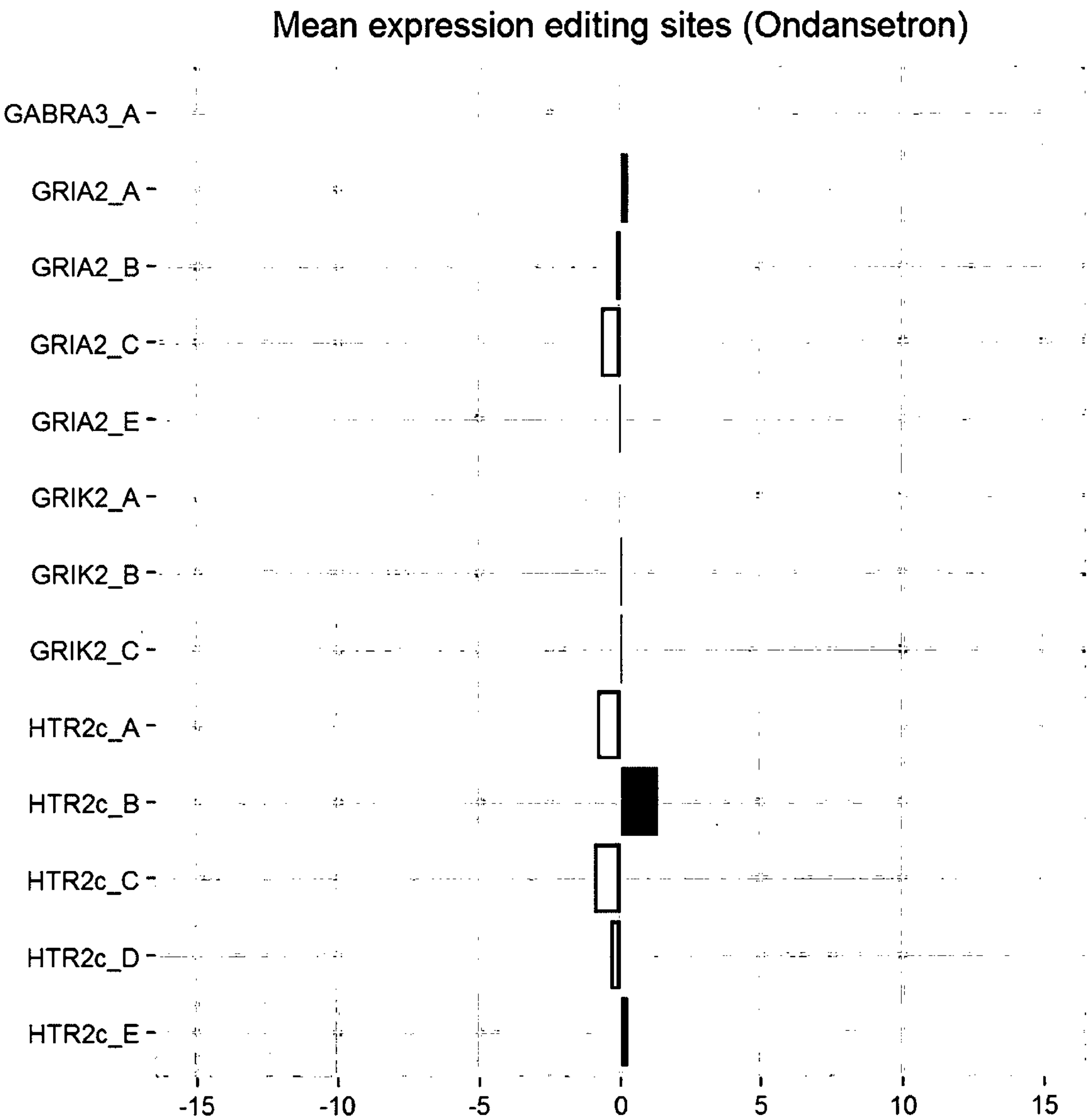
Mean expression editing sites (Ondansetron)
GABRA3_A
GRIA2_A
GRIA2_B
GRIA2_C
GRIA2_E
GRIK2_A
GRIK2_B
GRIK2_C
HTR2c_A
HTR2c_B
HTR2c_C
HTR2c_D
HTR2c_E
-15
-10
-5
0
5
10
15

FIG.21C

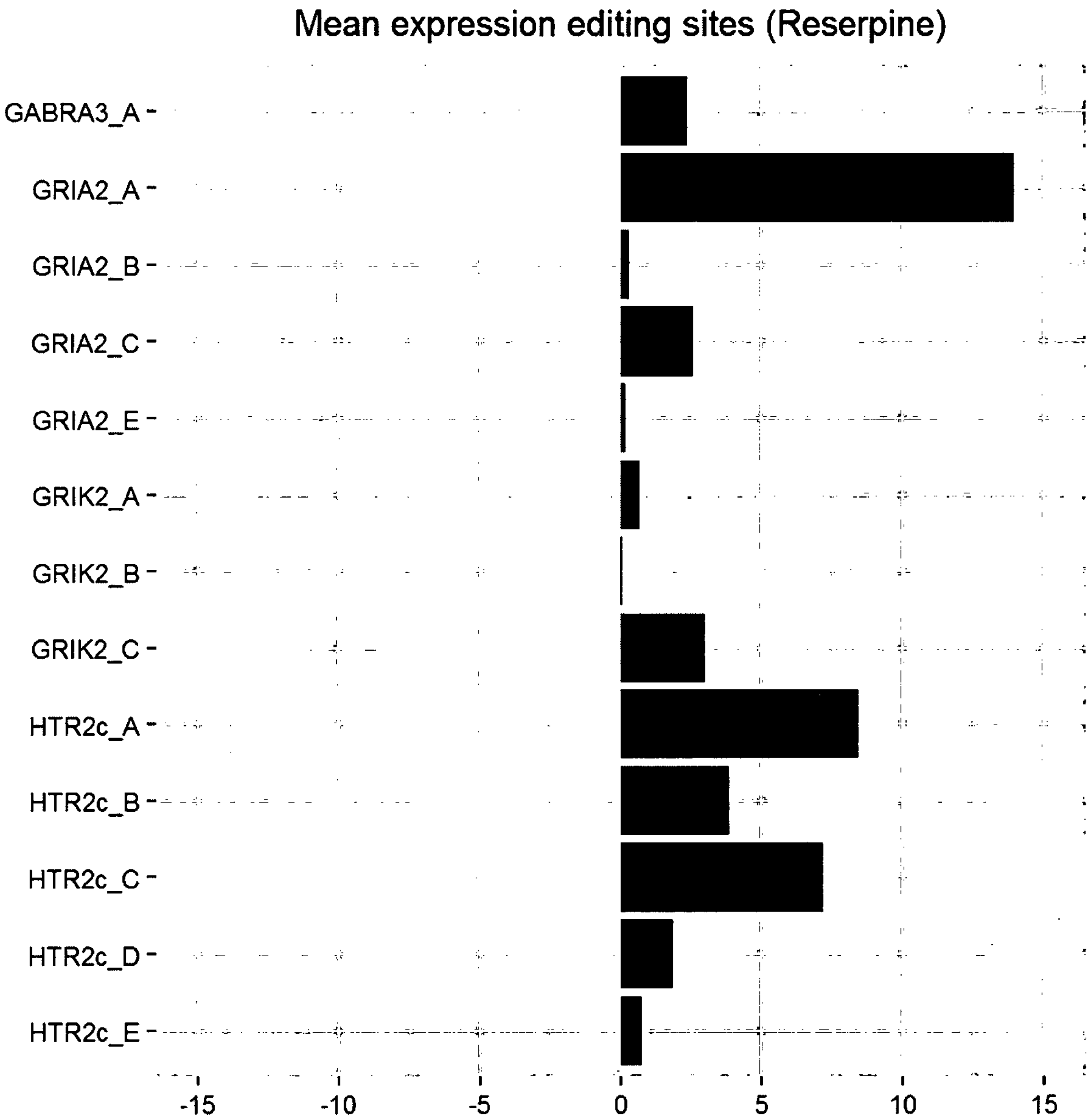
Mean expression editing sites (Reserpine)
GABRA3_A
GRIA2_A
GRIA2_B
GRIA2_C
GRIA2_E
GRIK2_A
GRIK2_B
GRIK2_C
HTR2c_A
HTR2c_B
HTR2c_C
HTR2c_D
HTR2c_E
-15
-10
-5
0
5
10
15

FIG.21D

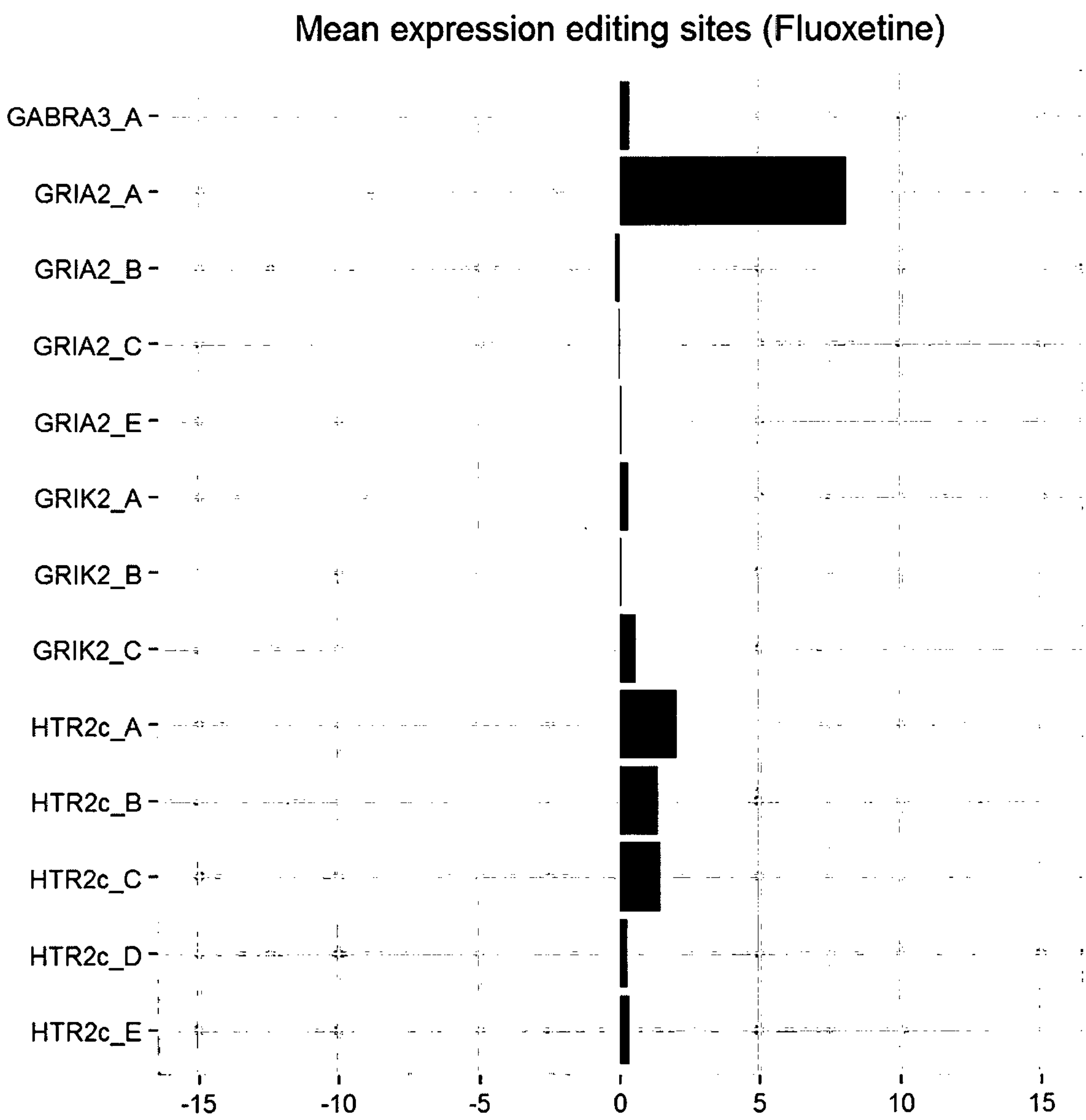
Mean expression editing sites (Fluoxetine)
GABRA3_A
GRIA2_A
GRIA2_B
GRIA2_C
GRIA2_E
GRIK2_A
GRIK2_B
GRIK2_C
HTR2c_A
HTR2c_B
HTR2c_C
HTR2c_D
HTR2c_E
-15
-10
-5
0
5
10
15

FIG.22A

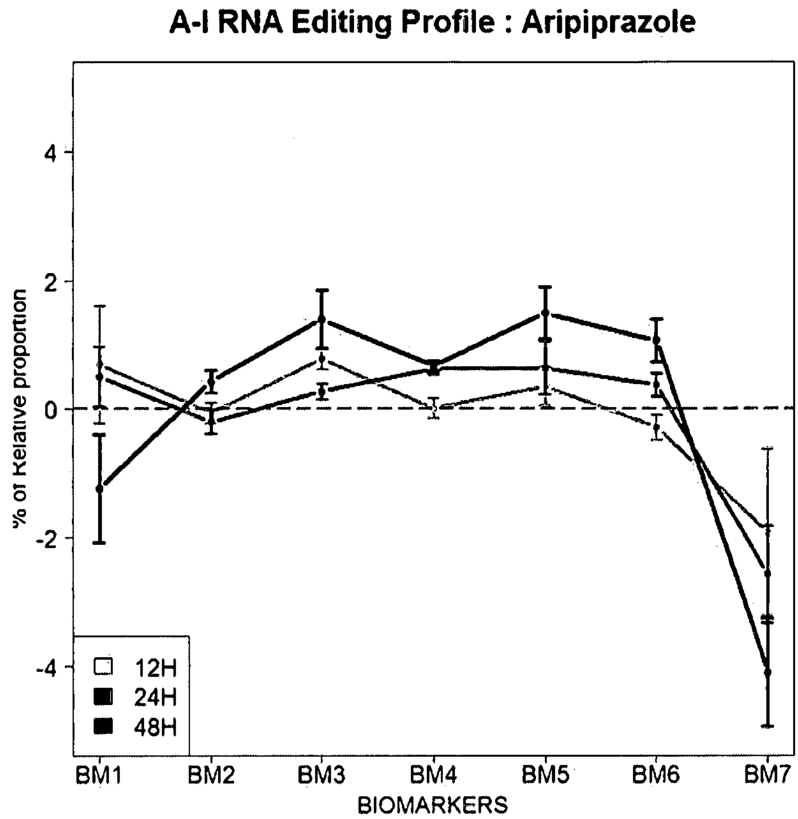


FIG.22B

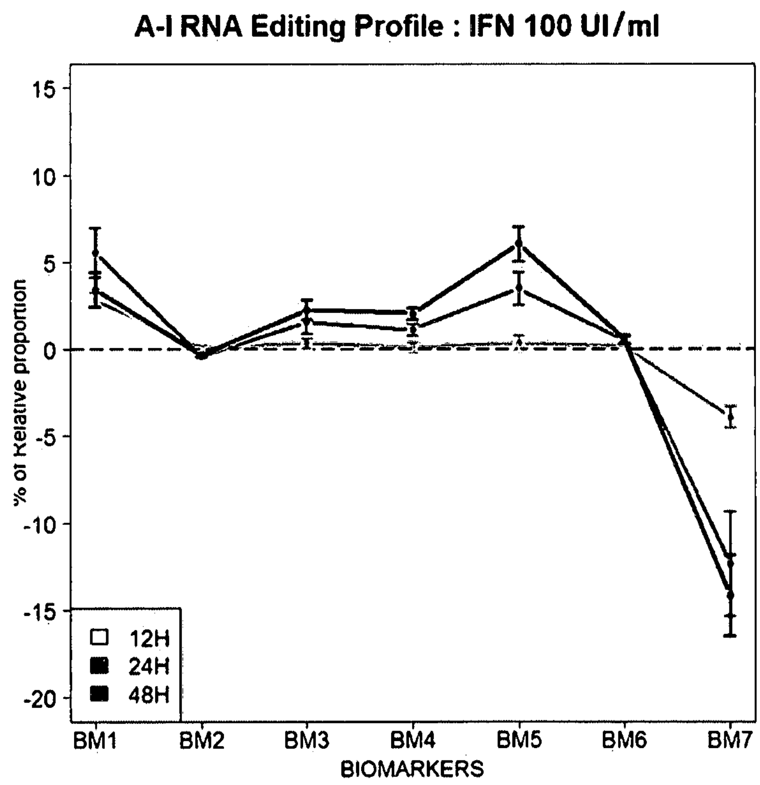

FIG.22C

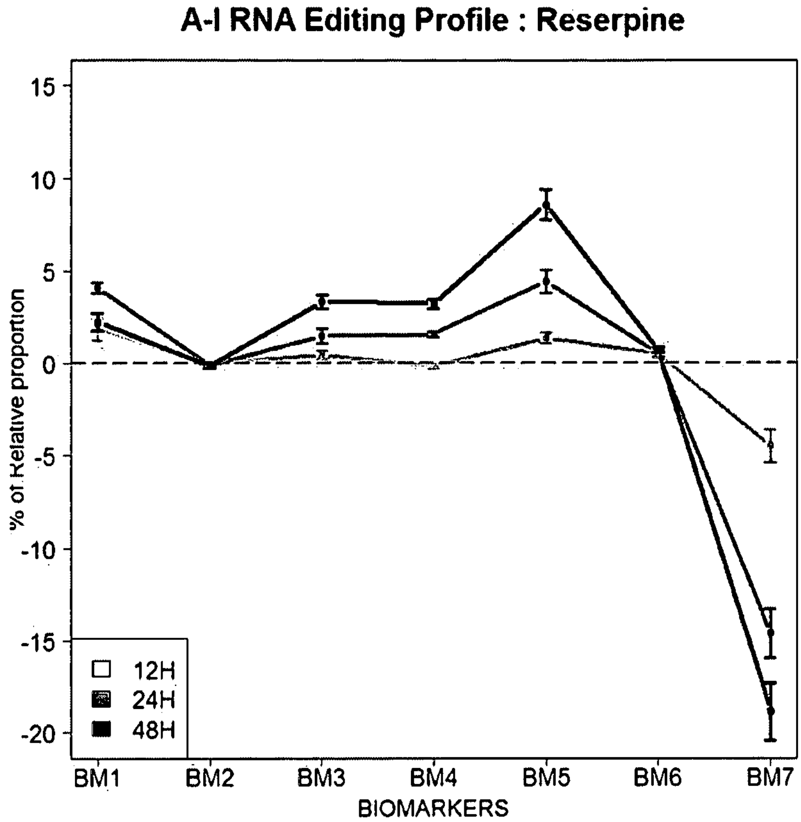


FIG.23A

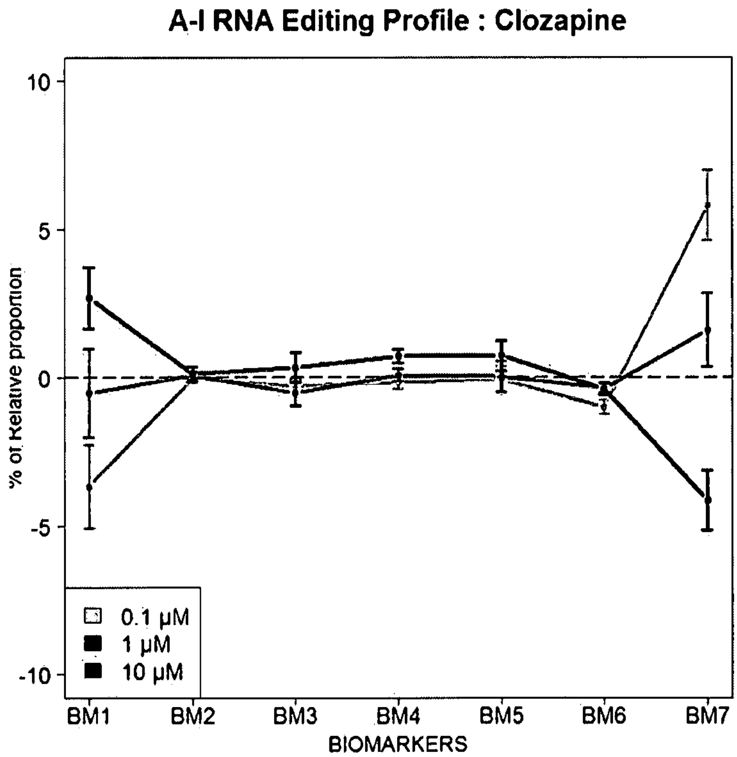

FIG.23B

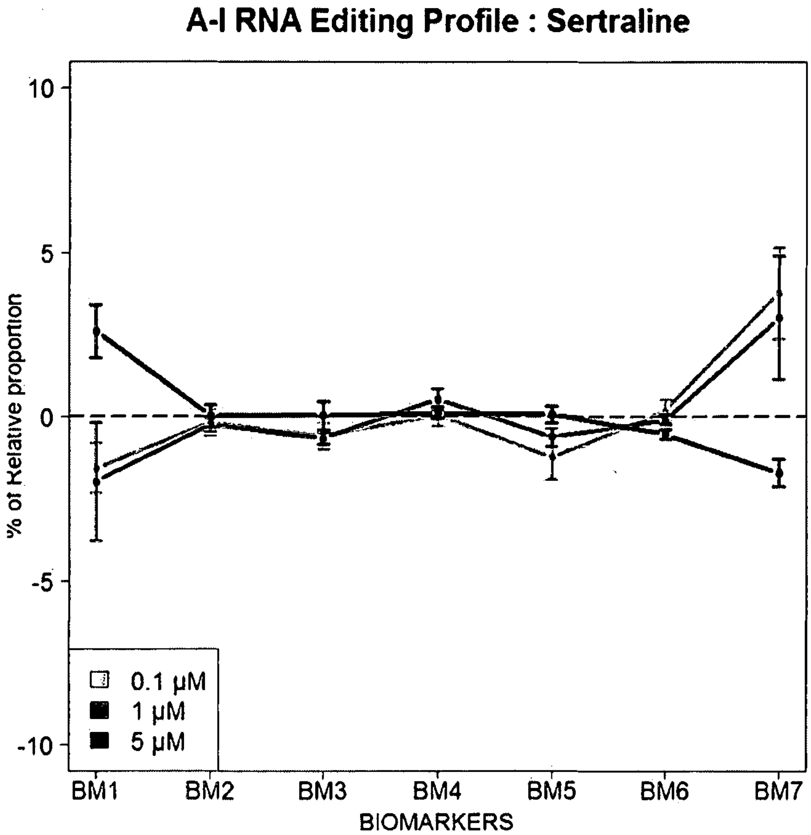


FIG.23C

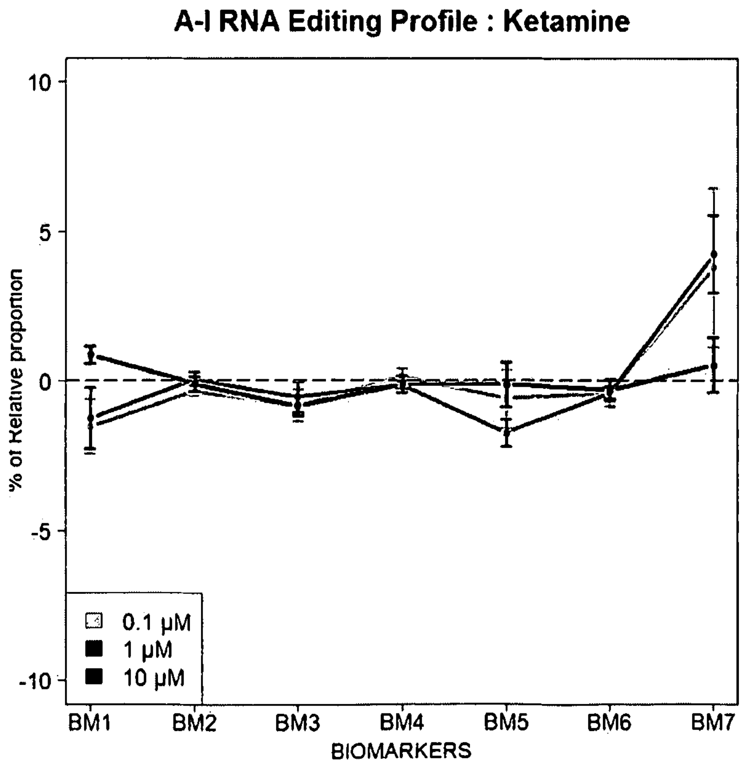

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010070074 PCT, Dinah Weissmann
- WO 2011161253 A, Dinah Weissmann
- WO 2008152146 A
- EP 16000600

### Non-patent literature cited in the description


- *Comput. Methods Programs Biomed.,* 2001, vol. 66, 199-207
- Preventing suicide: A global imperative. WHO, 2014
- **LABONTE B ; TURECKI G.** The epigenetics of suicide: explaining the biological effects of early life environmental adversity. *Archives of suicide research : official journal of the International Academy for Suicide Research,* 2010, vol. 14 (4), 291-310
- **GUREVICH I ; ENGLANDER MT ; ADLERSBERG M ; SIEGAL NB ; SCHMAUSS C.** Modulation of serotonin 2C receptor editing by sustained changes in serotonergic neurotransmission. *The Journal of neuroscience: the official journal of the Society for Neuroscience,* 15 December 2002, vol. 22 (24), 10529-32
- **SODHI MS ; BURNET PW ; MAKOFF AJ ; KERWIN RW ; HARRISON PJ.** RNA editing of the 5-HT(2C) receptor is reduced in schizophrenia. *Molecular psychiatry,* July 2001, vol. 6 (4), 373-9
- **ALON S ; GARRETT SC ; LEVANON EY ; OLSON S ; GRAVELEY BR ; ROSENTHAL JJ et al.** The majority of transcripts in the squid nervous system are extensively recoded by A-to-I RNA editing. *eLife,* 2015, vol. 4
- **KHERMESH K ; D'ERCHIA AM ; BARAK M ; ANNESE A ; WACHTEL C ; LEVANON EY et al.** Reduced levels of protein recoding by A-to-I RNA editing in Alzheimer's disease. *Rna,* February 2016, vol. 22 (2), 290-302
- **PORATH HT ; CARMI S ; LEVANON EY.** A genome-wide map of hyper-edited RNA reveals numerous new sites. *Nature communications,* 2014, vol. 5, 4726
- **SEEBURG PH ; HIGUCHI M ; SPRENGEL R.** RNA editing of brain glutamate receptor channels: mechanism and physiology. *Brain research Brain research reviews,* May 1998, vol. 26 (2-3), 217-29
- **YANG W ; WANG Q ; KANES SJ ; MURRAY JM ; NISHIKURA K.** Altered RNA editing of serotonin 5-HT2C receptor induced by interferon: implications for depression associated with cytokine therapy. *Brain research Molecular brain research,* 29 April 2004, vol. 124 (1), 70-8
- **DRACHEVA S ; PATEL N ; WOO DA ; MARCUS SM ; SIEVER LJ ; HAROUTUNIAN V.** Increased serotonin 2C receptor mRNA editing: a possible risk factor for suicide. *Molecular psychiatry,* November 2008, vol. 13 (11), 1001-10
- **MANN JJ ; BRENT DA ; ARANGO V.** The neurobiology and genetics of suicide and attempted suicide: a focus on the serotonergic system. *Neuropsychopharmacology: official publication of the American College of Neuropsychopharmacology,* May 2001, vol. 24 (5), 467-77
- **MANN JJ ; CURRIER DM.** Stress, genetics and epigenetic effects on the neurobiology of suicidal behavior and depression. *European psychiatry: the journal of the Association of European Psychiatrists,* June 2010, vol. 25 (5), 268-71
- **MANN JJ ; HUANG YY ; UNDERWOOD MD ; KASSIR SA ; OPPENHEIM S ; KELLY TM et al.** A serotonin transporter gene promoter polymorphism (5-HTTLPR) and prefrontal cortical binding in major depression and suicide. *Archives of general psychiatry,* August 2000, vol. 57 (8), 729-38
- **DINAH WEISSMANN ; LAURENT VINCENT ; MARK D. UNDERWOOD ; LAURENT CAVAREC ; NICOLAS SALVETAT ; SIEM VAN DER LAAN et al.** REGION SPECIFIC ALTERATIONS OF RNA EDITING OF SEROTONIN 2C RECEPTOR IN CORTEX OF SUICIDES WITH MAJOR DEPRESSION. *Translational psychiatry,* 2016
- **CHRISTENSEN R ; KRISTENSEN PK ; BARTELS EM ; BLIDDAL H ; ASTRUP A.** Efficacy and safety of the weight-loss drug rimonabant: a meta-analysis of randomised trials. *Lancet,* 17 November 2007, vol. 370 (9600), 1706-13

- **MIHANOVIC M ; RESTEK-PETROVIC B ; BODOR D ; MOLNAR S ; ORESKOVIC A ; PRESECKI P.** Suicidality and side effects of antidepressants and antipsychotics. *Psychiatria Danubina,* March 2010, vol. 22 (1), 79-84
- **MOREIRA FA ; CRIPPA JA.** The psychiatric side-effects of rimonabant. *Revista brasileira de psiquiatria,* June 2009, vol. 31 (2), 145-53
- **SUNDSTROM A ; ALFREDSSON L ; SJOLIN-FORSBERG G ; GERDEN B ; BERGMAN U ; JOKINEN J.** Association of suicide attempts with acne and treatment with isotretinoin: retrospective Swedish cohort study. *Bmj,* 2010, vol. 341, c5812
- **GE Y ; S D, SPEED TP.** Resampling-based multiple testing for microarray data hypothesis. *Sociedad de estadistica e investigacion operativa test,* 2003, vol. 12, 1-77
- **GENTLEMAN RC ; CAREY VJ ; BATES DM ; BOLSTAD B ; DETTLING M ; DUDOIT S et al.** Bioconductor: open software development for computational biology and bioinformatics. *Genome biology,* 2004, vol. 5 (10), R80
- **Y B, Y H.** Controlling the false discovery rate: a practical and powerful approach to multiple testing. *J Roy Statist Soc Ser.,* 1995, vol. B 57 (1), 289-300
- **D.G. K ; L.L. K ; E.M. M.** Applied regression analysis and other multivariate methods. PWS-KENT Publishing Company, 1988
- **L. BREIMAN ; J. FRIEDMAN ; OLSHEN R ; STONE. C.** CART: Classification and Regression Trees. Wadsworth International, 1984
- **BREIMAN L.** Random Forests. *Machine Learning,* 2001, vol. 45 (1), 5-32
- **SU JQ ; LIU JS.** Linear combinations of multiple diagnostic markers. *Journal of the American Statistical Association,* 1993, vol. 88, 1350-5
- **WANG H.** A note on iterative marginal optimization: a simple algorithm for maximum rank correlation estimation. *Computational Statistics and Data Analysis,* 2007, vol. 51, 2803-12
- **STAACK A ; BADENDIECK S ; SCHNORR D ; LOENING SA ; JUNG K.** Combined determination of plasma MMP2, MMP9, and TIMP1 improves the non-invasive detection of transitional cell carcinoma of the bladder. *BMC urology,* 2006, vol. 6, 19
- **CORTES C ; VAPNIK. V.** Support-Vector Networks. *Machine Learning,* 1995, 20
- **BAXT WG.** Application of artificial neural networks to clinical medicine. *Lancet,* 28 October 1995, vol. 346 (8983), 1135-8
- **N. F.** Using Bayesian networks to analyze expression data. *J Comput Biol.,* 2000, vol. 7 (3-4), 601-20
- Weighted k-Nearest-Neighbor Techniques and Ordinal Classification. **K. H ; K.P. S.** Discussion Paper 399, SFB 386. Ludwig-Maximilians University, 2004
- **WOLD S.** PLS-regression: a basic tool of chemometrics. *Chemometrics and Intelligent Laboratory Systems,* 2001, vol. 58 (108-130
- **FISHER RA.** The Use of Multiple Measurements in Taxonomic Problems. *Annals of Eugenics,* 1936, vol. 2, 179-188
- **CAVAREC L ; VINCENT L ; LE BORGNE C ; PLUSQUELLEC C ; OLLIVIER N ; NORMAND-IE-LEVI P et al.** In vitro screening for drug-induced depression and/or suicidal adverse effects: a new toxicogenomic assay based on CE-SSCP analysis of HTR2C mRNA editing in SH-SY5Y cells. *Neurotoxicity research,* January 2013, vol. 23 (1), 49-62

SEQUENCE LISTING

<110> ALCEDIAG
CNRS

<120> Algorithm and an in vitro method based on RNA editing to select particular effect induced by active compounds

<130> ALC B001 PCT

<150> EP16000600.3
<151> 2016-03-11

<160> 10

<170> PatentIn version 3.5

<210> 1
<211> 22
<212> DNA
<213> artificial

<220>
<223> PDE8A target:Forward primer

<400> 1
caacccactt atttctgcct ag 22

<210> 2
<211> 20
<212> DNA
<213> artificial

<220>
<223> PDE8A target:Reverse primer

<400> 2
ttctgaaaac aatgggcacc 20

<210> 3
<211> 20
<212> DNA
<213> artificial

<220>
<223> FNLB target:Forward primer

<400> 3
aaatgggtcg tgcggtgtat 20

<210> 4
<211> 21
<212> DNA
<213> artificial

<220>
<223> FNLB target:Reverse primer

<400> 4
cctgctcggg gtggtgttaa t 21

<210> 5
<211> 22
<212> DNA
<213> artificial

<220>
<223> GRIA2 target:Forward primer

<400> 5
ctctttagtg gagccagagt ct 22

<210> 6
<211> 20
<212> DNA
<213> artificial

<220>
<223> GRIA2 target:Reverse primer

<400> 6
tcctcagcac tttcgatggg

<210> 7
<211> 20
<212> DNA
<213> artificial

<220>
<223> GRIK2 target:Forward primer

<400> 7
cctgaatcct ctctcccctg

<210> 8
<211> 20
<212> DNA
<213> artificial

<220>
<223> GRIK2 target:Reverse primer

<400> 8
ccaaatgcct cccactatcc

<210> 9
<211> 20
<212> DNA
<213> artificial

<220>
<223> GABRA3 target:Forward primer

<400> 9
ccaccttgag tatcagtgcc

<210> 10
<211> 21
<212> DNA
<213> artificial

<220>
<223> GABRA3 target:Reverse primer

<400> 10
cgatgttgaa ggtagtgctg g 20